# EUROPÄISCHE PATENTSCHRIFT

(11) **EP 3 806 938 B1**
(45) Veröffentlichungstag und Bekanntmachung des Hinweises auf die Patenterteilung: **20.12.2023**
(21) Anmeldenummer: 19732870.1
(22) Anmeldetag: 12.06.2019
(51) Int. Cl.: A61M 5/24, B05B 9/08, B05B 11/02, A61M 15/00, A61M 5/28, B05B 11/00

(54) **SYSTEM, KARTUSCHE UND VERFAHREN**
SYSTEM, CARTRIDGE AND METHOD
SYSTÈME, CARTOUCHE ET PROCÉDÉ

(30) Priorität: 15.06.2018 EP 18177967
(43) Veröffentlichungstag der Anmeldung: 21.04.2021
(73) Patentinhaber: Boehringer Ingelheim International GmbH, 55216 Ingelheim am Rhein (DE)
(72) Erfinder: JUNG, Andree, 55216 Ingelheim am Rhein (DE); DUNNE, Stephen Terence, 1450-707 Porto (PT); EICHER, Joachim Carl Herbert, 55216 Ingelheim am Rhein (DE)
(74) Vertreter: Simon, Elke Anna Maria
(86) Internationale Anmeldenummer: PCT/EP2019/065344
(87) Internationale Veröffentlichungsnummer: WO 2019/238749

(56) Entgegenhaltungen:
- EP-A1- 0 599 649
- EP-A2- 1 616 591
- EP-A2- 2 801 530
- US-A1- 2003 111 552
- US-A1- 2006 178 641
- US-B1- 6 626 379

## Beschreibung

Die vorliegende Patentanmeldung betrifft eine Kartusche für eine Abgabevorrichtung bzw. einen Zerstäuber gemäß dem Oberbegriff des Anspruchs 1, 3 oder 7, ein System gemäß dem Oberbegriff des Anspruchs 15 und ein Verfahren zur Vorbereitung eines Systems für die Abgabe bzw. Zerstäubung eines Fluids gemäß dem Oberbegriff des Anspruchs 17.

Die vorliegende Erfindung betrifft die Zerstäubung bzw. Abgabe eines Fluids, vorzugsweise einer Flüssigkeit, insbesondere eines flüssigen Arzneimittels bzw. einer flüssigen Arzneimittelzusammensetzung, an einen Nutzer bzw. Patienten mittels einer Abgabevorrichtung, vorzugsweise eines insbesondere mechanischen Zerstäubers, und einer vorzugsweise austauschbaren Kartusche, die das zu zerstäubende Fluid enthält.

Um die Haltbarkeit eines für die Abgabe bzw. Zerstäubung vorgesehenen Arzneimittels zu verlängern, insbesondere auf mindestens zwei oder drei Jahre, und/oder um nachteiligen Veränderungen eines Arzneimittels entgegenzuwirken, die insbesondere bei längerer Lagerung des Arzneimittels auftreten, können einzelne Bestandteile des Arzneimittels in einer Kartusche getrennt aufbewahrt und erst kurz bzw. unmittelbar vor der Benutzung des Zerstäubers bzw. vor der Zerstäubung miteinander in Kontakt gebracht bzw. gemischt werden.

Die getrennt aufbewahrten Bestandteile des Arzneimittels können unterschiedliche Aggregatzustände haben bzw. flüssig, gasförmig oder fest, insbesondere pulverförmig, in der Kartusche vorliegen. Beispielsweise kann ein erster Bestandteil des Arzneimittels ein fester Arzneistoff - vorzugsweise in Pulverform - und ein zweiter Bestandteil des Arzneimittels ein - insbesondere flüssiges - Lösungsmittel sein.

Insbesondere kann der Arzneistoff in unterschiedlichen physikochemischen Zuständen bzw. fest, flüssig, gelöst oder suspendiert vorliegen.

Die WO 97/39831 A1 offenbart eine Kartusche mit zwei Kammern zur getrennten Aufbewahrung von Wirkstoff und Lösungsmittel eines Arzneimittels. Beim Einsetzen der Kartusche in einen Zerstäuber zur Zerstäubung des Arzneimittels wird die Kammer mit dem Wirkstoff mittels einer Kanüle durchstochen, so dass der Wirkstoff mit dem Lösungsmittel in Kontakt kommt und aufgelöst wird. Durch die getrennte Aufbewahrung von Wirkstoff und Lösungsmittel wird die Lagerzeit des Arzneimittels verlängert.

Die WO 00/23037 A1 offenbart ebenfalls eine derartige Kartusche für einen Zerstäuber, wobei die Kartusche zwei Kammern zur getrennten Aufbewahrung von einzelnen Bestandteilen eines Arzneimittels aufweist.

Die US 2003/0111552 A1 zeigt eine Sprühvorrichtung mit einer Sprühdüse, einer Hülse und mit mindestens zwei in der Hülse angeordneten, voneinander beabstandeten Stopfen, die eine erste und eine zweite Kammer der Hülse definieren, in denen sich jeweils eine Substanz befindet. Ein Kolben, der mit dem ersten Stopfen verbunden ist, kann in die Hülse gedrückt werden. Hierdurch werden die Kolben in der Hülse bewegt, so dass ein Bypass freigegeben wird und sich die Substanzen in den beiden Kammern mischen. Durch weiteres Drücken des Kolbens wird die vermischte Substanz über die Sprühdüse ausgegeben. Die Vorrichtung kann eine Kappe zum Verschließen der Sprühdüse aufweisen, die entweder vor der Betätigung der Sprühvorrichtung entfernt wird oder Luftkanäle aufweist, sodass Luft beim Mischen entweichen kann.

Die EP 0 568 321 A2 betrifft eine Fertigspritze. Die Spritze weist einen rohrförmigen Körper mit einer Injektionsnadel an der einen Seite und einem Kolben an der anderen Seite sowie ein dazwischen angeordnetes Trennstück auf. Das Trennstück unterteilt den Innenraum des rohrförmigen Körpers in zwei separate Kammern, in denen sich jeweils eine Substanz befindet. Ferner weist der rohrförmige Körper einen Bypass auf, um die Substanzen in den Kammern vor der Injektion zu mischen, indem das Trennstück durch Druck auf den Kolben so verschoben wird, dass es benachbart zum Bypass angeordnet ist.

Die US 2013/0197467 A1 betrifft ein abgedichtetes Gefäß, insbesondere eine Mehrkammer-Spritze. Die Spritze weist eine Hülse mit einem Bypass und drei Kolben auf, die den Innenraum der Hülse in drei Kammern unterteilen, wobei zwei der Kammern zu mischende Substanzen aufweisen. Durch Herunterdrücken einer Kolbenstange werden die Kolben verschoben, sodass sich die Substanzen über den Bypass mischen können.

Die US 6,626,379 B1 betrifft eine Abgabevorrichtung für pharmazeutische Produkte. Die Abgabevorrichtung weist eine erste Kammer mit einem Feststoff nahe der Ausgabeöffnung sowie eine zweite Kammer mit einer flüssigen Substanz an einer der Ausgabeöffnung abgewandten Unterseite auf. Die zweite Kammer ist durch einen Stopfen abgedichtet. Durch Ausüben von Druck auf die Unterseite wird eine Nadel durch den Stopfen gestochen und so die zweite Kammer zur Umgebung geöffnet. Durch weitere Ausübung von Druck wird die Flüssigkeit aus der zweiten Kammer in die Nadel und weiter in die erste Kammer geleitet, wo sie sich mit der festen Substanz mischt und anschließend ausgegeben wird. In einer alternativen Ausführungsform ist die erste Kammer zwischen dem Stopfen und der zweiten Kammer angeordnet, wobei ein zusätzlicher Kolben die beiden Kammern voneinander trennt. Bei Betätigung wird hier zunächst der Stopfen durchstochen und so die erste Kammer zur Umgebung geöffnet. Anschließend wird der Kolben heruntergedrückt, sodass sich ein Bypass öffnet und die Substanzen miteinander vermischt werden bevor sie durch die Nadel zu der Ausgabeöffnung geleitet werden.

Die EP 3 205 228 A1 betrifft einen kosmetischen Behälter zum Mischen von zwei kosmetischen Flüssigkeiten. Der Behälter weist einen starren Hauptkörper auf, in dem sich die erste Flüssigkeit befindet, sowie eine zusammendrückbare Tasche am unteren Ende des Hauptkörpers, in dem sich die zweite Flüssigkeit befindet. Zwischen Hauptkörper und Tasche befindet sich ein Ein-Wege-Ventil. Durch Zusammendrücken der Tasche wird die zweite Flüssigkeit durch das Ventil in den Hauptkörper gedrückt, wo sie sich mit der ersten Flüssigkeit mischt.

Die EP 1 707 495 A1 betrifft eine Vorrichtung zum Halten und Mischen von zwei Kosmetika, insbesondere zur Herstellung eines kosmetischen Haarbehandlungsmittels. Die Vorrichtung weist einen Behälter mit einer Öffnung und einer ersten Flüssigkeit auf. An der Behälteröffnung ist ein Zwischenteil befestigt, auf das eine Verschlusseinheit geschraubt ist, sodass der Behälter verschlossen ist. Zwischen dem Zwischenteil und der Verschlusseinheit ist eine Kammer mit einer zweiten Flüssigkeit gebildet. Durch teilweises Abdrehen der Verschlusseinheit wird die Kammer zur Behälteröffnung hin geöffnet, sodass die zweite Flüssigkeit in den Behälter fließt und sich mit der ersten Flüssigkeit mischt. Anschließend kann die Verschlusseinheit vollständig abgedreht werden und ein Abgabeteil zur dosierten Abgabe der gemischten Flüssigkeit in den Behälter eingesetzt werden.

Die US 5,836,479 A betrifft eine wiederbefüllbare Abgabevorrichtung, insbesondere eine Sprühflasche. Die Vorrichtung weist einen Behälter und ein oder mehrere Reservoirs für Chemikalien auf. Der Behälter kann mit Wasser gefüllt werden und anschließend das Reservoir oder eines der Reservoirs zum Behälter hin geöffnet werden, um eine verdünnte chemische Lösung herzustellen. Gemäß einer Ausführungsform ist das Reservoir als Kappe ausgebildet. Hierbei sind zwei Kammern in der Kappe gebildet, die jeweils eine Chemikalie enthalten. Durch Drehen der Kappe brechen die Wände der Kammern, sodass sich die Chemikalien mischen und in den Behälter abgegeben werden.

Die EP0,599,649 A1 offenbart eine Kartusche für eine Abgabevorrichtung zur Mischung zweier Arzneimittelbestandteile.

Der vorliegenden Erfindung liegt die Aufgabe zugrunde, ein verbessertes System, eine verbesserte Kartusche und ein verbessertes Verfahren zur Vorbereitung eines aus mehreren Bestandteilen zusammengesetzten Fluids für die Abgabe bzw. Zerstäubung anzugeben, vorzugsweise wobei ein einfaches, schnelles, sicheres, hygienisches, zuverlässiges und/oder homogenes Mischen von fluidisch voneinander getrennt aufbewahrten Bestandteilen eines Arzneimittels ermöglicht oder unterstützt wird.

Die obige Aufgabe wird durch eine Kartusche gemäß Anspruch 1, 3 oder 7, durch ein System gemäß Anspruch 15 oder durch ein Verfahren gemäß Anspruch 17 gelöst. Vorteilhafte Weiterbildungen sind Gegenstand der Unteransprüche.

Mittels des vorschlagsgemäßen Systems ist es vorzugsweise möglich, ein Fluid bzw. Arzneimittel zu zerstäuben bzw. an einen Nutzer bzw. Patienten insbesondere in Form eines Aerosols abzugeben.

Das vorschlagsgemäße System weist eine Abgabevorrichtung und eine Kartusche auf, insbesondere wobei die Kartusche mehrere Bestandteile eines Fluids bzw. Arzneimittels enthält und/oder fluidisch mit der Abgabevorrichtung verbunden oder verbindbar ist.

Das System ist vorzugsweise als Kit ausgebildet, wobei das Kit die Abgabevorrichtung und mindestens eine Kartusche enthält.

Ein Kit im Sinne der vorliegenden Erfindung ist insbesondere ein Verbund und/oder ein System mit der Abgabevorrichtung und mindestens einer Kartusche. Vorzugsweise bilden die Abgabevorrichtung und die Kartusche jeweils eine Komponente des Kits. Die Komponenten des Kits werden bevorzugt in einem Verbund in Verkehr gebracht, insbesondere in einer gemeinsamen Verpackung o. dgl. Es ist jedoch auch möglich, dass die genannten Komponenten einen losen Verbund zum gemeinsamen Einsatz bilden. Vorzugweise ist eine gemeinsame bzw. verbindende Komponente vorgesehen, beispielsweise eine Gebrauchsanleitung, Handlungsempfehlung oder Verweise in den Beschriftungen einer oder mehrerer der Komponenten des Kits bzw. der gemeinsamen Verpackung.

Eine Abgabevorrichtung im Sinne der vorliegenden Erfindung ist vorzugsweise eine Vorrichtung zur Verabreichung bzw. Abgabe eines Fluids bzw. Arzneimittels an einen Nutzer bzw. Patienten, vorzugsweise wobei die Applikation des Arzneimittels aural, inhalativ, intraokular, intranasal, intramuskulär, intravenös, oral, perkutan, subkutan, sublingual und/oder subkonjunktival erfolgt. Vorzugsweise ist eine Abgabevorrichtung im Sinne der vorliegenden Erfindung als Zerstäuber, Inhalator, Spritze o.dgl. ausgebildet. Die folgenden Ausführungen beziehen sich insbesondere auf Zerstäuber, gelten jedoch entsprechend auch für andere Abgabevorrichtungen.

Vorzugsweise weist die Abgabevorrichtung, insbesondere der Zerstäuber, ein Gehäuse zur Aufnahme der Kartusche und eine Pumpe auf, vorzugsweise wobei die Pumpe zur Entnahme einer Dosis des Fluids aus der Kartusche und zur Druckbeaufschlagung der entsprechenden Dosis für die Zerstäubung des Fluids ausgebildet ist.

Die vorschlagsgemäße Kartusche weist vorzugsweise einen Behälter und einen Verschluss zum fluidischen und/oder dichtenden Anschluss des Behälters an den Zerstäuber auf, vorzugsweise wobei die Kartusche, insbesondere der Behälter, mindestens zwei fluidisch voneinander getrennte, insbesondere geschlossene, Kammern aufweist, die jeweils mindestens einen Bestandteil des Fluids bzw. Arzneimittels enthalten.

Ein Aspekt der vorliegenden Erfindung liegt darin, dass die Kartusche eine - vorzugsweise von der Kartusche, insbesondere vom Behälter bzw. Verschluss, zumindest teilweise abnehmbare - Betätigungseinrichtung aufweist, wobei die Kammern durch Betätigen der Betätigungseinrichtung zueinander öffenbar bzw. fluidisch miteinander verbindbar sind, insbesondere um die in der Kartusche getrennt aufbewahrten Bestandteile für die Abgabe bzw. Zerstäubung des Fluids miteinander in Kontakt zu bringen bzw. zu mischen und/oder um - insbesondere durch Mischen der Bestandteile - das Fluid in der Kartusche bzw. in einer der Kammern zu erzeugen.

Ein Bestandteil eines Fluids bzw. Arzneimittels im Sinne der vorliegenden Erfindung ist vorzugsweise ein Stoff, insbesondere ein Reinstoff oder ein Stoffgemisch, der bzw. das - insbesondere zusammen mit einem weiteren Bestandteil - zur Herstellung bzw. Erzeugung des Fluids bzw. Arzneimittels benötigt wird. Insbesondere ist ein Arzneimittel durch Zusammenführen bzw. in Kontakt bringen und/oder Mischen bzw. Vermengen von mehreren Bestandteilen herstellbar bzw. vorbereitbar bzw. erzeugbar.

Ein Bestandteil im Sinne der vorliegenden Erfindung ist insbesondere ein Ausgangsstoff für die Erzeugung des Arzneimittels, vorzugsweise wobei der Ausgangsstoff bei der Herstellung des Arzneimittels keine chemische Reaktion eingeht bzw. nicht verbraucht wird und/oder nach der Herstellung des Arzneimittels als Bestandteil des hergestellten Arzneimittels vorliegt. Es ist jedoch auch möglich, dass der Bestandteil bzw. Ausgangsstoff bei der Herstellung des Arzneimittels eine chemische Reaktion eingeht bzw. zumindest teilweise oder vollständig verbraucht wird.

Besonders bevorzugt ist das fertige bzw. abgabebereite und/oder hergestellte bzw. erzeugte Arzneimittel ein Gemisch aus mindestens zwei Bestandteilen und/oder ein durch mindestens eine chemische Reaktion von mindestens zwei Bestandteilen erzeugtes Produkt.

Insbesondere kann ein Bestandteil eines Arzneimittels im Sinne der vorliegenden Erfindung ein Ausgangsstoff, ein insbesondere pharmazeutischer Hilfs- bzw. Zusatzstoff, wie eine - insbesondere ethanolische - Lösung, ein Lösungsmittel, ein Lösungsvermittler, ein - insbesondere wässriger - Puffer, ein Konservierungsstoff, ein Suspensionsmittel o.dgl., und/oder ein arzneilich wirksamer bzw. pharmakologisch wirkender Stoff (Wirkstoff bzw. Arzneistoff) sein und/oder einen solchen bzw. ein solches bzw. eine solche enthalten.

Beispielsweise kann der bzw. ein Wirkstoff bzw. Arzneistoff aus der Gruppe der Peptidhormone, der peptidischen Arzneistoffe, wie Insulin, Erythropoetin o.dgl., der - insbesondere kurz- und/oder langwirksamen - Anticholinergika, wie Ipratropiumbromid, Oxitropiumbromid, Aclidiniumbromid, Glycopyrroniumbromid, Tiotropiumbromid o.dgl., der- insbesondere schnell-, kurz- und/oder langwirksamen - Beta-2-Sympa-thomimetika, wie Salbutamol, Fenoterol, Terbutalin, Arformoterol, Indacaterol, Formoterol, Olodaterol, Salmeterol, Vilanterol, Tulobuterol o.dgl., der Glucocorticoide, wie Budesonid, Ciclesonid, Fluticason, Beclometason o.dgl., der Aminoglykoside, wie Tobramycin o.dgl., und/oder der Steroide stammen.

Vorzugsweise sind auch Kombinationen mehrerer Wirkstoffe bzw. Gruppen möglich. Insbesondere kann das Arzneimittel oder ein Bestandteil des Arzneimittels mehrere Wirkstoffe bzw. Wirkstoffgruppen enthalten, beispielsweise aus der Gruppe der Glucocorticoide, der Beta-2-Sympathomimetika und/oder der Anticholinergika.

Als Hilfs- bzw. Zusatzstoff kann beispielsweise Laktose, Mannitol, Lecithin, Cholesterol, Polyethylenglycol, Glycerin oder Ethanol eingesetzt werden.

Vorzugsweise unterscheiden sich die voneinander getrennt aufbewahrten Bestandteile im Hinblick auf mindestens eine insbesondere physikochemische Stoffeigenschaft, wie die chemische Struktur, die Dichte, den Aggregatzustand, die Viskosität (insbesondere sofern zwei unterschiedliche Lösungsmittel benötigt werden), den pH-Wert, das Molekulargewicht o. dgl. Insbesondere kann ein Bestandteil gelöst und ein anderer Bestandteil ungelöst vorliegen.

Besonders bevorzugt ist ein Bestandteil der mindestens zwei Bestandteile des Arzneimittels fest, vorzugsweise getrocknet, insbesondere gefriergetrocknet bzw. lyophilisiert, und/oder pulverförmig und/oder (teil-)verkapselt.

Ganz besonders bevorzugt liegt ein Bestandteil der mindestens zwei Bestandteile des Arzneimittels im Form von - insbesondere gefriergetrockneten bzw. lyophiliserten - Liposomen bzw. als - insbesondere gefriergetrocknete bzw. lyophiliserte - liposomale Formulierung vor, vorzugsweise um die Wirkdauer zu verlängern.

Vorzugsweise ist ein Bestandteil, insbesondere ein bzw. der Arzneistoff, in - insbesondere gefriergetrockneten bzw. lyophiliserten - Liposomen eingeschlossen bzw. im Inneren der Liposomen und/oder in die Membran der Liposomen eingebettet.

Beispielsweise können lipophile bzw. hydrophobe Wirkstoffe und/oder Wirkstoffe mit einer niedrige bzw. schlechten Löslichkeit in Wasser und/oder hoher bzw. guter Löslichkeit in Ethanol, wie Wirkstoffe aus der Gruppe der Glucocorticoide, in Liposomen, insbesondere in der Membran von Liposomen, eingeschlossen sein bzw. werden.

Zusätzlich oder alternativ können lipophobe bzw. hydrophile Wirkstoffe und/oder Wirkstoffe mit einer hohen bzw. guten Löslichkeit in Wasser und/oder einer niedrigen bzw. schlechten Löslichkeit in Ethanol, wie Wirkstoffe aus der Gruppe der Beta-2-Sympathomimetika, im Inneren bzw. Kern der Liposomen eingeschlossen sein bzw. werden.

Vorzugsweise ist ein (anderer) Bestandteil der mindestens zwei Bestandteile des Arzneimittels flüssig bzw. als Flüssigkeit ausgebildet. Es ist jedoch auch möglich, dass beide bzw. alle Bestandteile flüssig sind bzw. als Flüssigkeit in der Kartusche enthalten sind.

Vorzugsweise weisen die Bestandteile eine unterschiedliche (dynamische) Viskosität auf und/oder ist ein Bestandteil hochviskos bzw. weist ein Bestandteil eine (dynamische) Viskosität von mehr als 1 oder 1,5 mPa s, besonders bevorzugt mehr als 2 oder 2,5 mPa s, und/oder weniger als 50 oder 40 mPa s, besonders bevorzugt weniger als 30 oder 20 mPa s, auf. Vorzugsweise beträgt die (dynamische Viskosität) des durch Mischen der Bestandteile erzeugten Arzneimittels mehr als 1 oder 1,5 mPa s und/oder weniger als 30 oder 20 mPa s, insbesondere weniger als 15 oder 10 mPa s.

Die (dynamische) Viskosität wird vorzugsweise bei einer Temperatur von 20 °C und/oder einem Druck von 101,325 kPa und/oder gemäß DIN 53019-1:2008-09, DIN 53019-2:2001-02 und/oder DIN 53019-3:2008-09 ermittelt.

Gemäß einem weiteren, auch unabhängig realisierbaren Aspekt der vorliegenden Erfindung ist die Kartusche dazu ausgebildet, insbesondere durch Betätigen der Betätigungseinrichtung eine liposomale Formulierung als Arzneimittel bzw. ein liposomales Arzneimittel bzw. Liposomen - insbesondere in einer der Kammern der Kartusche - zu erzeugen.

Vorzugsweise enthält eine erste Kammer der Kartusche eine insbesondere ethanolische Lipidlösung als ersten Bestandteil des Arzneimittels und eine zweite Kammer der Kartusche einen wässrigen Puffer als zweiten Bestandteil, insbesondere um durch die Betätigung der Betätigungseinrichtung bzw. durch Zusammenführen der Bestandteile eine liposomale Formulierung als Arzneimittel bzw. ein liposomales Arzneimittel bzw. Liposomen zu erzeugen.

Dabei kann insbesondere vorgesehen sein, dass in der Lipidlösung und/oder in der wässrigen Phase (jeweils) mindestens ein Wirkstoff bzw. Arzneistoff enthalten ist. Beispielsweise kann in der Lipidlösung ein lipophiler bzw. hydrophober Wirkstoff, wie aus der Gruppe der Glucocorticoide, und/oder in der wässrigen Phase ein lipophober bzw. hydrophiler Wirkstoff, wie aus der Gruppe der Beta-2-Sympathomimetika, enthalten sein.

Mit der vorschlagsgemäßen Kartusche es möglich, die Bestandteile ausschließlich mittels der Kartusche bzw. unabhängig von der Abgabevorrichtung bzw. vom Zerstäuber oder anderen Vorrichtungen und/oder außerhalb der Abgabevorrichtung bzw. des Zerstäubers zu mischen, insbesondere um das Arzneimittel zu erzeugen. Dadurch wird ein besonders einfaches, hygienisches, sicheres, homogenes und/oder schnelles Mischen der Bestandteile ermöglicht oder unterstützt.

Insbesondere ist es möglich, die Bestandteile erst unmittelbar vor Verwendung der Kartusche zu mischen und/oder eine liposomale Formulierung als Arzneimittel bzw. in Liposomen eingebettete Arzneistoffe unmittelbar vor Verwendung der Kartusche bzw. des Zerstäubers mit der Kartusche zu erzeugen.

Besonders bevorzugt sind die Kammern im geschlossenen, d.h. nach außen bzw. gegenüber der Umgebung abgedichteten, insbesondere gas- und/oder flüssigkeitsdichten, Zustand der Kartusche bzw. des Behälters - insbesondere durch Betätigen der Betätigungseinrichtung - fluidisch miteinander verbindbar bzw. zueinander öffenbar. Auf diese Weise wird ein steriles bzw. hygienisches Mischen der Bestandteile ermöglicht oder unterstützt. Insbesondere wird ein Flüssigkeitsaustritt beim Mischen verhindert.

Eine Betätigungseinrichtung im Sinne der vorliegenden Offenbarung ist vorzugsweise eine konstruktive Einrichtung zum Mischen der in der Kartusche getrennt vorliegenden Bestandteile des Fluids. Insbesondere ist durch Betätigen der Betätigungseinrichtung der Mischvorgang aktivierbar bzw. initiierbar und/oder - vorzugsweise vollständig - durchführbar, ganz besonders bevorzugt unabhängig von der Abgabevorrichtung bzw. vom Zerstäuber und/oder außerhalb der Abgabevorrichtung bzw. des Zerstäubers bzw. vor Einsetzen der Kartusche in die Abgabevorrichtung bzw. den Zerstäuber.

Die Betätigungseinrichtung ist vorzugsweise manuell bzw. durch einen Nutzer, besonders bevorzugt durch Drehen, Drücken, Abnehmen und/oder Öffnen der Betätigungseinrichtung bzw. eines Betätigungselements der Betätigungseinrichtung, betätigbar, insbesondere unmittelbar vor dem erstmaligen Einsatz des Zerstäubers.

Die Betätigungseinrichtung ist vorzugsweise - insbesondere form-, kraft- und/oder stoffschlüssig - mit der Kartusche verbunden und/oder an der Kartusche befestigt. Besonders bevorzugt ist die Betätigungseinrichtung von der Kartusche bzw. dem Behälter oder dem Verschluss abnehmbar und/oder lösbar mit der Kartusche verbunden bzw. lösbar an der Kartusche befestigt.

Insbesondere ist die Betätigungseinrichtung zum Einsetzen der Kartusche in den Zerstäuber abnehmbar bzw. wird die Betätigungseinrichtung nach Mischen der Bestandteile des Fluids und vor Einsetzen der Kartusche in den Zerstäuber von der Kartusche, insbesondere dem Behälter bzw. Verschluss, abgenommen. Alternativ wird die Kartusche zusammen mit der Betätigungseinrichtung, insbesondere nach dem Mischen der Bestandteile, in den Zerstäuber eingesetzt. Insbesondere kann die Betätigungseinrichtung in die Kartusche, besonders bevorzugt in den Boden des Behälters und/oder in den Verschluss der Kartusche, integriert sein.

Die Betätigungseinrichtung ist - insbesondere nach Betätigung der Betätigungseinrichtung bzw. nach dem Öffnen bzw. fluidischen Verbinden der Kammern - vorzugsweise von dem Behälter und/oder dem Verschluss der Kartusche abnehmbar, insbesondere abdrehbar oder abziehbar, vorzugsweise um die Kartusche nach außen bzw. gegenüber der Umgebung zu öffnen und/oder in den Zerstäuber einzusetzen. Dies ermöglicht eine besonders kompakte Bauweise des Zerstäubers bzw. eine Erhöhung des Fluidvolumens, da die Betätigungseinrichtung nicht von dem Zerstäuber aufgenommen werden muss.

Vorzugsweise weist die Kartusche, insbesondere der Behälter, mindestens einen oder zwei bewegbare bzw. verschiebbare Kolben auf, vorzugsweise wobei der/die Kolben durch Betätigen der Betätigungseinrichtung in der Kartusche bzw. im Behälter verschiebbar ist/sind und/oder (jeweils) eine Wand der Kammer für die Bestandteile des Arzneimittels bildet/bilden.

Insbesondere ist bzw. sind eine der Kammern bzw. alle Kammern durch Betätigen der Betätigungseinrichtung verkleinerbar, vorzugsweise durch Verschieben der Kolben, insbesondere um die Kammern fluidisch miteinander zu verbinden und/oder die Bestandteile zu mischen.

Erfindungsgemäß sind die Kammern durch Betätigen der Betätigungseinrichtung selbsttätig, d.h. ohne ein weiteres Zutun seitens eines Nutzers bzw. Patienten, fluidisch miteinander verbindbar. Insbesondere ist einer der Bestandteile von einer in die andere Kammer durch Betätigen der Betätigungseinrichtung selbsttätig überführbar. Besonders bevorzugt wird durch Betätigen der Betätigungseinrichtung ein Mischvorgang in der Kartusche initiiert bzw. ausgelöst und/oder selbsttätig fortgeführt bzw. abgeschlossen. Auf diese Weise wird ein besonders einfaches bzw. benutzerfreundliches Mischen der Bestandteile ermöglicht oder unterstützt.

Die Betätigungseinrichtung ist erfindungsgemäß dazu ausgebildet, den/die Kolben in der Kartusche im unbetätigten Zustand der Betätigungseinrichtung in Position zu halten bzw. gegen ein Verschieben zu sichern. Erfindungsgemäß ist/sind durch Betätigen der Betätigungseinrichtung der/die Kolben in der Kartusche freigebbar, erfindungsgemäß derart, dass sich der/die Kolben selbsttätig, vorzugsweise durch eine in Richtung einer der Kammern wirkende Kraft, besonders bevorzugt durch eine - insbesondere durch unter Unterdruck in einer der Kammern erzeugte - Zugkraft, in der Kartusche verschiebt/verschieben.

Alternativ ist die Betätigungseinrichtung vorzugsweise mechanisch mit dem/den Kolben gekoppelt, insbesondere über ein Verbindungsteil, wie eine Gewindestange, um den/die Kolben zu verschieben bzw. mindestens eine der Kammern zu verkleinern. Gemäß einem weiteren, auch unabhängig realisierbaren Aspekt der vorliegenden Erfindung ist die Abgabevorrichtung bzw. der Zerstäuber dazu ausgebildet, im ungeöffneten bzw. nach außen bzw. gegenüber der Umgebung abgedichteten, insbesondere gas- und/oder flüssigkeitsdichten, Zustand der Kartusche bzw. des Behälters die Kammern - beispielsweise durch erstmaliges Betätigen der Abgabevorrichtung bzw. des Zerstäubers und/oder der Betätigungseinrichtung - fluidisch miteinander zu verbinden bzw. die Bestandteile für die Bereitstellung bzw. Zerstäubung des Fluids zu mischen. Auf diese Weise werden entsprechende Vorteile erzielt.

Mittels der vorschlagsgemäßen Abgabevorrichtung bzw. des vorschlagsgemäßen Zerstäubers ist es vorzugsweise möglich, die Kammern nach und/oder durch Einsetzen der Kartusche in die Abgabevorrichtung bzw. den Zerstäuber fluidisch miteinander zu verbinden und die Kartusche anschließend bzw. erst nach dem Mischen der Bestandteile zur Entnahme einer Dosis des in der Kartusche erzeugten Fluids bzw. nach au-ßen bzw. gegenüber der Umgebung zu öffnen.

Bei dem vorschlagsgemäßen Verfahren zur Vorbereitung bzw. Bereitstellung eines Arzneimittels für die Abgabe bzw. Zerstäubung werden mehrere zunächst fluidisch voneinander getrennte Kammern der Kartusche zueinander geöffnet bzw. fluidisch miteinander verbunden und die darin enthaltenen Bestandteile des Arzneimittels vorzugsweise vollständig gemischt, insbesondere um durch Zusammenführen bzw. Mischen der Bestandteile das Arzneimittel in der Kartusche bzw. einer der Kammern zu erzeugen, vorzugsweise wobei die Kartusche erst nach dem fluidischen Verbinden der Kammern bzw. nach dem insbesondere vollständigen Mischen der Bestandteile zur Entnahme einer Dosis des Arzneimittels bzw. nach außen bzw. gegenüber der Umgebung geöffnet und/oder in die Abgabevorrichtung bzw. den Zerstäuber eingesetzt bzw. mit der Abgabevorrichtung bzw. dem Zerstäuber fluidisch verbunden wird. Auf diese Weise werden entsprechende Vorteile realisiert.

Besonders bevorzugt ist die Kartusche nach dem Mischen bzw. vor dem Öffnen vollständig, insbesondere gas- und/oder flüssigkeitsdicht, geschlossen oder abgedichtet. Insbesondere wird erst durch das Öffnen der Kartusche eine fluidische Verbindung, insbesondere der Kammer/Kammern, nach außen bzw. zur Umgebung bzw. zur Abgabevorrichtung erzeugt

Durch das vorschlagsgemäße Verfahren wird ein besonders hygienisches bzw. steriles, zuverlässiges und/oder homogenes Mischen der Bestandteile und somit eine verbesserte Vorbereitung des Arzneimittels für die Abgabe bzw. Zerstäubung mittels der Abgabevorrichtung bzw. des Zerstäubers ermöglicht oder unterstützt

Besonders bevorzugt werden die Bestandteile des Arzneimittels außerhalb der Abgabevorrichtung bzw. des Zerstäubers und/oder unabhängig von der Abgabevorrichtung bzw. vom Zerstäuber gemischt, insbesondere durch Betätigen der Betätigungseinrichtung.

Gemäß einer ersten Variante des vorschlagsgemäßen Verfahrens wird die Betätigungseinrichtung nach dem Mischen der Bestandteile zunächst von der Kartusche, insbesondere dem Behälter und/oder dem Verschluss der Kartusche, abgenommen.

Die Kartusche wird erst anschließend - insbesondere ohne die Betätigungseinrichtung - in den Zerstäuber eingesetzt und/oder mit dem Zerstäuber fluidisch verbunden.

Gemäß einer nicht beanspruchten Variante des Verfahrens wird die Kartusche nach dem Mischen der Bestandteile zunächst außerhalb des Zerstäubers nach außen bzw. zur Entnahme einer Dosis des Fluids geöffnet, beispielsweise durch Abnehmen der Betätigungseinrichtung, und bevorzugt wird die Betätigungseinrichtung nach dem Mischen der Bestandteile zunächst von der Kartusche, insbesondere dem Behälter und/oder dem Verschluss der Kartusche, abgenommen. Vorzugsweise wird die Kartusche erst anschließend - insbesondere ohne die Betätigungseinrichtung - in den Zerstäuber eingesetzt und/oder mit dem Zerstäuber fluidisch verbunden. Bei dieser Variante des Verfahrens ist bevorzugt, durch das Entfernen bzw. Abnehmen der Betätigungseinrichtung die Kartusche zur Abgabe einer Dosis des Fluids zu öffnen.

Gemäß einer zweiten Variante des vorschlagsgemäßen Verfahrens wird die Kartusche nach dem fluidischen Verbinden der Bestandteile bzw. nach dem Mischen der Bestandteile zunächst - insbesondere ungeöffnet bzw. im nach außen bzw. gegenüber der Umgebung abgedichteten Zustand und/oder zusammen mit der Betätigungseinrichtung - in den Zerstäuber eingesetzt. Vorzugsweise wird die Kartusche erst anschließend bzw. im Zerstäuber - vorzugsweise durch erstmaliges Betätigen bzw. Spannen des Zerstäubers - zur Entnahme einer Dosis des Fluids bzw. nach außen bzw. gegenüber der Umgebung geöffnet und/oder mit dem Zerstäuber fluidisch verbunden.

Gemäß einer dritten, nicht beanspruchten Variante des Verfahrens werden die Kammern erst nach und/oder durch Einsetzen der Kartusche in den Zerstäuber fluidisch miteinander verbunden bzw. werden die Bestandteile erst nach und/oder durch Einsetzen der Kartusche in den Zerstäuber gemischt Anschließend, d.h. nach dem Mischen der Bestandteile bzw. nach dem fluidischen Verbinden der Kammern, wird die Kartusche vorzugsweise zur Entnahme einer Dosis des Fluids bzw. nach außen bzw. gegenüber der Umgebung geöffnet und/oder mit dem Zerstäuber fluidisch verbunden, beispielsweise durch erstmaliges Betätigen bzw. Spannen des Zerstäubers.

Bei allen Varianten des vorschlagsgemäßen Verfahrens wird vorzugsweise die bereits erwähnte Betätigungseinrichtung insbesondere manuell betätigt, um die Kammern fluidisch miteinander zu verbinden bzw. die Bestandteile für die Zerstäubung des Fluids zu mischen.

Das Fluid bzw. eine Dosis des Fluids ist durch bzw. nach Öffnen der Kartusche entnehmbar.

Insbesondere ist die Kartusche im geöffneten Zustand bzw. durch/nach Öffnen der Kartusche fluidisch bzw. pneumatisch mit der Umgebung verbunden und/oder nicht mehr nach außen bzw. gegenüber der Umgebung abgedichtet.

Ein Öffnen der Kartusche erfolgt erfindungsgemäß durch Öffnen, insbesondere Durchstechen, eines Siegels der Kartusche, insbesondere mittels eines Förderelements des Zerstäubers und vorzugsweise durch Abnehmen der Betätigungseinrichtung, durch Einführen eines Förderelements in die Kartusche, insbesondere in den Verschluss und/oder Behälter der Kartusche.

Das Mischen der Bestandteile bzw. das Verbinden der Kammern und/oder das (vollständige) Betätigen der Betätigungseinrichtung erfolgt zeitlich vor und bevorzugt unabhängig von dem Öffnen der Kartusche.

Ganz besonders bevorzugt wird die Kartusche nicht durch (vollständiges) Betätigen der Betätigungseinrichtung geöffnet bzw. bleibt die Kartusche trotz eines (vollständigen) Betätigens der Betätigungseinrichtung ungeöffnet bzw. nach außen bzw. gegenüber der Umgebung abgedichtet.

Insbesondere werden die Bestandteile gemischt bzw. die Kammern fluidisch miteinander verbunden bzw. wird die Betätigungseinrichtung (vollständig) betätigt, ohne die Kartusche zu öffnen.

Aufgrund dieser Unabhängigkeit ist es möglich, die Kartusche zu öffnen, ohne die Bestandteile zu mischen bzw. die Kammern fluidisch miteinander zu verbinden bzw. die Betätigungseinrichtung (vollständig) zu betätigen.

Wie bereits erläutert, werden die Bestandteile durch Betätigung der Betätigungseinrichtung vorzugsweise selbsttätig gemischt bzw. wird einer der Bestandteile selbsttätig, insbesondere durch eine durch Unterdruck in einer der Kammern erzeugte Kraft, von einer in die andere Kammer überführt.

Zusätzlich oder alternativ kann vorgesehen sein, die Kartusche nach Betätigung der Betätigungseinrichtung bzw. nach fluidischem Verbinden der Kammer derart auszurichten, dass sich die Bestandteile durch Schwerkraft mischen bzw. der erste Bestandteil durch Schwerkraft von der ersten Kammer in die zweite Kammer strömt, die den zweiten Bestandteil des Fluids enthält.

Insbesondere bei der ersten und/oder zweiten Variante des Verfahrens ist optional vorgesehen, die Betätigungseinrichtung nach der Betätigung von der Kartusche abzunehmen, um die Kartusche in den Zerstäuber einzusetzen.

Gemäß einem weiteren, auch unabhängig realisierbaren Aspekt der vorliegenden Erfindung, wird bzw. werden durch Betätigen der Betätigungseinrichtung bzw. durch Überführen eines Bestandteils aus einer der Kammern in eine andere der Kammern bzw. durch Mischen der Bestandteile eine liposomale Formulierung bzw. ein liposomal formuliertes Arzneimittel bzw. Liposomen in der Kartusche, insbesondere in einer der Kammern, erzeugt

Besonders bevorzugt wird dazu eine - insbesondere ethanolische - Lipidlösung als erster Bestandteil des Arzneimittels von einer ersten Kammer der Kartusche in eine zweite Kammer der Kartusche mit einem wässrigen Puffer als zweiten Bestandteil des Arzneimittels überführt, vorzugsweise wobei die Lipidlösung und/oder die wässrige Phase (jeweils) mindestens einen Wirkstoff enthält, wie bereits erläutert Auf diese Weise werden entsprechende Vorteile realisiert

Die oben genannten Aspekte und Merkmale der vorliegenden Erfindung sowie die sich aus den Ansprüchen und der nachfolgenden Beschreibung ergebenden Aspekte und Merkmale der vorliegenden Erfindung können grundsätzlich unabhängig voneinander, aber auch in beliebiger Kombination bzw. Reihenfolge realisiert werden.

Weitere Aspekte, Vorteile, Merkmale und Eigenschaften der vorliegenden Erfindung ergeben sich aus den Ansprüchen und der nachfolgenden Beschreibung bevorzugter Ausführungsformen anhand der Zeichnung. Es zeigt:
- Fig. 1: einen schematischen Schnitt eines bekannten Zerstäubers im ungespannten Zustand;
- Fig. 2: einen schematischen Schnitt des um 90° gedrehten Zerstäubers gemäß Fig. 1 im gespannten Zustand;
- Fig. 3: einen schematischen Schnitt einer vorschlagsgemäßen Kartusche mit zwei fluidisch voneinander getrennten Kammern gemäß einer ersten Ausführungsform;
- Fig. 4: einen schematischen Schnitt der Kartusche gemäß Fig. 3 mit fluidisch miteinander verbundenen Kammern;
- Fig. 5: einen schematischen Schnitt der Kartusche gemäß Fig. 3 mit vollständig gemischten Bestandteilen des Fluids;
- Fig. 6: einen schematischen Schnitt eines ausschnittsweise dargestellten vorschlagsgemäßen Zerstäubers mit eingesetzter Kartusche gemäß Fig. 5 im ungespannten Zustand;
- Fig. 7: einen schematischen Schnitt einer vorschlagsgemäßen Kartusche mit zwei fluidisch voneinander getrennten Kammern gemäß einer zweiten Ausführungsform;
- Fig. 8: einen schematischen Schnitt der Kartusche gemäß Fig. 7 mit fluidisch miteinander verbundenen Kammern;
- Fig. 9: einen schematischen Schnitt der um 180° gedrehten Kartusche gemäß Fig. 8;
- Fig. 10: einen schematischen Schnitt der Kartusche gemäß Fig. 9 mit vollständig gemischten Bestandteilen des Fluids;
- Fig. 11: einen schematischen Schnitt der um 180° gedrehten Kartusche gemäß Fig. 10;
- Fig. 12: einen schematischen Schnitt eines ausschnittsweise dargestellten vorschlagsgemäßen Zerstäubers mit eingesetzter Kartusche gemäß Fig. 11 im gespannten Zustand;
- Fig. 13: einen schematischen Schnitt einer vorschlagsgemäßen Kartusche mit zwei fluidisch voneinander getrennten Kammern gemäß einer dritten Ausführungsform;
- Fig. 14: einen schematischen Schnitt der Kartusche gemäß Fig. 13 mit fluidisch miteinander verbundenen Kammern;
- Fig. 15: einen schematischen Schnitt der Kartusche gemäß Fig. 14 mit abgenommener Betätigungseinrichtung; und
- Fig. 16: einen schematischen Schnitt eines vorschlagsgemäßen Zerstäubers mit eingesetzter Kartusche gemäß Fig. 15.

In den teilweise nicht maßstabsgerechten, nur schematischen Figuren werden für gleiche, gleichartige oder ähnliche Bauteile und Komponenten dieselben Bezugszeichen verwendet, wobei entsprechende oder vergleichbare Eigenschaften und Vorteile erreicht werden, auch wenn von einer wiederholten Beschreibung abgesehen wird.

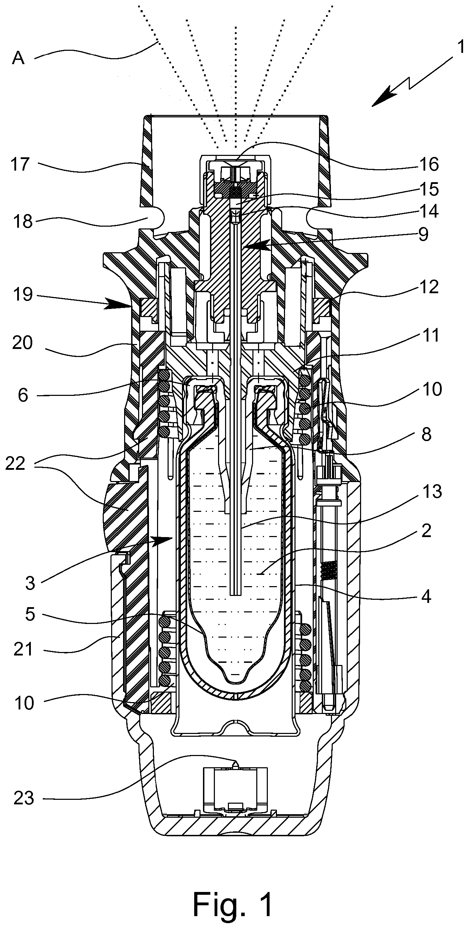

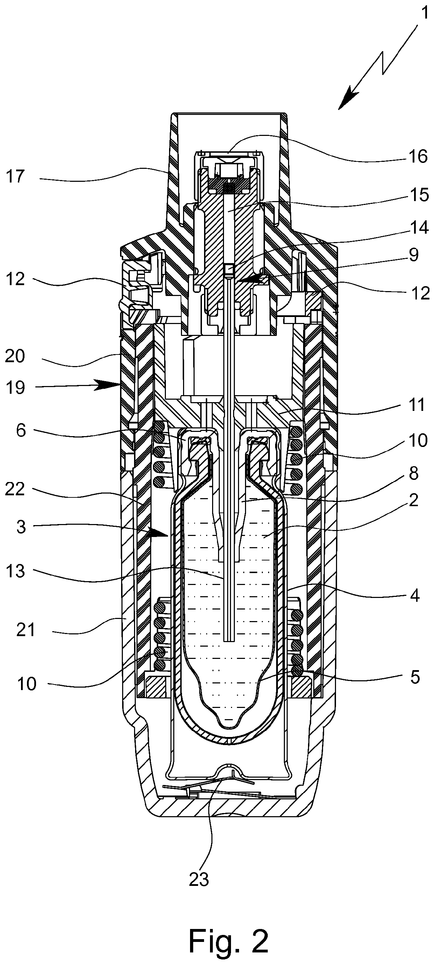

Fig. 1 und Fig. 2 zeigen ein bekanntes System mit einer bekannten Abgabevorrichtung bzw. einem bekannten Zerstäuber 1 zur Zerstäubung bzw. Abgabe eines Fluids 2, insbesondere eines Arzneimittels, wie einer pharmazeutischen Zusammensetzung, eines Medikaments o. dgl., wobei Fig. 1 die Abgabevorrichtung bzw. den Zerstäuber 1 im ungespannten Zustand bzw. bei der Abgabe einer Dosis des Fluids 2 und Fig. 2 die Abgabevorrichtung bzw. den Zerstäuber 1 im gespannten Zustand zeigt.

Die folgenden Ausführungen beziehen sich auf einen Zerstäuber, gelten jedoch entsprechend auch für andere Abgabevorrichtungen, wie Inhalatoren, Spritzen o.dgl.

Das System bzw. der Zerstäuber 1 ist vorzugsweise dazu ausgebildet, das Fluid 2 bzw. eine Dosis des Fluids 2 abzugeben bzw. zu zerstäuben.

Vorzugsweise wird ein Aerosol A gebildet bzw. abgegeben, wenn das Fluid 2 mittels des Zerstäubers 1 zerstäubt wird, wie in Fig. 1 durch gestrichelte Linien angedeutet. Das Aerosol A kann von einem Nutzer (nicht dargestellt) eingeatmet bzw. inhaliert werden.

Üblicherweise erfolgt die Anwendung des Zerstäubers 1 bzw. die Inhalation - insbesondere abhängig von den Beschwerden bzw. der Krankheit des Patienten bzw. Nutzers - mindestens einmal pro Tag, vorzugsweise mehrmals täglich, besonders bevorzugt in bestimmten Intervallen.

Der Zerstäuber 1 ist vorzugsweise als mobiler bzw. portabler Inhalator ausgebildet und/oder ohne elektrische Energie betreibbar.

Besonders bevorzugt wird der Zerstäuber 1 rein mechanisch und/oder ohne Verwendung eines Treibgases betrieben. Es sind jedoch andere konstruktive Lösungen möglich.

Das System bzw. der Zerstäuber 1 weist vorzugsweise eine - insbesondere einsetzbare und/oder austauschbare - Kartusche 3 auf, vorzugsweise wobei die Kartusche 3 das Fluid 2 beinhaltet bzw. ein Reservoir für das Fluid 2 bildet. Insbesondere ist der Zerstäuber 1 dazu ausgebildet, die Kartusche 3 teilweise oder vollständig aufzunehmen.

Fig. 1 und Fig. 2 zeigen den bekannten Zerstäuber 1 mit der schematisch dargestellten Kartusche 3.

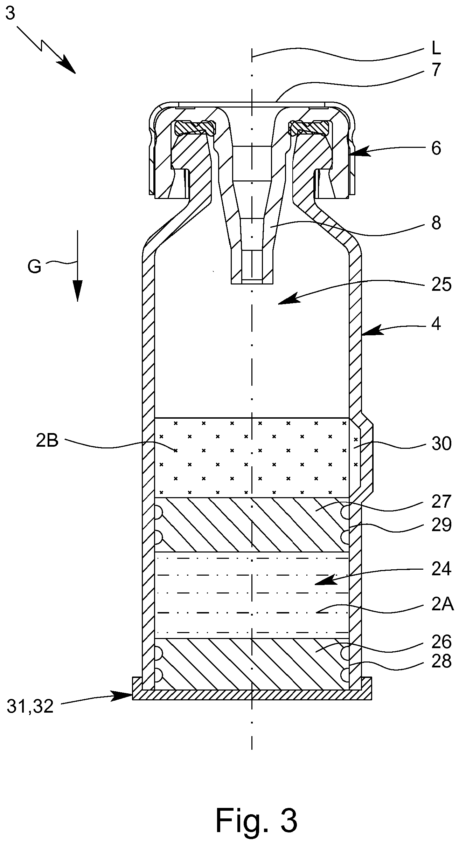

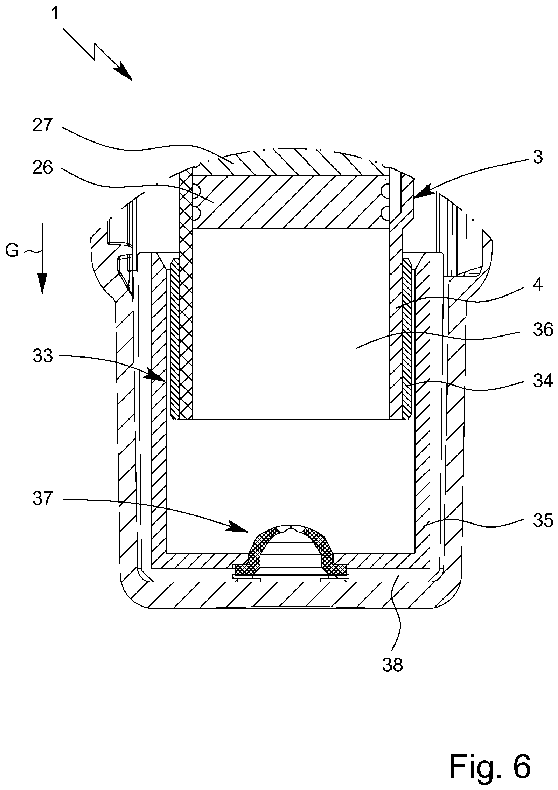

Fig. 3 bis 16 zeigen vorschlagsgemäße Kartuschen 3 bzw. Teile davon, wobei die vorschlagsgemäße Kartusche 3 mit dem Zerstäuber 1 verwendet werden kann, wie in den Fig. 6, 12 und 16 dargestellt.

Vorzugsweise sind die im Zusammenhang mit Fig. 1 und Fig. 2 beschriebenen Merkmale, Eigenschaften und Aspekte auf die bzw. den im Zusammenhang mit Fig. 3 bis 16 beschriebene Kartusche 3 bzw. beschriebenen Zerstäuber 1 anwendbar. Insbesondere kann die im Zusammenhang mit Fig. 3 bis 16 beschriebene Kartusche 3 ein oder mehrere Merkmale, Eigenschaften und Aspekte der im Zusammenhang mit Fig. 1 und Fig. 2 beschriebenen Kartusche 3 aufweisen und/oder grundsätzlich mit dem im Zusammenhang mit Fig. 1 und Fig. 2 beschriebenen Zerstäuber 1 verwendet werden.

Im Folgenden wird zunächst die bekannte Kartusche 3 anhand von Fig. 1 und Fig. 2 beschrieben.

Vorzugsweise weist die Kartusche 3 mehrere Dosen des Fluids 2 auf, insbesondere um mindestens 100, 150 oder 200 Abgabeeinheiten bzw. Dosen zur Verfügung zu stellen bzw. mindestens 100, 150 oder 200 Anwendungen zu ermöglichen.

Die Kartusche 3 beinhaltet vorzugsweise ein Volumen von mindestens 0,5 ml oder mindestens 4 ml und/oder höchstens 30 ml oder 20 ml. Die Anzahl der Dosen in der Kartusche 3 und/oder das Gesamtvolumen des Fluids 2 in der Kartusche 3 kann in Abhängigkeit vom Fluid 2, der Kartusche 3 und/oder der erforderlichen Medikation variieren. Optional ist die Anzahl der Dosen bzw. der Betätigungen beschränkt.

Vorzugsweise weist der Zerstäuber 1 eine Zähleinrichtung auf, die die Anzahl der Betätigungen des Zerstäubers 1 zählt und/oder anzeigt, vorzugsweise indem das Spannen des Zerstäubers 1 erfasst wird. Optional weist der Zerstäuber 1 bzw. die Zähleinrichtung eine Blockiereinrichtung auf, die ein Betätigen des Zerstäubers 1 bei Erreichen einer vorbestimmten Anzahl von Betätigungen blockiert.

Vorzugsweise ist der Zerstäuber 1 dazu ausgebildet, mindestens 1 µl oder 5 µl und/oder höchstens 100 µl, 50 µl oder 20 µl des Fluids 2 bei Betätigung bzw. Nutzung des Zerstäubers 1 und/oder mit einer Dosis des Fluids 2 zu zerstäuben bzw. abzugeben.

Der Zerstäuber 1 bzw. die Kartusche 3 weist vorzugsweise einen insbesondere starren Behälter 4 und/oder einen optionalen, vorzugsweise flexiblen bzw. kollabierbaren, Beutel 5 auf, insbesondere wobei der Behälter 4 bzw. der Beutel 5 das Fluid 2 beinhaltet. Der optionale Beutel 5 ist vorzugsweise innerhalb des Behälters 4 angeordnet und/oder im bzw. vom Behälter 4 gehalten.

Vorzugsweise ist die Kartusche 3 herausnehmbar bzw. austauschbar, wie beispielsweise in der WO 2012/162305 A1 offenbart.

Vorzugsweise ist die Anzahl der Anwendungen der Kartusche 3 bzw. die Anzahl der Dosen des Fluids 2 aus derselben bzw. der im Zerstäuber 1 eingesetzten Kartusche 3 beschränkt.

Vorzugsweise weist die Kartusche 3 dazu eine Zähleinrichtung auf, insbesondere wobei die Zähleinrichtung die Anzahl der Betätigungen des Zerstäubers 1 mit derselben Kartusche 3 zählt und/oder anzeigt, besonders bevorzugt indem das Spannen des Zerstäubers 1 erfasst wird. Optional weist die Kartusche 3 bzw. die Zähleinrichtung eine Blockiereinrichtung auf, die ein Betätigen des Zerstäubers 1 mit derselben Kartusche 3 bei Erreichen einer vorbestimmten Anzahl von Betätigungen mit derselben Kartusche 3 blockiert. Die WO 2015/169431 A2 offenbart eine derartige, vorzugsweise der Kartusche 3 zugeordnete Zähleinrichtung.

Optional ist die Anzahl von Kartuschen 3, die mittels desselben Zerstäubers 1 verwendet werden können, beschränkt, beispielsweise auf eine maximale Anzahl von vier, fünf oder sechs Kartuschen 3, vorzugsweise mittels der bzw. den bereits erwähnten Zähleinrichtung(en) bzw. Blockiereinrichtung(en). Die WO 2012/162305 A1 offenbart eine Beschränkung der Anzahl von Kartuschen 3, die mit demselben Zerstäuber 1 verwendet werden können.

Die Kartusche 3, insbesondere der Behälter 4, ist vorzugsweise länglich, starr und/oder zumindest im Wesentlichen zylindrisch bzw. als Hohlzylinder ausgebildet. Es sind jedoch auch andere konstruktive Lösungen möglich, insbesondere bei denen die Kartusche 3 bzw. der Behälter 4 zumindest im Wesentlichen bzw. zumindest teilweise kugelförmig ausgebildet ist.

Vorzugsweise ist der Behälter 4 an einem axialen Ende offen und/oder weist der Behälter 4 endseitig bzw. an einem axialen Ende eine Öffnung zur Entnahme des Fluids 2 auf.

Vorzugsweise ist der Zerstäuber 1 öffenbar und/oder kann die Kartusche 3 durch Öffnen des Zerstäubers 1 in den Zerstäuber 1 eingesetzt bzw. ausgetauscht werden, vorzugsweise von unten.

Vorzugsweise ist die Kartusche 3 zumindest im Auslieferungszustand nach außen bzw. gegenüber der Umgebung geschlossen und/oder abgedichtet, insbesondere gas- und/oder flüssigkeitsdicht.

Vorzugsweise weist die Kartusche 3 einen Verschluss 6 auf, vorzugsweise wobei der Verschluss 6 form-, kraft- und/oder stoffschlüssig mit dem Behälter 4 verbunden ist und/oder den Behälter 4 axial verschließt.

Insbesondere ist der Verschluss 6 dazu ausgebildet ist, den Behälter 4 bzw. den Beutel 5 - insbesondere axial bzw. am offenen Ende des Behälters 4 - zu verschlie-βen bzw. abzudichten, insbesondere gas- und/oder flüssigkeitsdicht.

Nachfolgend wird das Ende des Behälters 4, an dem der Verschluss 6 angeordnet ist, bzw. das Ende der Kartusche 3, das den Verschluss 6 aufweist, als das "obere Ende" bezeichnet. Entsprechend ist das dem Verschluss 6 (axial) gegenüberliegende Ende der Kartusche 3 bzw. des Behälters 4 das "untere Ende". Vorzugsweise entspricht dies der bevorzugten Orientierung der Kartusche 3 im Zerstäuber 1 bei der Verwendung. Es ist jedoch vorzugsweise auch möglich, den Zerstäuber 1 bzw. die Kartusche 3 in anderen Orientierungen zu verwenden.

Vorzugsweise ist der Verschluss 6 dazu ausgebildet, den Behälter 4 fluidisch mit dem Zerstäuber 1 zu verbinden, insbesondere wenn die Kartusche 3 in den Zerstäuber 1 eingesetzt ist bzw. wird.

Die Kartusche 3, insbesondere der Verschluss 6, weist erfindungsgemäß öffenbares, vorzugsweise durchstechbares, Siegel 7 und/oder einen insbesondere trichterförmigen und/oder konischen Anschluss 8 auf.

Das Siegel 7 ist beispielsweise in Fig. 3 gezeigt, in der die vorschlagsgemäße Kartusche 3 gemäß einer ersten Ausführungsform im geschlossenen Zustand bzw. im Auslieferungszustand dargestellt ist.

Das Siegel 7 ist vorzugsweise als Dichtung bzw. Barriere ausgebildet und/oder dichtet den Behälter 4, Beutel 5 und/oder Anschluss 8 - insbesondere axial - ab, vorzugsweise zumindest vor Einsetzen der Kartusche 3 in den Zerstäuber 1 bzw. vor Durchstechen des Siegels 7. Insbesondere bietet das Siegel 7 einen Verdunstungsschutz.

Vorzugsweise ist das Siegel 7 als Folie, Membran, Diaphragma, Septum o. dgl. ausgebildet.

Der bekannte Zerstäuber 1 gemäß Fig. 1 und Fig. 2 weist vorzugsweise eine insbesondere mechanische Pumpe 9 auf, vorzugsweise wobei die Pumpe 9 als Abgabe- bzw. Druckmechanismus ausgebildet ist. Insbesondere ist die Pumpe 9 dazu ausgebildet, das Fluid 2 bzw. eine Dosis des Fluids 2 aus dem Behälter 4 bzw. Beutel 5 zu saugen, unter Druck zu setzen, innerhalb des Zerstäubers 1 nach oben zu fördern und/oder zu zerstäuben bzw. abzugeben, vorzugsweise wobei das Volumen der mittels der Pumpe 9 geförderten Dosis des Fluids 2 vordefiniert und optional anpassbar ist.

Vorzugsweise ist das Fluid 2 bzw. eine Dosis des Fluids 2 mittels der Pumpe 9 aus der Kartusche 3, insbesondere dem Behälter 4 bzw. Beutel 5, entnehmbar bzw. heraussaugbar, wenn der Zerstäuber 1 gespannt bzw. geladen wird. Anschließend kann das entnommene Fluid 2 bzw. die Dosis des Fluids 2 abgegeben, insbesondere unter Druck gesetzt und/oder zerstäubt werden, insbesondere mittels der mechanischen Energie, die während des Spannens bzw. Ladens des Zerstäubers 1 gespeichert wurde, wie im Folgenden erläutert wird.

Der Zerstäuber 1 weist vorzugsweise einen Energiespeicher auf, der während des Ladens bzw. Spannens des Zerstäubers 1 geladen bzw. gespannt wird. Die im Energiespeicher gespeicherte Energie wird zur Abgabe bzw. Zerstäubung des Fluids 2 bzw. einer Dosis des Fluids 2, die während des Spannens bzw. Ladens der Kartusche 3, insbesondere dem Behälter 4 bzw. Beutel 5, entnommen wurde, verwendet. Der übliche Gebrauch des Zerstäubers 1 beinhaltet folglich einen Lade- bzw. Spannvorgang und einen (anschließenden) Abgabe- bzw. Zerstäubungsvorgang.

Vorzugsweise weist der Zerstäuber 1 eine Antriebsfeder 10 auf, vorzugsweise wobei die Antriebsfeder 10 den Energiespeicher oder einen Teil des Energiespeichers bildet. Besonders bevorzugt ist die Antriebsfeder 10 - insbesondere beim Lade- bzw. Spannvorgang des Zerstäubers 1 - spannbar, besonders bevorzugt stauchbar, vorzugsweise um Energie für die (anschließende) Abgabe des Fluids 2 bzw. einer Dosis des Fluids 2 bzw. für den Abgabe- bzw. Zerstäubungsvorgang zu speichern.

Der Zerstäuber 1 weist vorzugsweise ein insbesondere ringförmiges Sperrelement 12 auf, insbesondere wobei das Sperrelement 12 dazu ausgebildet ist, ein - versehentliches oder unmittelbares - Entspannen der gespannten Antriebsfeder 10 zu blockieren bzw. zu verhindern. Vorzugsweise ist das Sperrelement 12 mit einem insbesondere von außen zugänglichen Knopf zur vorzugsweise manuellen Betätigung versehen. Insbesondere ist durch Betätigen des Sperrelements 12 bzw. dessen Knopfes die Antriebsfeder 10 freigebbar bzw. entspannbar, insbesondere derart, dass der Abgabe- bzw. Zerstäubungsvorgang durchgeführt wird.

Der Zerstäuber 1, insbesondere die Pumpe 9, weist vorzugsweise einen Halter 11 auf, vorzugsweise wobei der Halter 11 dazu ausgebildet ist, die Kartusche 3, insbesondere den Verschluss 6 der Kartusche 3, und/oder die dem Halter 11 zugeordnete Antriebsfeder 10 bzw. den dem Halter 11 zugeordneten Teil der Antriebsfeder 10 und/oder das Sperrelement 12 insbesondere form- und/oder kraftschlüssig zu halten.

Der Halter 11 ist vorzugsweise dazu ausgebildet, die Kartusche 3 lösbar zu halten, insbesondere derart, dass die Kartusche 3 herausgenommen bzw. herausgezogen oder ausgewechselt werden kann.

Das Sperrelement 12 ist vorzugsweise dazu ausgebildet, den Halter 11 insbesondere form- und/oder kraftschlüssig gegen eine axiale Bewegung zu sichern bzw. zu sperren. Insbesondere kann das Sperrelement 12 manuell betätigt werden, um den Halter 11 und somit die Antriebsfeder 10 freizugeben.

Der Zerstäuber 1, insbesondere die Pumpe 9, weist vorzugsweise ein Förderelement 13, ein Einwegventil 14, eine Druckkammer 15 und/oder eine Düse 16 zur Zerstäubung des Fluids 2 bzw. einer Dosis des Fluids 2 auf.

Vorzugsweise ist das Förderelement 13 als länglicher Hohlzylinder, insbesondere als Nadel, Kanüle o. dgl., ausgebildet und/oder weist das Förderelement 13 ein spitz zulaufendes bzw. scharfes Ende auf, insbesondere um das Siegel 7 zu durchtrennen. Vorzugsweise ist das Förderelement 13 starr und/oder aus Metall, insbesondere aus rostfreiem Edelstahl, hergestellt.

Die vorzugsweise vollständig eingesetzte Kartusche 3 ist vorzugsweise mittels des Halters 11 im Zerstäuber 1 derart fixiert bzw. gehalten, dass das Förderelement 13 die Kartusche 3, insbesondere den Behälter 4 bzw. Beutel 5, mit dem Zerstäuber 1, insbesondere der Pumpe 9, (fluidisch) verbindet.

Besonders bevorzugt durchdringt das Förderelement 13 das Siegel 7 und/oder ragt das Förderelement 13 zumindest teilweise in die Kartusche 3, insbesondere in den Behälter 4 bzw. Beutel 5, wenn die Kartusche 3 in den Zerstäuber 1 eingesetzt ist bzw. wird.

Insbesondere ist die Kartusche 3, besonders bevorzugt der Behälter 4 bzw. Beutel 5, durch Einführen des Förderelements 13 in die Kartusche 3, insbesondere in den Behälter 4 bzw. Beutel 5 und/oder in den Verschluss 6 bzw. Anschluss 8, und/oder durch Durchstechen des Siegels 7 mittels des Förderelements 13 öffenbar bzw. fluidisch mit dem Zerstäuber 1 bzw. der Pumpe 9 verbindbar.

Wenn die Antriebsfeder 10 beim Spannvorgang des Zerstäubers 1 gespannt, insbesondere gestaucht, wird, werden der Halter 11, die Kartusche 3 und das Förderelement 13 vorzugsweise zusammen nach unten bzw. in Richtung des Bodens der Kartusche 3 bewegt.

Durch die Bewegung des Förderelements 13 nach unten bzw. in Richtung des Bodens der Kartusche 3 wird das Volumen der Druckkammer 15 der Pumpe 9 vergrö-βert bzw. der Druck innerhalb der Druckkammer 15 reduziert, insbesondere derart, dass das Fluid 2 aus der Kartusche 3, insbesondere dem Behälter 4 bzw. Beutel 5, mittels des Förderelements 13 insbesondere über das Einwegventil 14 in die Pumpe 9 bzw. Druckkammer 15 gesaugt wird.

Vorzugsweise verrastet am Ende des Spannvorgangs der Halter 11 mit dem Sperrelement 12 und/oder liegt der Halter 11 am Ende des Spannvorgangs am Sperrelement 12 an, insbesondere derart, dass die Antriebsfeder 10 gespannt bleibt. In diesem Zustand ist der Zerstäuber 1 gespannt bzw. aktiviert und somit abgabebereit.

Der anschließende Abgabevorgang wird durch Betätigen des Zerstäubers 1, insbesondere des Sperrelements 12, eingeleitet. Insbesondere wird durch Betätigen des Zerstäubers 1, insbesondere des Sperrelements 12, der Halter 11 bzw. die Antriebsfeder 10 freigegeben, insbesondere derart, dass sich die Antriebsfeder 10 zumindest teilweise entspannen kann.

Durch die Entspannung der Antriebsfeder 10 während des Abgabevorgangs, also nach Betätigung des Zerstäubers 1 bzw. des Sperrelements 12, bewegt sich das Förderelement 13 vorzugsweise zusammen mit dem nun geschlossenen Einwegventil 14 zurück in Richtung der Druckkammer 15, in Fig. 1 und Fig. 2 nach oben, und verringert auf diese Weise das Volumen der Druckkammer 15. Aufgrund des während des Abgabevorgangs geschlossenen Einwegventils 14 wird das in der Druckkammer 15 eingebrachte Fluid 2 bzw. die dort eingebrachte Dosis des Fluids 2 unter Druck gesetzt. Das Einwegventil 14 dient beim Abgabevorgang folglich vorzugsweise als Druckstößel bzw. -kolben.

Der auf diese Weise erzeugte Druck zwingt das Fluid 2 bzw. die entnommene Dosis des Fluids 2 durch die Düse 16, wodurch die Zerstäubung des Fluids 2 bzw. der Dosis des Fluids 2 erfolgt bzw. das Aerosol A gebildet wird, wie in Fig. 1 durch Strichlinien angedeutet.

Vorzugsweise wird durch die Pumpe 9, insbesondere die Antriebsfeder 10, ein Druck von mindestens 5 MPa oder 10 MPa und/oder von höchstens 300 MPa oder 250 MPa, insbesondere von höchstens 100 MPa oder 50 MPa, besonders bevorzugt von zumindest im Wesentlichen 30 MPa, erzeugt.

Auf diese Weise wird das Fluid 2 zum Aerosol A zerstäubt, vorzugsweise wobei der (durchschnittliche) aerodynamische Durchmesser der Tropfen des Aerosols A weniger als 20 µm, vorzugsweise mindestens 3 µm und/oder höchstens 10 µm, beträgt.

Vorzugsweise weist der auf diese Weise generierte Sprühstrahl des Aerosols A einen (Sprüh-) Winkel zwischen 20° und 160°, besonders bevorzugt zwischen 80° und 100°, auf.

Die genannten Werte gelten insbesondere auch für den vorschlagsgemäßen Zerstäuber 1.

Ein Nutzer bzw. Patient kann das auf diese Weise generierte Aerosol A inhalieren, vorzugsweise während gleichzeitig Luft über ein Mundstück 17, insbesondere durch mindestens eine optional vorgesehene Belüftungsöffnung 18, eingesaugt wird.

Der Zerstäuber 1 weist vorzugsweise ein Gehäuse 19 zur Aufnahme der Kartusche 3 auf, vorzugsweise wobei das Gehäuse 19 mehrteilig aufgebaut ist und/oder ein oberes Gehäuseteil 20, ein unteres Gehäuseteil 21 und/oder ein inneres Gehäuseteil 22 aufweist.

Das innere Gehäuseteil 22 und/oder das untere Gehäuseteil 21 sind/ist vorzugsweise relativ zum oberen Gehäuseteil 20 drehbar. Insbesondere ist das untere Gehäuseteil 21 manuell betätigbar und/oder freigebbar mit dem inneren Gehäuseteil 22 verbunden bzw. auf das innere Gehäuseteil 22 aufgesteckt, insbesondere über ein entsprechendes Halteelement des inneren Gehäuseteils 22.

Das Gehäuse 19 ist vorzugsweise - insbesondere bodenseitig - öffenbar, insbesondere um die Kartusche 3 in den Zerstäuber 1 bzw. das Gehäuse 19 - insbesondere von unten - einzusetzen bzw. auszutauschen. Insbesondere kann das untere Gehäuseteil 21 vom Zerstäuber 1, insbesondere vom inneren Gehäuseteil 22, zum Einsetzen bzw. Auswechseln der Kartusche 3 abgenommen werden.

Vorzugsweise wird die Kartusche 3 in den Zerstäuber 1 eingesetzt und im Gehäuse 19 geöffnet bzw. mit dem Zerstäuber 1, insbesondere der Pumpe 9, fluidisch verbunden, vorzugsweise bevor das Gehäuse 19 geschlossen und/oder der untere Gehäuseteil 21 mit dem inneren Gehäuseteil 22 bzw. dem oberen Gehäuseteil 20 verbunden wird. Insbesondere wird die Kartusche 3 durch Einsetzen der Kartusche 3 in den Zerstäuber 1 bzw. in das Gehäuse 19 geöffnet und/oder fluidisch mit dem Zerstäuber 1, insbesondere der Pumpe 9, verbunden. Alternativ kann die Kartusche 3 durch (vollständiges) Schließen des Gehäuses 19 bzw. (vollständiges) Verbinden des unteren Gehäuseteils 21 mit dem oberen Gehäuseteil 20 bzw. dem inneren Gehäuseteil 22 geöffnet und/oder fluidisch mit dem Zerstäuber 1, insbesondere der Pumpe 9, verbunden werden, besonders bevorzugt automatisch oder zumindest im Wesentlichen zeitgleich. Alternativ wird die Kartusche 3 erst geöffnet bzw. fluidisch mit dem Zerstäuber 1 bzw. der Pumpe 9 verbunden, wenn der Zerstäuber 1 erstmalig mit der eingesetzten Kartusche 3 gespannt wird.

Vorzugsweise ist der Zerstäuber 1 bzw. die Antriebsfeder 10 manuell betätigbar, aktivierbar bzw. spannbar, insbesondere durch Aktivierung bzw. Drehen des unteren Gehäuseteil 21. Hier sind jedoch auch andere Lösungen möglich.

Vorzugsweise ist das untere Gehäuseteil 21 zusammen mit dem inneren Gehäuseteil 22 betätigbar, insbesondere relativ zum oberen Gehäuseteil 20 drehbar, um den Zerstäuber 1 zu spannen.

Das innere Gehäuseteil 22 dient vorzugsweise als Getriebe und/oder ist vorzugsweise zur Umwandlung der Rotationsbewegung des unteren Gehäuseteils 21 in eine Axial- bzw. Hubbewegung des Halters 11 bzw. der Kartusche 3 ausgebildet. Insbesondere wird mittels des inneren Gehäuseteils 22 die Antriebsfeder 10 gespannt, besonders bevorzugt in axialer Richtung gestaucht.

Das untere Gehäuseteil 21 formt vorzugsweise einen Boden des Zerstäubers 1 und/oder nimmt die Kartusche 3 zumindest teilweise axial auf.

Während des Spannvorgangs werden die Kartusche 3 und der Halter 11 vorzugsweise axial nach unten bzw. in Richtung des unteren Gehäuseteils 21 bewegt, bis die Kartusche 3 in eine Endposition/Spannposition gelangt, wie in Fig. 2 dargestellt.

In dem aktivierten bzw. gespannten Zustand bzw. in der Endposition steht die Antriebsfeder 10 unter Spannung, vorzugsweise wobei die Antriebsfeder 10 mittels des Sperrelements 12 gegen ein Entspannen gesichert bzw. gesperrt wird, wie bereits erläutert.

Während des Abgabevorgangs wird die Kartusche 3 durch die (Kraft der) Antriebsfeder 10 zurück in ihre ursprüngliche bzw. nicht gespannte Position bzw. Ruheposition gebracht, wie in Fig. 1 dargestellt. Die Kartusche 3 führt folglich eine Axial- bzw. Hubbewegung im Zerstäuber 1 während des Spann- und Abgabevorgangs aus.

Vorzugsweise weist der Zerstäuber 1, insbesondere das Gehäuse 19, besonders bevorzugt das untere Gehäuseteil 21, ein Belüftungsmittel 23, wie ein Anstechelement, auf, vorzugsweise wobei das Belüftungsmittel 23 dazu ausgebildet ist, die Kartusche 3, insbesondere den Behälter 4, zu öffnen bzw. anzustechen, wenn die Kartusche 3 erstmalig mit dem Belüftungsmittel 23 verbunden wird bzw. in Kontakt gelangt. Auf diese Weise kann Luft in die Kartusche 3, insbesondere in den Behälter 4, gelangen und ein Druckausgleich bei der Entnahme des Fluids 2 bzw. einer Dosis des Fluids 2 aus der Kartusche 3 stattfinden.

Wie bereits erläutert, sind jedoch auch andere konstruktive Lösungen möglich, insbesondere bei denen der optionale Beutel 5 weggelassen wird. Das Belüftungsmittel 23 kann dann gegebenenfalls entfallen. Derartige Ausführungsformen ohne Beutel 5 werden im Folgenden anhand von Fig. 3 bis 16 beschrieben.

Die Kartusche 3 gemäß der verschiedenen, in Fig. 3 bis 16 gezeigten Ausführungsformen ist als Mehrkammer-Kartusche, insbesondere Doppelkammer-Kartusche, ausgebildet und/oder weist vorzugsweise mehrere bzw. mindestens zwei Kammern 24, 25 auf, vorzugsweise wobei die Kammern 24, 25 - zumindest im Auslieferungszustand der Kartusche 3 - fluidisch voneinander getrennt, voneinander beabstandet und/oder jeweils geschlossen sind.

Das Fluid 2 ist vorzugsweise aus mehreren Komponenten bzw. Bestandteilen 2A, 2B zusammengesetzt bzw. zusammensetzbar.

Die Bestandteile 2A, 2B des Fluids 2 sind - zumindest im Auslieferungszustand der Kartusche 3 - vorzugsweise fluidisch voneinander getrennt bzw. separat und/oder beabstandet zueinander in der Kartusche 3, insbesondere im Behälter 4, aufbewahrt bzw. enthalten, insbesondere um die Haltbarkeit des Fluids 2 zu erhöhen. Insbesondere ist das Fluid 2 erst durch Zusammenführen bzw. fluidisches Verbinden und/oder Mischen der einzelnen Bestandteile 2A, 2B herstellbar bzw. für die Abgabe bzw. Zerstäubung vorbereitet.

Vorzugsweise enthalten die Kammern 24, 25 jeweils mindestens einen der Bestandteile 2A, 2B des Fluids 2. Insbesondere enthält die erste Kammer 24 den ersten Bestandteil 2A und/oder die zweite Kammer 25 den zweiten Bestandteil 2B.

Bei den dargestellten Ausführungsformen weist die Kartusche 3 zwei Kammern 24, 25 auf. Es sind jedoch auch konstruktive Lösungen möglich, bei denen die Kartusche 3 mehr als zwei Kammern, beispielsweise drei oder vier Kammern, aufweist, insbesondere um mehr als zwei Bestandteile, beispielsweise drei oder vier Bestandteile, des Fluids 2 getrennt voneinander aufzubewahren.

Wie eingangs bereits erläutert, können die Bestandteile 2A, 2B fest oder flüssig sein. Insbesondere kann ein Bestandteil der Bestandteile 2A, 2B, vorzugsweise der zweite Bestandteil 2B, fest und ein anderer Bestandteil der Bestandteile 2A, 2B, vorzugsweise der erste Bestandteil 2A, flüssig sein. Es ist jedoch auch möglich, dass beide bzw. alle Bestandteile 2A, 2B flüssig sind.

Die Kartusche 3 bzw. der Behälter 4 ist vorzugsweise länglich und/oder zumindest im Wesentlichen zylindrisch, insbesondere als länglicher Hohlzylinder, ausgebildet. Insbesondere weist die Kartusche 3 bzw. der Behälter 4 eine Längsachse L auf, vorzugsweise wobei die Kartusche 3 bzw. der Behälter 4 um die Längsachse L zumindest im Wesentlichen rotations- bzw. zylindersymmetrisch ist und/oder die Längsachse L eine Rotationsachse der Kartusche 3 bzw. des Behälters 4 bildet. Vorzugsweise verläuft die Längsachse L mittig durch die Kartusche 3 bzw. den Behälter 4.

Vorzugsweise sind die Kammern 24, 25 im bzw. durch den Behälter 4 gebildet. Insbesondere begrenzt der Behälter 4 bzw. die Wandung des Behälters 4 die Kammern 24, 25 radial bzw. seitlich. Hier sind jedoch auch andere Lösungen möglich.

Vorzugsweise sind die Kammern 24, 25 übereinander angeordnet, wie in Fig. 3 dargestellt. Insbesondere verläuft die Längsachse L der Kartusche 3 zumindest im Wesentlichen mittig sowohl durch die erste Kammer 24 als auch durch die zweite Kammer 25 und/oder sind die Kammern 24, 25 axial zueinander beabstandet.

Vorzugsweise ist die zweite Kammer 25 zwischen der ersten Kammer 24 und dem Verschluss 6 angeordnet und/oder ist die zweite Kammer 25 näher am Verschluss 6 der Kartusche 3 angeordnet als die erste Kammer 24.

Es sind jedoch grundsätzlich auch konstruktive Lösungen möglich, bei denen die Kammern 24, 25 nebeneinander und/oder auf zumindest im Wesentlichen gleicher Höhe in der Kartusche 3 angeordnet, radial zueinander versetzt und/oder zu der Längsachse L (unterschiedlich) beabstandet sind.

Die Kammern 24, 25 sind vorzugsweise zumindest im Auslieferungszustand der Kartusche 3 bzw. temporär fluidisch voneinander getrennt, und/oder jeweils geschlossen, vorzugsweise flüssigkeits- und/oder gasdicht, insbesondere derart, dass sich die Bestandteile 2A, 2B des Fluids 2 nicht mischen bzw. nicht miteinander in Kontakt gelangen können und/oder die Bestandteile 2A, 2B fluidisch voneinander getrennt bzw. separat und/oder beabstandet zueinander in der Kartusche 3 aufbewahrt werden.

Vorzugsweise sind die Volumina der Kammern 24, 25 unterschiedlich. Besonders bevorzugt ist das Volumen der zweiten Kammer 25 größer als das Volumen der ersten Kammer 24, vorzugsweise um mindestens das 1,2- oder 1,5-fache, besonders bevorzugt um mindestens das 2,0- oder das 2,5-fache. Insbesondere ist die zweite Kammer 25 dazu ausgebildet, das (gesamte) Volumen der ersten Kammer 24 aufzunehmen, wie im Folgenden noch näher erläutert wird.

Vorzugsweise ist die erste Kammer 24 im Auslieferungszustand der Kartusche 3 zumindest im Wesentlich vollständig mit dem ersten Bestandteil 2A gefüllt und/oder ist mindestens 90 % des Volumens der ersten Kammer 24 durch den ersten Bestandteil 2A eingenommen.

Vorzugsweise ist die zweite Kammer 25 im Auslieferungszustand der Kartusche 3 nur teilweise mit dem zweiten Bestandteil 2B gefüllt und/oder ist höchstens 80 % oder 70 %, insbesondere höchstens 60 % oder 50 %, des Volumens der zweiten Kammer 25 durch den zweiten Bestandteil 2B eingenommen. Besonders bevorzugt weist die zweite Kammer 25 im Auslieferungszustand der Kartusche 3 genügend Platz bzw. Kapazität auf, um vorzugsweise den gesamten ersten Bestandteil 2A, mindestens jedoch 80 % oder 90 % des ersten Bestandteils 2A, aufzunehmen.

Der Auslieferungszustand der Kartusche 3 ist vorzugsweise der Zustand der Kartusche 3, in dem die Kartusche 3 ungeöffnet bzw. nach außen bzw. gegenüber der Umgebung abgedichtet und/oder unbetätigt ist, wie in Fig. 3, 7 und 13 dargestellt. Insbesondere ist das Volumen der ersten Kammer 24 und/oder das Volumen der zweiten Kammer 25 im Auslieferungszustand der Kartusche 3 maximal bzw. das maximale Volumen, das die erste Kammer 24 bzw. zweite Kammer 25 einnehmen kann.

Vorzugsweise ist mindestens eine der Kammern 24, 25, insbesondere sowohl die erste Kammer 24 als auch die zweite Kammer 25, verkleinerbar.

Vorzugsweise weist die Kartusche 3 mindestens einen bewegbaren bzw. verschiebbaren Kolben 26 auf, vorzugsweise wobei durch Bewegen bzw. Verschieben des Kolbens 26 das Volumen der ersten Kammer 24 verkleinerbar ist.

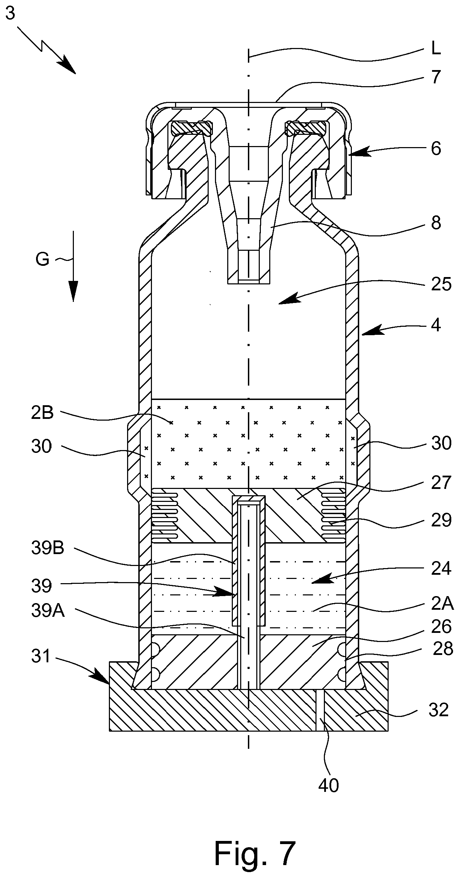

Bei der ersten, in Fig. 3 bis Fig. 6 dargestellten und der zweiten, in Fig. 7 bis Fig. 12 dargestellten Ausführungsform weist die Kartusche 3, insbesondere der Behälter 4, mehrere Kolben 26, 27 bzw. einen ersten Kolben 26 und einen zweiten Kolben 27 auf, vorzugsweise wobei die Kolben 26, 27 axial in der Kartusche 3 bzw. im Behälter 4 bewegbar bzw. verschiebbar sind und/oder die Kartusche 3, insbesondere der Behälter 4 und/oder der Verschluss 6, einen Zylinder für die Kolben 26, 27 aufweist oder bildet.

Vorzugsweise sind die Kolben 26, 27 seitlich bzw. radial vom Behälter 4 geführt und/oder liegen die Kolben 26, 27 seitlich bzw. radial - insbesondere unmittelbar - am Behälter 4 bzw. der Wandung des Behälters 4 an.

Vorzugsweise sind die Kolben 26, 27 entlang der Längsachse L bewegbar bzw. verschiebbar und/oder axial zueinander beabstandet bzw. übereinander angeordnet.

Vorzugsweise ist der zweite Kolben 27 zwischen dem Verschluss 6 und dem ersten Kolben 26 und/oder näher am Verschluss 6 als der erste Kolben 26 angeordnet.

Vorzugsweise begrenzen die Kolben 26, 27 die Kammern 24, 25 axial und/oder bilden die Kolben 26, 27 (jeweils) mindestens eine Wand der Kammern 24, 25.

Vorzugsweise begrenzt der erste Kolben 26 die erste Kammer 24 nach unten und/oder bildet der erste Kolben 26 einen Boden der ersten Kammer 24. Vorzugsweise begrenzt der zweite Kolben 27 die erste Kammer 24 nach oben und/oder die zweite Kammer 25 nach unten und/oder bildet der zweite Kolben 27 einen Boden der zweiten Kammer 25.

Besonders bevorzugt trennt der zweite Kolben 27 die erste Kammer 24 von der zweiten Kammer 25 und/oder dichtet der zweite Kolben 27 die erste Kammer 24 gegen die zweite Kammer 25 ab, zumindest im Auslieferungszustand der Kartusche 3.

Die Kolben 26, 27 sind vorzugsweise zumindest im Wesentlichen baugleich. Insbesondere entspricht die Geometrie des ersten Kolbens 26 zumindest im Wesentlichen der Geometrie des zweiten Kolbens 27. Ganz besonders bevorzugt sind die Kolben 26, 27 aus demselben Material hergestellt und/oder weisen die Kolben 26, 27 eine zumindest im Wesentlichen gleiche Höhe und/oder einen zumindest im Wesentlichen gleichen Durchmesser auf. Hier sind jedoch auch andere Lösungen möglich, wie im Folgenden noch anhand von Fig. 7 bis Fig. 12 erläutert wird.

Vorzugsweise sind die Kolben 26, 27 dichtend in der Kartusche 3, insbesondere im Behälter 4, geführt, insbesondere derart, dass kein Fluid 2 von einer auf die andere Seite der Kolben 26, 27 gelangen kann, zumindest im Auslieferungszustand der Kartusche 3.

Vorzugsweise liegen die Kolben 26, 27 radial bzw. dichtend an der Innenwand der Kartusche 3, insbesondere des Behälters 4, an, besonders bevorzugt unmittelbar. Insbesondere entspricht der Außendurchmesser der Kolben 26, 27 zumindest im Wesentlichen dem Innendurchmesser der Kartusche 3 bzw. des Behälters 4. Es ist bevorzugt, dass der Außendurchmesser der Kolben 26, 27 geringfügig größer als der Innendurchmesser der Kartusche 3 bzw. des Behälters 4 ist, vorzugsweise um mindestens 0,1 mm oder 0,3 mm und/oder höchstens 1 mm oder 0,8 mm, insbesondere derart, dass die Kolben 26, 27 in der Kartusche 3 bzw. dem Behälter 4 eingepresst sind und/oder dichtend an der Innenwand der Kartusche 3 bzw. des Behälters 4 anliegen.

Vorzugsweise weisen die Kolben 26, 27 jeweils eine Kolbendichtung 28 bzw. 29 auf, vorzugsweise wobei die Kolbendichtungen 28, 29 dazu ausgebildet sind, den Bereich zwischen den Kolben 26, 27 und der Wandung bzw. Innenwand der Kartusche 3 bzw. des Behälters 4 abzudichten.

Bei der dargestellten Ausführungsform sind die Kolbendichtungen 28, 29 durch mehrere umlaufende, radiale Vorsprünge gebildet.

Wie bereits erläutert, ist insbesondere der zweiten Kolben 27 dazu ausgebildet, die Kammern 24, 25 fluidisch voneinander zu trennen, zumindest im Auslieferungszustand der Kartusche 3.

Vorzugsweise sind die Kammern 24, 25 durch Bewegen bzw. Verschieben des ersten Kolbens 26 und/oder des zweiten Kolbens 27 fluidisch miteinander verbindbar, insbesondere derart, dass der erste Bestandteil 2A und der zweite Bestandteil 2B miteinander in Kontakt gelangen bzw. gemischt werden können.

Bei der dargestellten Ausführungsform ist durch Verschieben des zweiten Kolbens 27 in Richtung des Verschlusses 6 bzw. nach oben eine fluidische Verbindung zwischen den Kammern 24, 25 herstellbar.

Vorzugsweise weist die Kartusche 3, insbesondere der Behälter 4, eine Fluidverbindung 30 auf bzw. bildet die Kartusche 3, insbesondere der Behälter 4, eine Fluidverbindung 30, insbesondere wobei die Fluidverbindung 30 dazu ausgebildet ist, die Kammern 24, 25 fluidisch miteinander zu verbinden und/oder eine Überführung des ersten Bestandteils 2A von der ersten Kammer 24 in die zweite Kammer 25 zu ermöglichen.

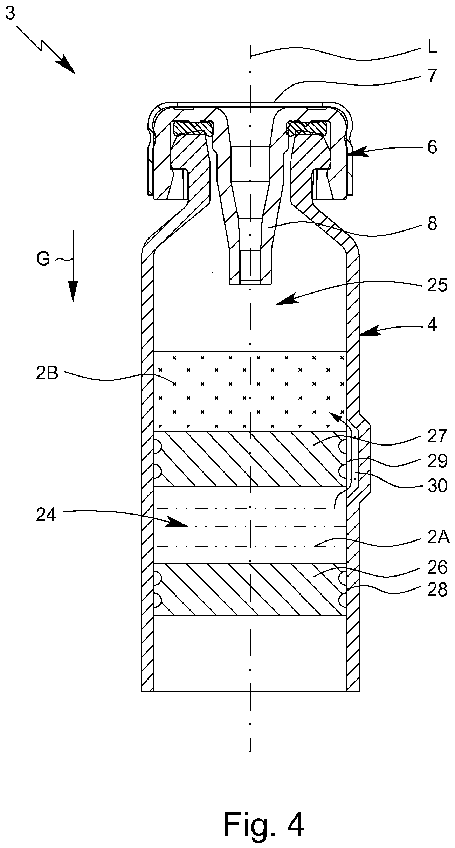

Vorzugsweise ist die Fluidverbindung 30 durch Verschieben des ersten Kolbens 26 und/oder des zweiten Kolbens 27, bei der ersten Ausführungsform durch Verschieben des zweiten Kolbens 27, freigebbar, insbesondere derart, dass die Kammern 24, 25 fluidisch miteinander verbunden werden, wie in Fig. 4 dargestellt.

Die Fluidverbindung 30 ist vorzugsweise länglich und/oder als Kanal bzw. Bypass ausgebildet.

Vorzugsweise ist die Fluidverbindung 30 außermittig und/oder beabstandet zu der Längsachse L angeordnet und/oder verläuft die Fluidverbindung 30 zumindest im Wesentlichen parallel zu der Längsachse L der Kartusche 3.

Insbesondere ist die Fluidverbindung 30 durch den Behälter 4 bzw. die Wandung des Behälters 4, ganz besonders bevorzugt durch eine (radiale) Ausstülpung des Behälters 4 bzw. eine (lokale) Querschnittsvergrößerung des Behälters 4, gebildet.

Vorzugsweise ist der Querschnitt der Kartusche 3 bzw. des Behälters 4 zur Bildung der Fluidverbindung 30 lokal vergrößert und/oder weist die Kartusche 3 bzw. der Behälter 4 einen Abschnitt auf, bei dem der Innendurchmesser der Kartusche 3 bzw. des Behälters 4 größer als der Außendurchmesser des ersten Kolbens 26 und/oder zweiten Kolbens 27 ist, vorzugsweise um mindestens 0,5 mm oder 1 mm, insbesondere derart, dass der erste Kolben 26 und/oder der zweite Kolben 27 zumindest auf der Höhe des Abschnittes bzw. der Fluidverbindung 30 radial nicht über den gesamten Umfang des Kolbens 26 bzw. 27 an der Kartusche 3 bzw. am Behälter 4 anliegt.

Vorzugsweise weist die Fluidverbindung 30 einen maximalen Durchmesser bzw. eine maximale Breite von mindestens 0,5 mm oder 1 mm und/oder höchstens 5 mm oder 4 mm auf bzw. ist der Innendurchmesser der Kartusche 3 bzw. des Behälters 4 an der Stelle der Fluidverbindung 30 um diese Werte vergrößert.

Die Fluidverbindung 30 weist vorzugsweise eine Längserstreckung auf, die länger ist als die Höhe des ersten bzw. zweiten Kolbens 26, 27. Vorzugsweise weist die Fluidverbindung 30 eine Längserstreckung von mindestens 3 mm oder 4 mm, besonders bevorzugt von mindestens 6 mm oder 8 mm, und/oder höchstens 20 mm oder 15 mm, besonders bevorzugt höchstens 10 mm, auf und/oder ist die Fluidverbindung 30 um mindestens 1 mm oder 2 mm, vorzugsweise um mindestens 5 mm oder 6 mm, länger als die Höhe des ersten bzw. zweiten Kolbens 26, 27.

Wie bereits erläutert, ist die Fluidverbindung 30 vorzugsweise lediglich lokal ausbildet bzw. erstreckt sich die Fluidverbindung 30 vorzugsweise weder über die gesamte Länge noch über den gesamten Umfang der Kartusche 3 bzw. des Behälters 4. Es sind jedoch konstruktive Lösungen möglich, bei denen die Kartusche 3 bzw. der Behälter 4 mehrere, insbesondere über den Umfang des Behälters 4 verteilte Fluidverbindungen 30 aufweist, wie im Folgenden noch anhand der zweiten Ausführungsform erläutert wird.

Die Kartusche 3 weist vorzugsweise eine Betätigungseinrichtung 31 auf, insbesondere wobei die Kammern 24, 25 durch Betätigen der Betätigungseinrichtung 31 fluidisch miteinander verbindbar und/oder der bzw. die Kolben 26, 27 in der Kartusche 3 bzw. im Behälter 4 durch Betätigen der Betätigungseinrichtung 31 verschiebbar ist bzw. sind. Insbesondere sind die Bestandteile 2A, 2B durch Betätigen der Betätigungseinrichtung 31 in Kontakt bringbar bzw. mischbar.

Die Betätigungseinrichtung 31 weist vorzugsweise ein Betätigungselement 32 auf, insbesondere wobei die Betätigungseinrichtung 31 bzw. die Kartusche 3 durch Betätigen des Betätigungselements 32 betätigbar ist.

Die Betätigungseinrichtung 31 bzw. das Betätigungselement 32 ist vorzugsweise starr.

Besonders bevorzugt ist die Betätigungseinrichtung 31 bzw. das Betätigungselement 32 plattenförmig, kreisförmig, kappenartig und/oder zylindrisch und/oder als Kappe, Zylinder und/oder Aufsatz ausgebildet.

Die Betätigungseinrichtung 31 ist insbesondere form-, kraft- und/oder stoffschlüssig, besonders bevorzugt durch Verschrauben oder Einrasten, mit der Kartusche 3, insbesondere dem Behälter 4 oder Verschluss 6, verbunden.

Vorzugsweise ist die Betätigungseinrichtung 31 bzw. das Betätigungselement 32 auf die Kartusche 3, insbesondere den Behälter 4 oder Verschluss 6, aufgesteckt bzw. aufgeschraubt oder in die Kartusche 3, insbesondere den Behälter4 oder Verschluss 6, eingesetzt.

Bei den dargestellten Ausführungsformen ist die Betätigungseinrichtung 31 bzw. das Betätigungselement 32 auf die Kartusche 3, insbesondere den Behälter 4 oder Verschluss 6, aufgesteckt bzw. aufgeschraubt bzw. umgreift die Betätigungseinrichtung 31 bzw. das Betätigungselement 32 die Kartusche 3, insbesondere den Behälter 4 oder Verschluss 6.

Besonders bevorzugt ist der maximale Außendurchmesser der Betätigungseinrichtung 31 bzw. des Betätigungselements 32 größer als der maximale Außendurchmesser des Teils der Kartusche 3, insbesondere des Behälters 4 oder Verschlusses 6, mit dem die Betätigungseinrichtung 31 bzw. das Betätigungselement 32 verbunden ist. Es sind jedoch auch Lösungen möglich, bei denen die Betätigungseinrichtung 31 bzw. das Betätigungselement 32 in die Kartusche 3, insbesondere den Behälter 4 oder Verschluss 6, eingesetzt bzw. eingeschraubt bzw. eingesteckt ist.

Die Betätigungseinrichtung 31 ist vorzugsweise an einem axialen Ende der Kartusche 3, insbesondere des Behälters 4 oder des Verschlusses 6, angeordnet und/oder verschließt die Kartusche 3, insbesondere den Behälter 4 oder den Verschluss 6, vorzugsweise axial. Insbesondere bildet die Betätigungseinrichtung 31 ein axiales Ende, einen Boden und/oder einen Deckel der Kartusche 3.

Bei der ersten und zweiten Ausführungsform ist die Betätigungseinrichtung 31 vorzugsweise am dem Verschluss 6 (axial) gegenüberliegenden Ende der Kartusche 3 bzw. des Behälters 4 angeordnet. Insbesondere bildet die Betätigungseinrichtung 31 dann einen Boden der Kartusche 3.

Bei der dritten Ausführungsform ist die Betätigungseinrichtung 31 vorzugsweise an dem Verschluss 6 bzw. dem (axialen) Ende der Kartusche 3 angeordnet, das den

Verschluss 6 aufweist. Insbesondere bildet die Betätigungseinrichtung 31 dann einen Deckel der Kartusche 3.

Bei der ersten und zweiten Ausführungsform ist die Betätigungseinrichtung 31 vorzugsweise mit dem Behälter 4 verbunden bzw. am Behälter 4 befestigt. Es sind jedoch auch Lösungen möglich, bei denen die Betätigungseinrichtung 31 mit dem Verschluss 6 der Kartusche 3 verbunden ist, wie im Folgenden noch anhand der dritten Ausführungsform erläutert wird.

Bei der ersten Ausführungsform ist die Betätigungseinrichtung 31 vorzugsweise am unteren axialen Ende der Kartusche 3 bzw. des Behälters 4 angeordnet und/oder bildet die Betätigungseinrichtung 31 einen Boden der Kartusche 3 bzw. des Behälters 4.

Optional ist die Betätigungseinrichtung 31 dazu ausgebildet, den ersten Kolben 26 und den zweiten Kolben 27 im unbetätigten Zustand der Betätigungseinrichtung 31 in Position zu halten bzw. gegen ein axiales Verschieben zu sichern, insbesondere derart, dass die Fluidverbindung 30 nicht ungewollt freigegeben wird.

Vorzugsweise ist durch Betätigen der Betätigungseinrichtung 31 der erste Kolben 26 und/oder der zweite Kolben 27 freigebbar, vorzugsweise derart, dass sich der erste Kolben 26 und/oder der zweite Kolben 27 selbsttätig bzw. ohne weiteres Zutun eines Nutzers in der Kartusche 3 bzw. dem Behälter 4 verschieben/verschiebt. Hierdurch wird ein besonders einfaches bzw. benutzerfreundliches Mischen der Bestandteile 2A, 2B ermöglicht oder unterstützt. Insbesondere ist zum Mischen der Bestandteile 2A, 2B lediglich ein Freigeben der Kolben 26, 27 durch Betätigen der Betätigungseinrichtung 31 notwendig.

Bei der ersten Ausführungsform ist vorgesehen, dass zumindest auf den zweiten Kolben 27 - vorzugsweise bereits im Auslieferungszustand der Kartusche 3 - eine Kraft in Richtung der zweiten Kammer 25 wirkt. Vorzugsweise herrscht in der zweiten Kammer 25 ein Unterdruck bzw. Vakuum bzw. ein Druck, der niedriger als der Umgebungsdruck ist, vorzugsweise derart, dass auf den zweiten Kolben 27 eine Zugkraft wirkt.

Vorzugsweise beträgt der Druck in der zweiten Kammer 25 weniger als 100 kPa oder 50 kPa, besonders bevorzugt weniger als 40 kPa oder 20 kPa, insbesondere weniger als 1000 Pa oder 100 Pa, und/oder mehr als 100 mPa oder 1 Pa, und/oder ist der Druck in der zweiten Kammer 25 um mindestens 10 kPa oder 20 kPa, insbesondere um mindestens 50 kPa oder 80 kPa, und/oder um höchstens 100 kPa niedriger als der Umgebungsdruck.

Zusätzlich oder alternativ kann die Kraft zum Verschieben der Kolben 26, 27 durch eine - insbesondere bereits im Auslieferungszustand der Kartusche 3 - gespannte Feder bereitgestellt werden. Es sind insbesondere konstruktive Lösungen möglich, bei denen zusätzlich oder alternativ zum Unterdruck in der zweiten Kammer 25 eine gestreckte Feder angeordnet ist, die den zweiten Kolben 27 in Richtung des Verschlusses 6 zieht und/oder eine Zugkraft in Richtung des Verschlusses 6 erzeugt.

Die Betätigungseinrichtung 31 ist dazu ausgebildet, der auf den zweiten Kolben 27 und somit auf den Behälter 4 wirkenden Kraft Stand zu halten.

Die Betätigung der Betätigungseinrichtung 31 bzw. des Betätigungselements 32 kann durch Abnehmen der Betätigungseinrichtung 31 bzw. des Betätigungselements 32 vom Behälter 4 oder Verschluss 6, durch Drehen, durch Niederdrücken und/oder durch Öffnen bzw. Zertrennen der Betätigungseinrichtung 31 bzw. des Betätigungselements 32 erfolgen.

Bei der ersten Ausführungsform ist die Betätigungseinrichtung 31 vorzugsweise durch das Betätigungselement 32 gebildet bzw. ist die Betätigungseinrichtung 31 einstückig ausgebildet. Es sind jedoch auch Lösungen möglich, bei denen die Betätigungseinrichtung 31 mehrere Bauteile aufweist bzw. mehrteilig ausgebildet ist, wie im Folgenden noch näher erläutert wird.

Bei der ersten Ausführungsform ist die Betätigungseinrichtung 31 bzw. das Betätigungselement 32 vom Boden des Behälters 4 abnehmbar, insbesondere abziehbar oder abdrehbar, vorzugsweise um den ersten Kolben 26 und/oder den zweiten Kolben 27 freizugeben und/oder axial zu verschieben und/oder den ersten Bestandteil 2A von der ersten Kammer 24 über die Fluidverbindung 30 in die zweite Kammer 25 zu überführen und/oder den ersten Bestandteil 2A und den zweiten Bestandteil 2B zu mischen.

Aufgrund der Betätigungseinrichtung 31 ist es vorzugsweise möglich, die Kammern 24, 25 im ungeöffneten bzw. nach außen bzw. gegenüber der Umgebung abgedichteten, insbesondere gas- und/oderflüssigkeitsdichten, Zustand der Kartusche 3 bzw. des Behälters 4 fluidisch miteinander zu verbinden bzw. die Bestandteile 2A, 2B miteinander zu mischen. Insbesondere durch Betätigen der Betätigungseinrichtung 31 können die Bestandteile 2A, 2B zusammengeführt werden, ohne die Kartusche 3 bzw. die Kammern 24, 25 nach außen bzw. zur Umgebung zu öffnen. Auf diese Weise wird ein besonders steriles bzw. hygienisches Mischen der Bestandteile 2A, 2B ermöglicht. Insbesondere wird ein Flüssigkeitsaustritt beim Mischen verhindert.

Bei der ersten Ausführungsform führt ein Betätigen der Betätigungseinrichtung 31 bzw. des Betätigungselements 32, insbesondere ein Abnehmen der Betätigungseinrichtung 31 bzw. des Betätigungselements 32, vorzugsweise nicht dazu, dass die Kammern 24, 25 nach außen bzw. gegenüber der der Umgebung geöffnet werden.

Durch Betätigung, insbesondere Abnehmen, der Betätigungseinrichtung 31 bzw. des Betätigungselements 32 wird vorzugsweise der erste Kolben 26 und/oder der zweite Kolben 27 freigegeben, so dass sich der erste Kolben 26 und/oder der zweite Kolben 27 - erfindungsgemäß selbsttätig bzw. aufgrund des Unterdrucks in der zweiten Kammer 25 - axial bzw. nach oben bzw. in Richtung des Verschlusses 6 bewegen können/kann, wie in Fig. 4 dargestellt.

Der Unterdruck bzw. die Zugkraft in der zweiten Kammer 25 bewirkt insbesondere, dass sich der zweite Kolben 27 zusammen mit dem ersten Kolben 26 in Richtung des Verschlusses 6 bzw. der zweiten Kammer 25 verschiebt, insbesondere derart, dass zunächst ausschließlich das Volumen der zweiten Kammer 25 verkleinert wird. Vorzugsweise bewegen sich der erste Kolben 26 und der zweite Kolben 27 zumindest im Wesentlichen zeitgleich und/oder um dieselbe Strecke bzw. denselben Hub, zumindest bis der zweite Kolben 27 die Fluidverbindung 30 freigibt.

Wenn der zweite Kolben 27 auf die Höhe der Fluidverbindung 30 gelangt bzw. die Fluidverbindung 30 freigibt, wird vorzugsweise der in der zweiten Kammer 25 wirkende Druck über die Fluidverbindung 30 und die erste Kammer 24 bzw. durch Bewegen des ersten Kolbens 26 bzw. Verkleinern der ersten Kammer 24 ausgeglichen. Durch den Unterdruck in der zweiten Kammer 25 wird vorzugsweise der erste Bestandteil 2A aus der ersten Kammer 24 über die Fluidverbindung 30 in die zweite Kammer 25 gesaugt bzw. wird nach Freigabe bzw. Öffnen der Fluidverbindung 30 das Volumen der ersten Kammer 24 verkleinert bzw. der erste Kolben 26 in Richtung des zweiten Kolbens 27 und/oder bis zum zweiten Kolben 27 verschoben.

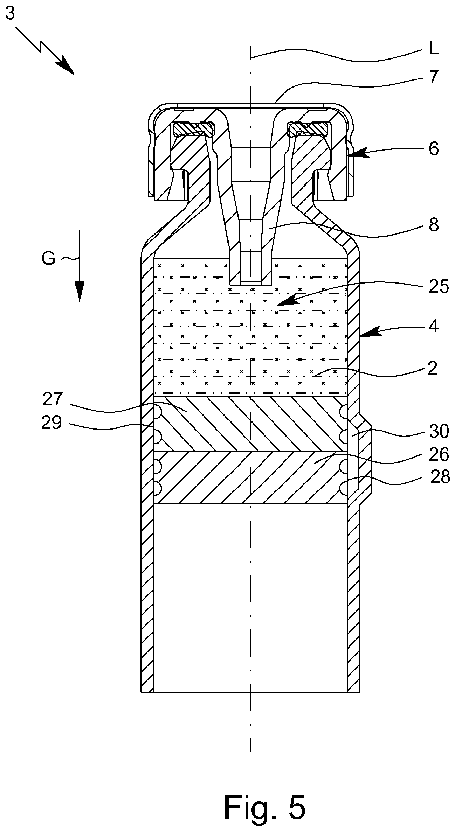

Vorzugsweise wird der erste Kolben 26 derart weit verschoben, bis der erste Kolben 26 zumindest im Wesentlichen vollflächig bzw. stirnseitig am zweiten Kolben 27 anliegt und/oder der erste Bestandteil 2A zumindest im Wesentlichen vollständig in die zweite Kammer 25 überführt wurde, wie in Fig. 5 dargestellt.

Vorzugsweise verschließen/verschließt der erste Kolben 26 und/oder der zweite Kolben 27 die Fluidverbindung 30, wenn der erste Kolben 26 am zweiten Kolben 27 anliegt bzw. seine Endposition einnimmt.

Vorzugsweise sind/ist der Unterdruck und/oder die zwischen den Kolben 26, 27 und dem Behälter 4 herrschende Reibung derart gewählt, dass sich der erste Kolben 26 vollflächig an den zweiten Kolben 27 anlegt bzw. die erste Kammer 24 vollständig entleert wird bzw. der erste Bestandteil 2A vollständig in die zweite Kammer 25 überführt wird.

Je nach gewähltem Unterdruck und/oder der zwischen den Kolben 26, 27 und dem Behälter 4 herrschenden Reibung erfolgt der Mischvorgang unterschiedlich schnell und/oder bewegen sich die Kolben 26, 27 nach erfolgtem Mischen der Bestandteile 2A, 2B bzw. vollständigem Überführen des ersten Bestandteils 2A in die zweite Kammer 25 zusammen bzw. axial in Richtung des Verschlusses 6 weiter, wie in Fig. 5 dargestellt.

Die Kolben 26, 27 erreichen vorzugsweise ihre (gemeinsame) Endposition, wenn die aufgrund des Unterdrucks auf die Kolben 26, 27 wirkende Zugkraft zumindest im Wesentlichen der Reibungskraft der Kolben 26, 27 entspricht, wobei der Einfluss der Schwerkraft G unberücksichtigt wird bzw. werden kann.

Vorzugsweise ist die Kartusche 3, insbesondere der Behälter 4, besonders bevorzugt die zweite Kammer 25, in der (gemeinsamen) Endposition der Kolben 26, 27 bzw. im vollständig gemischten Zustand der Bestandteile 2A, 2B um mindestens 80 % oder 85 %, besonders bevorzugt um mindestens 90 % oder 95 %, mit dem durch Mischen der Bestandteile 2A, 2B hergestellten Fluid 2 gefüllt.

Besonders bevorzugt ist bzw. sind die Kartusche 3 bzw. der Behälter 4 bzw. die Kammern 26, 27 auch nach Betätigen der Betätigungseinrichtung 31 bzw. während bzw. nach dem Mischen der Bestandteile 2A, 2B, an beiden axialen Enden abgedichtet bzw. geschlossen, insbesondere nach unten durch den ersten und/oder zweiten Kolben 26, 27 und nach oben durch den Verschluss 6.

Die auf diese Weise vorbereitete Kartusche 3 kann anschließend in den Zerstäuber 1 eingesetzt und/oder mittels des Zerstäubers 1 verwendet werden.

Optional ist einer der Kolben 26, 27, insbesondere der erste Kolben 26, - insbesondere mittels der Betätigungseinrichtung 31 bzw. des Betätigungselements 32 und/oder nach Mischen der Bestandteile 2A, 2B - aus der Kartusche 3, insbesondere dem Behälter 4, herausnehmbar bzw. herausziehbar.

Insbesondere kann die Betätigungseinrichtung 31 bzw. das Betätigungselement 32 mit dem ersten Kolben 26 verbunden sein, beispielsweise über ein Verbindungsteil, wie ein Seil o.dgl.

Durch Herausnehmen des ersten Kolbens 26 wird vorzugsweise lediglich der zweite Kolben 27 verschoben, wenn eine Dosis des Fluids 2 aus der Kartusche 3 bzw. dem Behälter 4 entnommen wird. Auf diese Weise wird die Entnahme einer Dosis des Fluids 2 vereinfacht bzw. die zu überwindende Reibung reduziert, wie im Folgenden noch anhand der zweiten Ausführungsform erläutert wird.

Es ist bevorzugt, die Kartusche 3 erst nach Betätigen der Betätigungseinrichtung 31 bzw. nach Mischen der Bestandteile 2A, 2B bzw. nach Überführung des ersten Bestandteils 2A von der ersten Kammer 24 in die zweite Kammer 25 nach außen bzw. gegenüber der Umgebung bzw. zur Entnahme einer Dosis des Fluids 2 zu öffnen, insbesondere mittels des Förderelements 13 bzw. indem das Förderelement 13 in den Verschluss 6 eingeführt und/oder indem das Siegel 7 zerstört bzw. durchstochen wird.

Vorzugsweise erfolgt ein Mischen der Bestandteile 2A, 2B außerhalb des Zerstäubers 1 bzw. unmittelbar vor Einsetzen der Kartusche 3 in den Zerstäuber 1.

Die insbesondere auf diese Weise vorbereitete Kartusche 3, d. h. die Kartusche 3 mit den gemischten Bestandteilen 2A, 2B bzw. dem abgabebereiten Fluid 2 in der zweiten Kammer 25, kann anschließend in das Gehäuse 19 eingesetzt und/oder beispielsweise im Zerstäuber 1 geöffnet werden, vorzugsweise indem das Förderelement 13 das Siegel 7 durchdringt und somit die Kartusche 3 zur Entnahme einer Dosis des Fluids 2 öffnet, wie bereits im Zusammenhang mit Fig. 1 und Fig. 2 beschrieben.

Alternativ kann vorgesehen sein, die Kartusche 3 zunächst in den Zerstäuber 1 einzusetzen und erst anschließend die Bestandteile 2A, 2B zu mischen. Insbesondere kann bei einer derartigen Verfahrensvariante vorgesehen sein, dass durch Einsetzen der Kartusche 3 in den Zerstäuber 1 bzw. durch Schließen des Gehäuses 19, insbesondere durch Verbinden des unteren Gehäuseteils 21 mit dem oberen Gehäuseteil 20 bzw. dem inneren Gehäuseteil 22, oder durch erstmaliges Spannen des Zerstäubers 1 die Betätigungseinrichtung 31 bzw. das Betätigungselement 32 betätigt wird und sich somit die Bestandteile 2A, 2B innerhalb des Zerstäubers 1 mischen. Beispielsweise kann durch Einsetzen der Kartusche 3 in den Zerstäuber 1 bzw. Schlie-ßen des Gehäuses 19 oder durch erstmaliges Spannen des Zerstäubers 1 das Betätigungselement 32 insbesondere durch das untere Gehäuseteil 21 durchtrennt werden, insbesondere derart, dass die Kolben 26, 27 freigegeben werden und sich die Bestandteile 2A, 2B mischen, wie bereits erläutert.

Der Zerstäuber 1 weist vorzugsweise eine optionale Luftpumpe 33 auf, insbesondere um - zumindest temporär bzw. beim Spannvorgang - das Fluid 2 in der Kartusche 3 bzw. im Behälter 4 unter Druck zu setzen, um eine Entnahme des Fluids 2 aus der Kartusche 3 bzw. dem Behälter 4 zu erleichtern bzw. ein Verschieben des/der Kolben 26, 27 zu unterstützen und/oder um einen Druckausgleich in der zweiten Kammer 25 nach Mischen der Bestandteile 2A, 2B zu erzielen bzw. einen Restunterdruck in der zweiten Kammer 25 nach Mischen der Bestandteile 2A, 2B für die Entnahme des Fluids 2 auszugleichen.

Die Luftpumpe 33 ist vorzugsweise im unteren Gehäuseteil 21 und/oder im Boden des Zerstäubers 1 angeordnet, wie in Fig. 6 dargestellt.

Vorzugsweise weist die Luftpumpe 33 einen Pumpkolben 34, einen Pumpzylinder 35 und eine Pumpkammer 36 auf, vorzugsweise wobei der Pumpkolben 34 axial im Pumpzylinder 35 geführt ist und/oder der Pumpkolben 34 und der Pumpzylinder 35 die Pumpkammer 36 begrenzen. Insbesondere ist die Luftpumpe 33 dazu ausgebildet, zumindest temporär Luft in die Kartusche 3 bzw. den Behälter 4 zu pumpen.

Vorzugsweise arbeitet die Luftpumpe 33 insbesondere ausschließlich mechanisch. Insbesondere wird die Luftpumpe 33 vorzugsweise durch die (Hub-)Bewegung der Kartusche 3 im Zerstäuber 1, insbesondere durch Spannen des Zerstäubers 1, aktiviert.

Vorzugsweise ermöglicht die Luftpumpe 33 eine vordefinierte bzw. begrenzte Druckerhöhung in der Kartusche 3 bzw. in der Pumpkammer 36.

Die Pumpkammer 36 ist zumindest teilweise in der bzw. durch die Kartusche 3 bzw. durch Verschieben der Kolben 26, 27 gebildet, wie in Fig. 6 dargestellt.

Vorzugsweise weist die Luftpumpe 33 ein Pumpventil 37 auf, wobei das Pumpventil 37 dazu ausgebildet ist, die Luftzufuhr und die Luftabfuhr in die Pumpkammer 36 zu steuern, insbesondere zu ermöglichen und/oder zu verhindern.

Insbesondere ist das Pumpventil 37 dazu ausgebildet, einen Unterdruck in der Luftpumpe 33, insbesondere in der Pumpkammer 36, beim Abgabevorgang bzw. bei der Abgabe einer Dosis des Fluids 2 bzw. bei Betätigung des Zerstäubers 1 bzw. bei einer Bewegung der Kartusche 3 in Richtung des Mundstücks 17 (in Fig. 6 nicht dargestellt) zu verhindern, insbesondere indem sich das Pumpventil 37 öffnet und/oder eine Luftzufuhr in die Pumpkammer 36 ermöglicht.

Vorzugsweise ist das Pumpventil 37 dazu ausgebildet, die Luftabfuhr aus der Pumpkammer 36 beim Spannvorgang bzw. bei der Bewegung der Kartusche 3 nach unten bzw. beim Verkleinern der Pumpkammer 36 zu verhindern oder zu begrenzen, insbesondere derart, dass eine Druckerhöhung in der Pumpkammer 36 erfolgt.

Fig. 6 zeigt einen Ausschnitt des Zerstäubers 1 mit der eingesetzten Kartusche 3 gemäß der ersten Ausführungsform, wobei der Zerstäuber 1 ungespannt ist.

Vorzugsweise bildet die Kartusche 3 bzw. der Behälter 4 einen Teil der Luftpumpe 33. Insbesondere bildet die Kartusche 3 bzw. der Behälter 4 den Pumpkolben 34 und/oder wird die Kartusche 3 bzw. der Behälter 4 als Pumpkolben 34 eingesetzt und/oder im Pumpzylinder 35 radial geführt.

Insbesondere wirkt die Kartusche 3 bzw. der Behälter 4 zusammen mit dem Pumpzylinder 35 als Kolben-Zylinder-Anordnung, um Luft in den Behälter 4 bzw. in die Pumpkammer 36 zu pumpen bzw. temporär bzw. beim Spannvorgang des Zerstäubers 1 den Druck in der Pumpkammer 36 zu erhöhen und somit die Entnahme einer Dosis des Fluids 2 aus dem Behälter 4 zu unterstützen.

Zusätzlich ist es mittels der Luftpumpe 33 möglich, den bzw. die Kolben 26, 27 bei erstmaliger Betätigung des Zerstäubers 1 derart zu verschieben, dass ein eventuell nach dem Mischen der Bestandteile 2A, 2B verbleibender Unterdruck in der zweiten Kammer 25 zumindest im Wesentlichen vollständig bzw. gegenüber der Umgebung ausgeglichen wird.

Vorzugsweise weist die Luftpumpe 33 eine Pumpbelüftung 38 auf, insbesondere um das Pumpventil 37 mit der Umgebung (fluidisch bzw. pneumatisch) zu verbinden. Die Pumpbelüftung 38 kann durch eine Öffnung, einen Kanal o. dgl. im Gehäuse 19, insbesondere im unteren Gehäuseteil 21, gebildet sein.

Fig. 7 bis Fig. 12 zeigen die vorschlagsgemäße Kartusche 3 gemäß einer zweiten Ausführungsform.

Die zweite Ausführungsform der Kartusche 3 entspricht zumindest im Wesentlichen der ersten Ausführungsform der Kartusche 3.

Vorzugsweise gelten die Ausführungen zu der ersten Ausführungsform der Kartusche 3 für die zweite Ausführungsform der Kartusche 3 entsprechend. Insbesondere kann die zweite Ausführungsform bzw. die im Zusammenhang mit Fig. 7 bis Fig. 12 erläuterte Kartusche 3 ein oder mehrere Merkmale der ersten Ausführungsform bzw. der im Zusammenhang mit Fig. 3 bis Fig. 6 erläuterten Kartusche 3 aufweisen und/oder grundsätzlich mit dem im Zusammenhang mit Fig. 1 und Fig. 2 beschriebenen Zerstäuber 1 verwendet werden.

Im Gegensatz zu der ersten Ausführungsform werden die Kolben 26, 27 der Kartusche 3 gemäß der zweiten Ausführungsform jedoch vorzugsweise nicht oder nicht ausschließlich selbsttätig, beispielsweise durch einen Unterdruck in der zweiten Kammer 25, in der Kartusche 3 verschoben, sondern (zusätzlich) durch eine mittels der Betätigungseinrichtung 31 bzw. des Betätigungselements 32 eingebrachte Kraft. Auch bei der zweiten Ausführungsform kann in der zweiten Kammer 25 ein Unterdruck bzw. Vakuum vorliegen bzw. der Druck in der zweiten Kammer 25 - zumindest im Auslieferungszustand der Kartusche 3 - kleiner als der Druck in der ersten Kammer 24 sein.

Besonders bevorzugt ist die Betätigungseinrichtung 31 bzw. das Betätigungselement 32 zumindest mit dem ersten Kolben 26 mechanisch verbunden bzw. gekoppelt und/oder weist die Betätigungseinrichtung 31 bzw. das Betätigungselement 32 den ersten Kolben 26 auf oder bildet diesen, insbesondere derart, dass durch Betätigen der Betätigungseinrichtung 31 bzw. des Betätigungselements 32 der erste Kolben 26 axial in der Kartusche 3 bzw. in dem Behälter 4 verschoben wird.

Vorzugsweise weist die Betätigungseinrichtung 31 ein Verbindungsteil 39 auf, insbesondere wobei das Verbindungsteil 39 den ersten Kolben 26 und/oder den zweiten Kolben 27 mit dem Betätigungselement 32 mechanisch verbindet/koppelt.

Durch das Verbindungsteil 39 ist es möglich, eine Bewegung der Betätigungseinrichtung 31 bzw. des Betätigungselements 32 auf den ersten Kolben 26 bzw. den zweiten Kolben 27 zu übertragen.

Bei der zweiten Ausführungsform ist die Betätigungseinrichtung 31 erfindungsgemäß durch Drehen des Betätigungselements 32 betätigbar.

Das Verbindungsteil 39 ist vorzugsweise dazu ausgebildet, die Drehbewegung des Betätigungselements 32 in eine Axial- bzw. Hubbewegung des ersten Kolbens 26 und/oder des zweiten Kolbens 27 umzuwandeln.

Vorzugsweise ist das Betätigungselement 32 form-, kraft- und/oder stoffschlüssig mit dem Verbindungsteil 39 verbunden, insbesondere derart, dass ein Drehmoment von dem Betätigungselement 32 auf das Verbindungsteil 39 übertragen werden kann bzw. durch Drehen des Betätigungselements 32 das Verbindungsteil 39 gedreht wird.

Vorzugsweise ist das Verbindungsteil 39 länglich bzw. stabförmig, insbesondere als Gewindestange, ausgebildet und/oder weist das Verbindungsteil 39 zumindest teilweise bzw. abschnittsweise ein Gewinde, insbesondere ein Außengewinde, auf.

Vorzugsweise erstreckt sich das Verbindungsteil 39 insbesondere mittig durch den ersten Kolben 26 bzw. die erste Kammer 24 und/oder den zweiten Kolben 27 bzw. bis in die zweite Kammer 25 hindurch.

Vorzugsweise weisen/weist der erste Kolben 26 und/oder der zweite Kolben 27 einen entsprechenden Durchbruch bzw. eine entsprechende Bohrung für das Verbindungsteil 39 auf.

Bei der dargestellten zweiten Ausführungsform weist vorzugsweise lediglich der erste Kolben 26 einen Durchbruch bzw. eine Bohrung auf bzw. erstreckt sich das Verbindungsteil 39 lediglich durch den ersten Kolben 26 und die erste Kammer 24 bis zum zweiten Kolben 27.

Der zweite Kolben 27 weist vorzugsweise eine Aufnahme bzw. Aussparung auf, um das axiale Ende des Verbindungsteils 39 aufzunehmen.

Vorzugsweise weist zumindest der erste Kolben 26 ein zu dem Außengewinde des Verbindungsteils 39 korrespondierendes Innengewinde auf, insbesondere derart, dass eine Drehung des Verbindungsteils 39 zu einem Verschieben des ersten Kolbens 26 führt.

Vorzugsweise ist der zweite Kolben 27 zumindest in Umfangsrichtung bzw. Drehrichtung des Verbindungsteils 39 form- und/oder kraftschlüssig mit dem Verbindungsteil 39 verbunden, insbesondere derart, dass ein Drehmoment von dem Verbindungsteil 39 auf den zweiten Kolben 27 übertragen werden kann bzw. eine Drehung des Verbindungsteils 39 eine Drehung des zweiten Kolbens 27 bewirkt.

Vorzugsweise liegt/liegen der/die Kolben 26, 27 dichtend an dem Verbindungsteil 39 an, insbesondere derart, dass ein Austreten der Bestandteile 2A, 2B zwischen dem/den Kolben 26, 27 und dem Verbindungsteil 39 verhindert wird.

Das Verbindungsteil 39 ist vorzugsweise mehrteilig ausgebildet. Vorzugsweise weist das Verbindungsteil 39 ein erstes Verbindungselement 39A und ein zweites Verbindungselement 39B auf, vorzugsweise wobei das erste Verbindungselement 39A das zweite Verbindungselement 39B mit dem Betätigungselement 32 verbindet und/oder das zweite Verbindungselement 39B ein axiales Ende des Verbindungsteils 39 aufweist oder bildet.

Besonders bevorzugt ist das Verbindungsteil 39 als Teleskopauszug bzw. Teleskoprohr ausgebildet und/oder ist das Verbindungsteil 39 durch Drehen des ersten Verbindungselements 39A relativ zum zweiten Verbindungselement 39B verlängerbar, insbesondere teleskopartig.

Besonders bevorzugt sind die Verbindungselemente 39A, 39B miteinander verschraubt. Das erste Verbindungselement 39A weist vorzugsweise ein Außengewinde und das zweite Verbindungselement 39B ein dazu korrespondierendes Innengewinde auf, vorzugsweise wobei das zweite Verbindungselement 39B zumindest im Auslieferungszustand der Kartusche 3 auf das erste Verbindungselement 39A aufgeschraubt ist.

Insbesondere ist das zweite Verbindungselement 39B als Hohlzylinder mit Innengewinde und/oder das erste Verbindungselement 39A zumindest teilweise als Gewindestange ausgebildet. Es sind jedoch auch andere Lösungen möglich.

Vorzugsweise weist das zweite Verbindungselement 39B zumindest endseitig ein Außenprofil auf, das eine Drehmomentübertragung auf den zweiten Kolben 27 ermöglicht. Beispielsweise kann das zweite Verbindungselement 39B zumindest endseitig eckig ausgebildet sein.

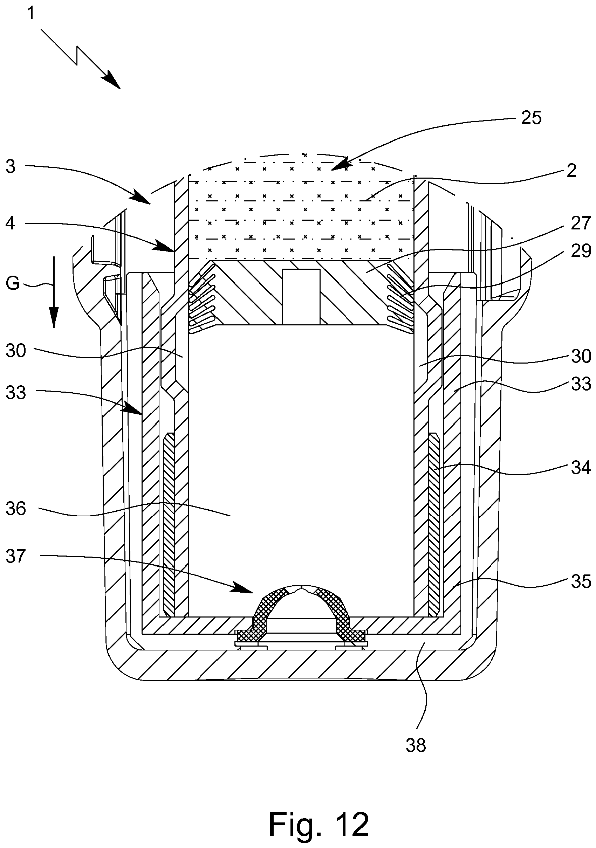

Vorzugsweise wird durch Drehen der Betätigungseinrichtung 31 bzw. des Betätigungselements 32 bzw. des Verbindungsteils 39 der erste Kolben 26 nach oben bzw. in Richtung des Verschlusses 6 verschoben. Auf diese Weise wird der Abstand zwischen dem ersten Kolben 26 und dem Betätigungselement 32 vergrößert bzw. ein Freiraum zwischen dem Betätigungselement 32 und dem zweiten Verbindungselement 39B gebildet, der im Zerstäuber 1 optional als Pumpkammer 36 dient, wie bereits anhand der ersten Ausführungsform erläutert und für die zweite Ausführungsform in Fig. 12 dargestellt.

Um zu verhindern, dass durch Drehen des Betätigungselements 32 ein Unterdruck in dem Freiraum zwischen dem Betätigungselement 32 und dem ersten Kolben 26 entsteht, weist die Betätigungseinrichtung 31, insbesondere das Betätigungselement 32, eine optionale Belüftung 40 auf, wie in Fig. 7 bis Fig. 10 dargestellt, vorzugsweise wobei die Belüftung 40 als Durchbruch, Kanal, Öffnung o. dgl. im Betätigungselement 32 ausgebildet ist und/oder den Freiraum mit der Umgebung fluidisch bzw. pneumatisch verbindet.

Durch die Belüftung 40 wird jedoch vorzugsweise nur der Freiraum zwischen dem ersten Kolben 26 und dem Betätigungselement 32 belüftet. Insbesondere sind die Kammern 24 und 25 weiterhin geschlossen bzw. abgedichtet.

Vorzugsweise wird durch Betätigen der Betätigungseinrichtung 31 bzw. des Betätigungselements 32 das insbesondere teleskopartige Verbindungsteil 39 ausgefahren bzw. verlängert und/oder das zweite Verbindungselement 39B - insbesondere zusammen mit dem ersten Kolben 26 - axial verschoben.

Insbesondere wird durch die Drehbewegung des Betätigungselements 32 und somit des Verbindungsteils 39 das - vorzugsweise formschlüssig vom zweiten Kolben 27 gehaltene - zweite Verbindungselement 39B zusammen mit dem zweiten Kolben 27 axial verschoben. Auf diese Weise ist es möglich, durch Betätigung der Betätigungseinrichtung 31 bzw. des Betätigungselements 32 sowohl den ersten Kolben 26 als auch den zweiten Kolben 27 in der Kartusche 3 zu verschieben, insbesondere zeitgleich bzw. simultan bzw. um den gleichen Hub.

Durch Drehen des Betätigungselements 32 wird die erste Kammer 24 zusammen mit dem ersten Kolben 26 und dem zweiten Kolben 27 axial verschoben und/oder die zweite Kammer 25 verkleinert, wie bereits im Zusammenhang mit der ersten Ausführungsform erläutert.

Insbesondere wird der Druck in der zweiten Kammer 25 durch Betätigen der Betätigungseinrichtung 31 bzw. des Betätigungselements 32 bzw. durch Verschieben der Kolben 26, 27 zumindest temporär bzw. bis zum fluidischen Verbinden der Kammern 24, 25 erhöht.

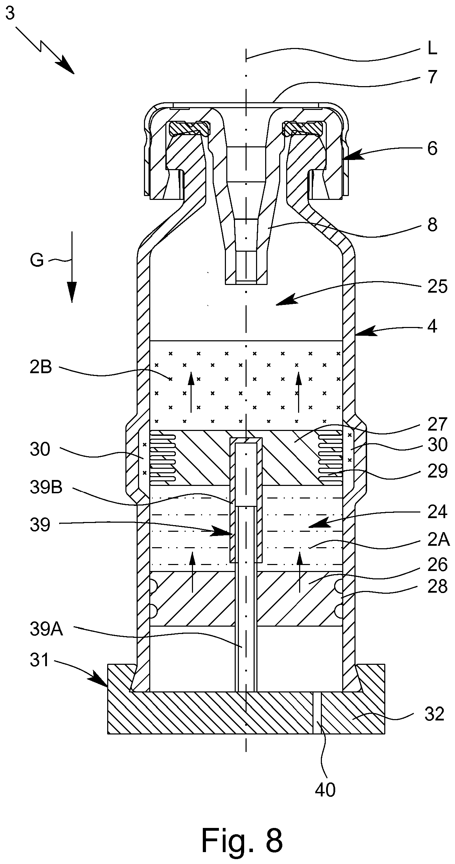

Die Kolben 26, 27 werden vorzugsweise durch Betätigung der Betätigungseinrichtung 31 bzw. des Betätigungselements 32 in der Kartusche 3 bzw. im Behälter 4 bis zu einer Position verschoben, in der der zweite Kolben 27 auf der Höhe der Fluidverbindung 30 angeordnet ist, die Fluidverbindung 30 freigibt und/oder die Kammern 24, 25 fluidisch miteinander verbunden sind, wie in Fig. 8 dargestellt.

Durch die Freigabe der Fluidverbindung 30 wird vorzugsweise ein - insbesondere durch Verschieben der Kolben 26, 27 herbeigeführter - Druckunterschied zwischen den Kammern 24, 25 ausgeglichen. Insbesondere wird ein ggf. aufgebauter Überdruck in der zweiten Kammer 25 reduziert. Es ist jedoch auch möglich, dass ein eventuell vorhandener Unterdruck in der zweiten Kammer 25 durch fluidisches Verbinden der Kammern 24, 25 ausgeglichen wird.

Bei der dargestellten zweiten Ausführungsform sind vorzugsweise mehrere Fluidverbindungen 30, hier zwei Fluidverbindungen 30, vorgesehen, insbesondere um ein Ausströmen des ersten Bestandteils 2A aus der ersten Kammer 24 zu erleichtern.

Sobald die Kolben 26, 27 die Position zum Mischen der Bestandteile 2A, 2B erreicht haben, kann der Mischvorgang durchgeführt werden.

Vorzugsweise zeigt der Zerstäuber 1 einem Nutzer - insbesondere optisch, akustisch und/oder haptisch - an, wenn die Betätigung bzw. das Drehen des Betätigungselements 32 für die Mischung der Bestandteile 2A, 2B eingestellt werden soll bzw. der Mischvorgang gestartet werden kann bzw. die Position erreicht ist, in der die Fluidverbindung 30 freigegeben ist. Beispielsweise kann die Kartusche 3 bzw. der Behälter 4 zumindest teilweise bzw. abschnittsweise transparent ausgebildet sein und/oder ein Sichtfenster aufweisen, um den ersten Kolben 26 und/oder den zweiten Kolben 27 von außen sehen zu können, insbesondere dann, wenn der/die Kolben 26, 27 die Position zum Mischen der Bestandteile 2A, 2B erreicht hat/haben.

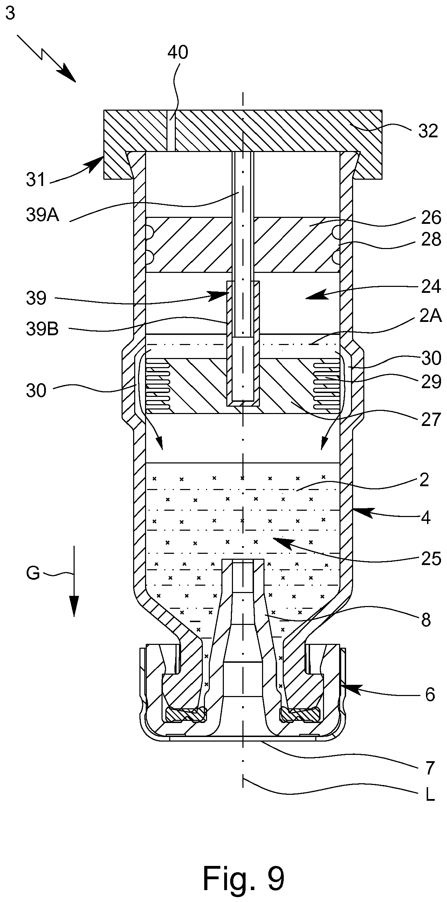

Nach Freigabe der Fluidverbindung(en) 30 wird vorzugsweise der Mischvorgang eingeleitet. Dazu wird die Kartusche 3 derart ausgerichtet, dass die erste Kammer 24 oberhalb der zweiten Kammer 25 angeordnet ist und/oder die Schwerkraft G den ersten Bestandteil 2A von der ersten Kammer 24 über die Fluidverbindung(en) 30 in die zweite Kammer 25 überführt, wie in Fig. 9 dargestellt.

Die Fluidverbindung(en) 30 ist bzw. sind insbesondere derart dimensioniert, dass ein Ausströmen des ersten Bestandteils 2A von der ersten Kammer 24 in die zweite Kammer 25 ausschließlich in Folge der Schwerkraft G und/oder ohne eine (zusätzliche) Krafteinwirkung und/oder ein insbesondere gleichzeitiges Entlüften der zweiten Kammer 25 bzw. ein insbesondere gleichzeitiges Ausströmen eines Gases von der zweiten Kammer 25 in die erste Kammer 24 erfolgt.

Wie bereits erwähnt, ist bei der zweiten Ausführungsform vorzugsweise kein Unterdruck bzw. Vakuum in der zweiten Kammer 25 erforderlich. Ein Gas, das in der zweiten Kammer 25 beispielsweise produktionstechnisch bedingt vorliegt, kann beim Mischen der Bestandteile 2A, 2B über die Fluidverbindung(en) 30 von der zweiten Kammer 25 in die erste Kammer 24 bzw. nach oben strömen/entweichen. Folglich erfolgt ein Befüllen der zweiten Kammer 25 bei gleichzeitigem Entlüften der zweiten Kammer 25.

Es ist jedoch auch möglich, dass auch bei der zweiten Ausführungsform ein Unterdruck bzw. Vakuum in der zweiten Kammer 25 vorgesehen ist, beispielsweise um ein Verschieben der Kolben 26, 27 zu unterstützen.

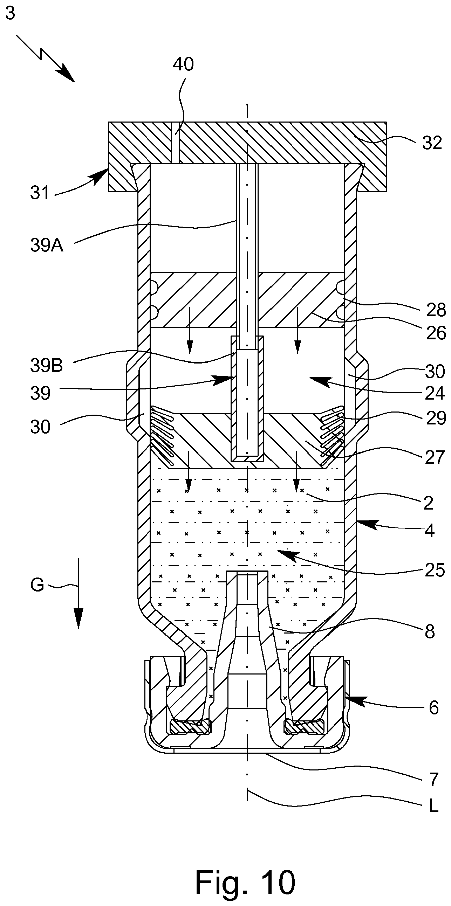

Sobald die erste Kammer 24 vollständig entleert ist bzw. der Mischvorgang abgeschlossen ist, wird die Betätigungseinrichtung 31 bzw. das Betätigungselement 32 vorzugsweise erneut betätigt, insbesondere gedreht, insbesondere um die Fluidverbindung(en) 30 wieder zu schließen, insbesondere mittels des zweiten Kolbens 27, wie in Fig. 10 dargestellt.

Insbesondere wird durch ein erneutes Betätigen der Betätigungseinrichtung 31 bzw. des Betätigungselements 32 das Verbindungsteil 39 weiter verlängert bzw. ausgefahren und/oder das zweite Verbindungselement 39B bzw. der zweite Kolben 27 axial weiter verschoben, insbesondere über die Fluidverbindung(en) 30 hinaus bzw. in Richtung des Verschlusses 6, besonders bevorzugt bis der zweite Kolben 27 (wieder) vollumfänglich bzw. radial an der Wandung der Kartusche 3 bzw. des Behälters 4 anliegt und/oder die Fluidverbindung(en) 30 verschließt.

Vorzugsweise wird nach Verschließen der Fluidverbindung(en) 30 und/oder durch (anschließendes) Verschieben der Kolben 26, 27 der Druck in der zweiten Kammer 25 (erneut) erhöht bzw. ein Druckunterschied zwischen den Kammern 24, 25 aufgebaut, insbesondere derart, dass der Druck in der zweiten Kammer 25 größer als der Druck in der ersten Kammer 24 ist.

Vorzugsweise ist die Kolbendichtung 29 des zweiten Kolbens 27 flexibel bzw. nachgiebig und/oder lamellenartig ausgebildet. Insbesondere kann bei Überschreiten eines vorbestimmten Drucks in der zweiten Kammer 25 die Kolbendichtung 29 des zweiten Kolbens 27 derart nachgeben bzw. sich partiell derart öffnen, dass ein Gas bzw. eine Flüssigkeit zumindest teilweise in die Kolbendichtung 29 eindringt, wie in Fig. 10 angedeutet. Dadurch ist es möglich, ein in der zweiten Kammer 25 eingeschlossenes Gas durch Verschieben des zweiten Kolbens 27 in Richtung des Verschlusses 6 in die Kolbendichtung 29 hineinzudrücken, insbesondere derart, dass die zweite Kammer 25 zumindest im Wesentlichen gasfrei ist und/oder das Volumen des eingeschlossenen Gases in der zweiten Kammer 25 minimiert wird.

Das Freigeben der Fluidverbindung(en) 30 bzw. das Mischen der Bestandteile 2A, 2B erfolgt besonders bevorzugt im ungeöffneten bzw. gas- oder fluiddichten Zustand der Kartusche 3. Insbesondere ist bzw. sind die Kartusche 3, der Behälter 4 bzw. die Kammern 24, 25 auch während bzw. nach dem Freigeben bzw. Mischen abgedichtet bzw. geschlossen, insbesondere nach unten durch den ersten und/oder zweiten Kolben 26, 27 und nach oben durch den Verschluss 6.

Besonders bevorzugt gelten für das Öffnen der Kartusche 3 die gleichen oder entsprechende Ausführungen und Erläuterungen wie bei der ersten Ausführungsform. Insbesondere erfolgt das Öffnen erst nach dem Mischen der Bestandteile 2A, 2B. Das Öffnen kann vor, nach oder durch das Einsetzen in den Zerstäuber 1 erfolgen.

Wie bereits erläutert, ist die Betätigungseinrichtung 31 vorzugsweise - insbesondere vollständig - von der Kartusche 3, insbesondere vom Behälter 4 und/oder Verschluss 6, abnehmbar, insbesondere um nach Mischen der Bestandteile 2A, 2B die Kartusche 3 in den Zerstäuber 1 einsetzen zu können.

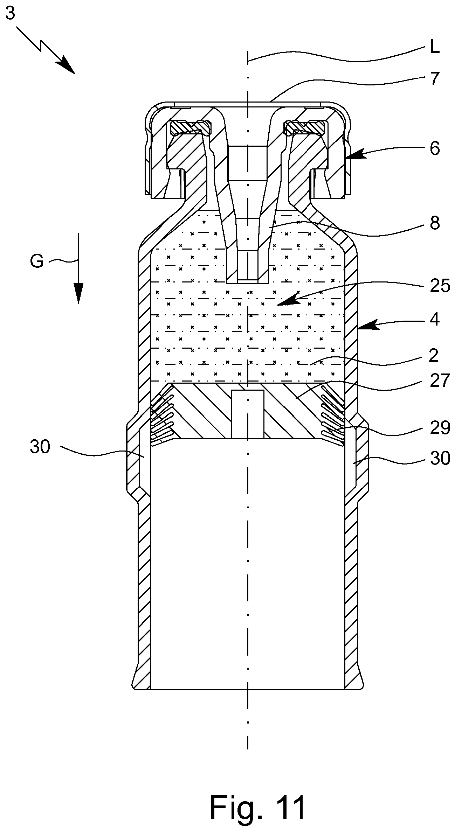

Fig. 11 zeigt die Kartusche 3 mit abgenommener Betätigungseinrichtung 31 und somit im einsatzbereiten Zustand.

Vorzugsweise ist die Betätigungseinrichtung 31, insbesondere das Betätigungselement 32 und/oder das Verbindungsteil 39, zusammen mit dem ersten Kolben 26 abnehmbar bzw. aus der Kartusche 3 bzw. dem Behälter 4 herausziehbar.

Durch Abnehmen des Betätigungselements 32 wird vorzugsweise der erste Kolben 26 von der Kartusche 3 entfernt bzw. aus dem Behälter 4 gezogen. Es sind jedoch auch Lösungen möglich, bei denen sowohl der erste Kolben 26 als auch der zweite Kolben 27 in der Kartusche 3 bzw. im Behälter 4 verbleibt, wie im Zusammenhang mit der ersten Ausführungsform erläutert.

Die optionale Entnahme des ersten Kolbens 26 aus der Kartusche 3 bzw. dem Behälter 4 hat den Vorteil, dass zur Entnahme einer Dosis des Fluids 2 lediglich der zweite Kolben 27 verschoben werden muss und somit weniger Reibung in der Kartusche 3 vorliegt. Auf diese Weise wird die Entnahme einer Dosis des Fluids 2 erleichtert.

Wie bereits im Zusammenhang mit der ersten Ausführungsform erläutert, kann die Entnahme einer Dosis des Fluids 2 bzw. die Bewegung des zweiten Kolbens 27 durch eine optionale Luftpumpe 33 unterstützt werden, wie in Fig. 12 dargestellt.

Fig. 12 zeigt den Zerstäuber 1 mit eingesetzter Kartusche 3 gemäß der zweiten Ausführungsform, wobei - im Gegensatz zu dem in Fig. 6 dargestellten Zerstäuber 1 - der Zerstäuber 1 im gespannten Zustand dargestellt ist. Die im Zusammenhang mit der Fig. 6 gemachten Ausführungen gelten entsprechend.

Optional weist die Kartusche 3, insbesondere der Behälter 4, eine - vorzugsweise integrierte - Mischeinrichtung, wie ein Rührwerk, auf (nicht dargestellt), vorzugsweise wobei die Mischeinrichtung dazu ausgebildet ist, ein Mischen der Bestandteile 2A, 2B zu unterstützen.

Beispielsweise kann die Kartusche 3, insbesondere der Behälter 4, einen Rührer, besonders bevorzugt Schnecken-, Propeller-, Schrägblatt-, Gitter-, Blattrührer o.dgl., aufweisen, vorzugsweise wobei der Rührer fest, insbesondere form-, kraft- und/oder stoffschlüssig, mit dem zweiten Kolben 27 verbunden ist, insbesondere derart, dass eine durch das Betätigungselement 32 auf den zweiten Kolben 27 übertragene Drehbewegung auf den Rührer übertragen wird.

Durch die optionale Mischeinrichtung wird ein Mischen der Bestandteile 2A, 2B in der zweiten Kammer 25 unterstützt.

Zusätzlich oder alternativ kann der zweite Kolben 27 eine Mischeinrichtung aufweisen oder bilden. Beispielsweise kann die der zweiten Kammer 25 zugewandte Seite des zweiten Kolbens 27 aufgeraut sein und/oder Erhöhungen bzw. Vertiefungen aufweisen, vorzugsweise um Verwirbelungen bzw. Turbulenzen im Fluid 2 zu erzeugen und somit den Mischprozess zu unterstützen.

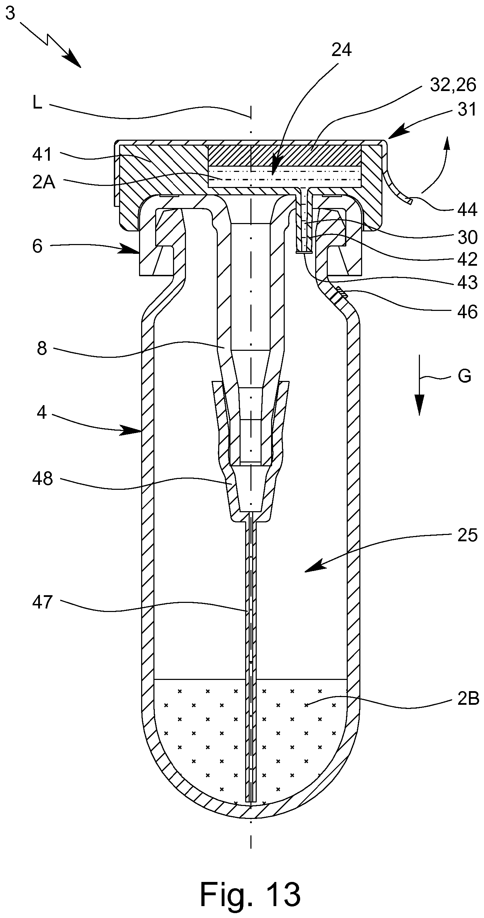

Fig. 13 bis Fig. 16 zeigen die Kartusche 3 gemäß einer dritten Ausführungsform. Vorzugsweise gelten die Ausführungen zu der ersten und/oder zweiten Ausführungsform der Kartusche 3 für die dritte Ausführungsform der Kartusche 3 entsprechend. Insbesondere kann die dritte Ausführungsform bzw. die im Zusammenhang mit Fig. 13 bis Fig. 16 erläuterte Kartusche 3 ein oder mehrere Merkmale der ersten und/oder zweite Ausführungsform bzw. der im Zusammenhang mit Fig. 3 bis Fig. 6 bzw. Fig. 7 bis Fig. 12 erläuterten Kartusche 3 aufweisen und/oder grundsätzlich mit dem im Zusammenhang mit Fig. 1 und Fig. 2 beschriebenen Zerstäuber 1 verwendet werden.

Bei der dritten Ausführungsform ist die Betätigungseinrichtung 31 vorzugsweise am Verschluss 6 der Kartusche 3 angeordnet und/oder mit dem Verschluss 6 der Kartusche 3 - insbesondere form-, kraft- und/oder stoffschlüssig - verbunden.

Insbesondere bildet die Betätigungseinrichtung 31 einen Aufsatz, eine Kappe o.dgl. für die Kartusche 3 bzw. den Verschluss 6. Es sind erfindungsgemäß auch konstruktive Lösungen möglich, bei denen die Betätigungseinrichtung 31 in den Verschluss 6 der Kartusche 3 integriert ist.

Vorzugsweise weist die Betätigungseinrichtung 31 einen Grundkörper 41 auf, vorzugsweise wobei der Grundkörper 41 mit dem Verschluss 6 fest und/oder form-, kraft- und/oder stoffschlüssig verbunden ist.

Bei der dargestellten dritten Ausführungsform ist die Betätigungseinrichtung 31, insbesondere der Grundkörper 41, vorzugsweise auf den Verschluss 6 insbesondere lösbar aufgesteckt, insbesondere derart, dass die Betätigungseinrichtung 31 vom Verschluss 6 abgenommen werden kann.

Vorzugsweise ist die erste Kammer 24 in die Betätigungseinrichtung 31 integriert bzw. enthält die Betätigungseinrichtung 31 die erste Kammer 24 bzw. den ersten Bestandteil 2A des Fluids 2.

Insbesondere bildet die Betätigungseinrichtung 31, vorzugsweise der Grundkörper 41, ein Reservoir bzw. die erste Kammer 24 für den ersten Bestandteil 2A.

Vorzugsweise weist die Betätigungseinrichtung 31 den ersten Kolben 26 auf und/oder ist der erste Kolben 26 in die Betätigungseinrichtung 31 bzw. den Grundkörper 41 eingelassen.

Insbesondere verschließt der erste Kolben 26 die erste Kammer 24 axial nach oben und/oder begrenzt der Grundkörper 41 die erste Kammer 24 seitlich bzw. radial und/oder axial nach unten.

Die erste Kammer 24 ist vorzugsweise zumindest im Wesentlichen zylinderförmig ausgebildet. Hier sind jedoch auch andere Lösungen möglich, insbesondere bei denen die erste Kammer 24 ringförmig ausgebildet ist und/oder sich ringförmig um die Längsachse L bzw. - insbesondere in einer Draufsicht auf die Kartusche 3 - um den Verschluss 6 erstreckt. Besonders bevorzugt weist die Betätigungseinrichtung 31, insbesondere der Grundkörper 41, eine mittige Aussparung bzw. einen mittigen Durchbruch auf, durch die das Förderelement 13 des Zerstäubers 1 in den Verschluss 6 bzw. Anschluss 8 einführbar ist, insbesondere ohne vorheriges Abnehmen der Betätigungseinrichtung 31.

Vorzugsweise bildet das Betätigungselement 32 den Kolben 26 und/oder ist das Betätigungselement 32 einstückig mit dem Kolben 26 ausgebildet.

Insbesondere kann durch Betätigen der Betätigungseinrichtung 31 bzw. des Betätigungselements 32 die Kammer 24 im Grundkörper 41 verkleinert werden, insbesondere derart, dass der in der Kammer 24 enthaltene Bestandteil 2A über die Fluidverbindung 30 in die zweite Kammer 25 gelangt, wie im Folgenden erläutert wird.

Vorzugsweise erstreckt sich bzw. ragt die Betätigungseinrichtung 31, insbesondere der Grundkörper 41, in den Behälter 4 und/oder den Verschluss 6 hinein.

Vorzugsweise weist die Betätigungseinrichtung 31, insbesondere der Grundkörper 41, einen insbesondere länglichen Stutzen 42 auf, vorzugsweise wobei der Stutzen 42 zumindest teilweise in den Behälter 4 bzw. die zweite Kammer 25 hineinragt.

Bei der dargestellten Ausführungsform ist der Grundkörper 41 und der Stutzen 42 einstückig ausgebildet. Hier sind jedoch auch andere Lösungen möglich.

Der Stutzen 42 ist vorzugsweise dazu ausgebildet, die erste Kammer 24 fluidisch mit der zweiten Kammer 25 zu verbinden.

Vorzugsweise weist die Betätigungseinrichtung 31, besonders bevorzugt der Grundkörper41, insbesondere der Stutzen 42, die Fluidverbindung 30 auf oder bildet diese.

Bei der dargestellten Ausführungsform ist der Stutzen 42 zumindest im Wesentlichen zylindrisch bzw. als zylindrischer Hohlkörper ausgebildet und/oder trichterförmig. Es sind jedoch auch andere Lösungen möglich, insbesondere bei denen sich der Stutzen 42 ringartig um die Längsachse L bzw. den Verschluss 6 erstreckt.

Vorzugsweise weist der Verschluss 6 eine Öffnung 45 auf, durch die sich der Stutzen 42 erstreckt bzw. in die die Betätigungseinrichtung 31, besonders bevorzugt der Grundkörper 41, insbesondere der Stutzen 42, eingesetzt ist.

Die erste Kammer 24, insbesondere der Stutzen 42, ist vorzugsweise - zumindest im Auslieferungszustand der Kartusche 3 - verschlossen bzw. versiegelt, insbesondere derart, dass ein Austritt des ersten Bestandteils 2A aus der ersten Kammer 24 in die zweite Kammer 25 verhindert wird.

Besonders bevorzugt weist die Betätigungseinrichtung 31 ein Verschlusselement 43 auf, vorzugsweise wobei das Verschlusselement 43 die erste Kammer 24 bzw. den Stutzen 42 nach unten bzw. gegenüber der zweiten Kammer 25 verschließt. Das Verschlusselement 43 ist vorzugsweise als Siegel, Folie, Stopfen oder dergleichen ausgebildet und/oder am axialen bzw. freien Ende des Stutzens 42 befestigt.

Vorzugsweise ist die Betätigungseinrichtung 31, insbesondere erste Kammer 24, durch Betätigen der Betätigungseinrichtung 31 bzw. des Betätigungselements 32 bzw. des Kolbens 26 öffenbar.

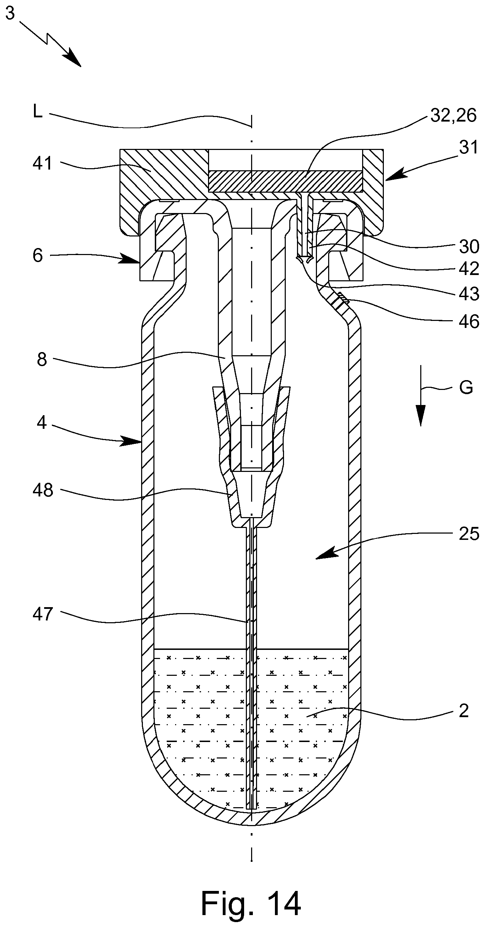

Vorzugsweise wird das Verschlusselement 43 zumindest teilweise geöffnet, weggeklappt und/oder zerstört bzw. zertrennt, wenn die Betätigungseinrichtung 31 bzw. das Betätigungselement 32 bzw. der Kolben 26 betätigt wird, wie in Fig. 14 schematisch dargestellt.

Optional weist die Betätigungseinrichtung 31, insbesondere auf einer dem Behälter 4 abgewandten Seite, eine Abdeckung 44, wie eine Folie o.dgl., auf, vorzugsweise wobei die Abdeckung 44 dazu ausgebildet ist, ein versehentliches Betätigen der Betätigungseinrichtung 31 und/oder Auslaufen der Kammer 24 zu verhindern. Vorzugsweise ist die Abdeckung 44 zur Betätigung der Betätigungseinrichtung 31 vom Grundkörper 41 abnehmbar, insbesondere abziehbar, wie in Fig. 13 angedeutet.

Zum Mischen der Bestandteile 2A, 2B in der Kartusche 3 wird in einem ersten optionalen Schritt die Abdeckung 44 von der Betätigungseinrichtung 31 bzw. dem Grundkörper 41 abgenommen bzw. abgezogen.

Vorzugsweise sind die Kammern 24, 25 auch nach Abnehmen bzw. Abziehen der Abdeckung 44 abgedichtet bzw. geschlossen, insbesondere nach unten durch den Behälter 4 und nach oben durch den Kolben 26.

Anschließend wird die Betätigungseinrichtung 31 bzw. das Betätigungselement 32 betätigt bzw. niedergedrückt, insbesondere derart, dass der erste Bestandteil 2A aus der Betätigungseinrichtung 31, insbesondere der ersten Kammer 24, in den Behälter 4 bzw. die zweite Kammer 25 gedrückt bzw. überführt wird.

Insbesondere wird durch Betätigen der Betätigungseinrichtung 31 bzw. durch Niederdrücken des Betätigungselements 32 bzw. des Kolbens 26 der Druck in der ersten Kammer 24 erhöht, bis das Verschlusselement 43 den Stutzen 42 bzw. die Fluidverbindung 30 freigibt und/oder eine fluidische Verbindung zwischen der ersten Kammer 24 und der zweiten Kammer 25 bzw. zwischen der Betätigungseinrichtung 31 und dem Behälter 4 hergestellt ist.

Anschließend kann durch Niederdrücken des Betätigungselements 32 das Volumen der ersten Kammer 24 verkleinert werden und somit der erste Bestandteil 2A aus der ersten Kammer 24 über den Stutzen 42 bzw. die Fluidverbindung 30 in die zweite Kammer 25 geleitet werden.

Fig. 14 zeigt die Kartusche 3 bei bereits vollständig entleerter Betätigungseinrichtung 31 bzw. Kammer 24.

Auch während bzw. nach dem Betätigen der Betätigungseinrichtung 31 bzw. Niederdrücken des Betätigungselements 32 bzw. des Kolbens 26 ist/sind die Kartusche 3, der Behälter 4 bzw. die Kammern 24, 25 abgedichtet bzw. geschlossen. Besonders bevorzugt gelten für das Öffnen der Kartusche 3 die gleichen oder ähnliche Ausführungen und Erläuterungen wie bei der ersten Ausführungsform. Insbesondere erfolgt das Öffnen erst nach dem Mischen der Bestandteile 2A, 2B. Das Öffnen kann vor, nach oder durch das Einsetzen in den Zerstäuber 1 erfolgen.

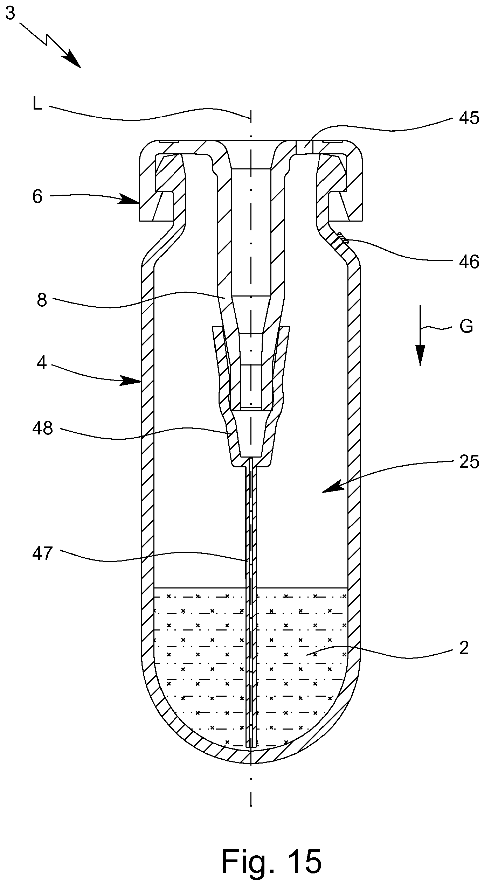

Es ist bevorzugt, die Betätigungseinrichtung 31 nach Mischen der Bestandteile 2A, 2B von der Kartusche 3, insbesondere dem Verschluss 6 abzunehmen bzw. abzuziehen, insbesondere bevor die Kartusche 3 in den Zerstäuber 1 eingesetzt wird. Fig. 15 zeigt eine entsprechende Darstellung der Kartusche 3 mit abgenommener Betätigungseinrichtung 31, so dass die dargestellte Kartusche 3 in den Zerstäuber 1 eingesetzt werden kann.

Es sind jedoch auch Lösungen möglich, bei denen die Betätigungseinrichtung 31 in den Verschluss 6 integriert ist (erfindungsgemäß) und/oder den Verschluss 6 bildet. Wie bereits erläutert, kann die Betätigungseinrichtung 31 beispielsweise eine entsprechende mittige Aussparung bzw. Bohrung aufweisen, so dass das Förderelement 13 durch die Betätigungseinrichtung 31 in den Anschluss 8 eingeführt wird bzw. sich das Förderelement 13 im eingesetzten Zustand der Kartusche 3 durch die Betätigungseinrichtung 31 in den Behälter 4 bzw. die zweite Kammer 25 erstreckt. In diesem Fall ist bevorzugt, die Kartusche 3 zusammen mit der Betätigungseinrichtung 31 in den Zerstäuber 1 einzusetzen.

Vorzugsweise wird die Kartusche 3 durch Abnehmen der Betätigungseinrichtung 31 von der Kartusche 3 bzw. dem Verschluss 6 gegenüber der Umgebung bzw. nach außen geöffnet, insbesondere durch Herausziehen des Stutzens 42 aus Öffnung 45. Die Öffnung 45 kann im eingesetzten Zustand der Kartusche 3 als Belüftung des Behälters 4 dienen. Alternativ kann die Öffnung 45 beispielsweise durch einen Pfropfen und/oder den Halter 11 des Zerstäubers 1 verschlossen werden.

Auch bei der dritten Ausführungsform kann das Öffnen also vor, nach oder durch das Einsetzen in den Zerstäuber 1 erfolgen. Falls die Betätigungseinrichtung 31 zum Einsetzen der Kartusche 3 abnehmbar ist, erfolgt vorzugsweise hierdurch bzw. vor dem Einsetzen in den Zerstäuber 1 ein Öffnen der Kartusche 3, wie zuvor erläutert. Bei Lösungen, in denen die Betätigungseinrichtung 31 zusammen mit der Kartusche 3 in den Zerstäuber 1 eingesetzt wird, ist auch ein Öffnen während oder nach dem Einsetzen möglich.

Ferner gelten für das Öffnen der Kartusche 3 vorzugsweise die gleichen oder entsprechende Ausführungen und Erläuterungen wie bei der ersten und/oder zweiten Ausführungsform. Insbesondere erfolgt das Öffnen erst nach Mischen der Bestandteile 2A, 2B.

Die Kartusche 3 weist vorzugsweise ein optionales Belüftungsventil 46 auf, vorzugsweise wobei das Belüftungsventil 46 in der Wandung des Behälters 4 angeordnet ist und/oder eine fluidische bzw. pneumatische Verbindung der Kartusche 3 nach außen bzw. zu der Umgebung ermöglicht.

Durch die Öffnung 45 und/oder das Belüftungsventil 46 ist vorzugsweise ein Druckausgleich nach außen bzw. zwischen dem Behälter 4 bzw. der zweiten Kammer 25 und der Umgebung möglich.

Bei der dargestellten dritten Ausführungsform verkleinert sich das Volumen der zweiten Kammer 25 bzw. des Behälters 4 während der Entnahme einer Dosis des Fluids 2 vorzugsweise nicht. Durch Spannen des Zerstäubers 1 bzw. durch die Entnahme einer Dosis des Fluids 2 entsteht folglich vorzugsweise ein Unterdruck in der zweiten Kammer 25 bzw. im Behälter 4, der insbesondere durch die Öffnung 45 und/oder das Belüftungsventil 46 ausgeglichen wird.

Zusätzlich ist es mittels des Belüftungsventils 46 möglich, einen Überdruck im Behälter 4 zu vermeiden, wenn die Betätigungseinrichtung 31 betätigt wird bzw. der erste Bestandteil 2A in die zweite Kammer 25 bzw. in den Behälter 4 gedrückt wird. Die dargestellte Kartusche 3 weist vorzugsweise ein insbesondere flexibles Förderrohr 47 und/oder einen Adapter 48 auf, vorzugsweise wobei das Förderrohr 47 insbesondere über den Adapter 48 fluidisch mit dem Verschluss 6 bzw. dem Anschluss 8 verbunden ist.

Das Förderrohr 47 dient vorzugsweise als Verlängerung des Förderelements 13 und/oder ermöglicht eine zumindest im Wesentlichen von der Ausrichtung des Zerstäubers 1 bzw. der Kartusche 3 unabhängige Entnahme des Fluids 2.

Optional ist das Förderrohr 47 als Rührwerk ausgebildet und/oder dient das Förderrohr 47 dazu, ein Mischen der Bestandteile 2A, 2B in der Kartusche 3 bzw. dem Behälter 4 zu unterstützen. Beispielsweise sind konstruktive Lösungen möglich, bei denen das Förderohr 47 spiralförmig ausgebildet ist.

Es sind jedoch auch konstruktive Lösungen möglich, bei denen die Kartusche 3 bzw. der Behälter 4 den im Zusammenhang mit Fig. 1 und Fig. 2 erläuterten flexiblen Beutel 5 aufweist, vorzugsweise wobei der Beutel 5 die zweite Kammer 25 aufweist oder bildet.

Alternativ kann die Kartusche 3 auch bei der dritten Ausführungsform einen zweiten Kolben 27 aufweisen, vorzugsweise wobei der zweite Kolben 27 den Behälter 4 bzw. die zweite Kammer 25 bodenseitig verschließt, wie bereits im Zusammenhang mit der ersten und zweiten Ausführungsform erläutert. Bei derartigen alternativen Konstruktionsweisen kann von dem dargestellten Förderrohr 47, Adapter 48 und/oder Belüftungsventil 46 abgesehen werden.

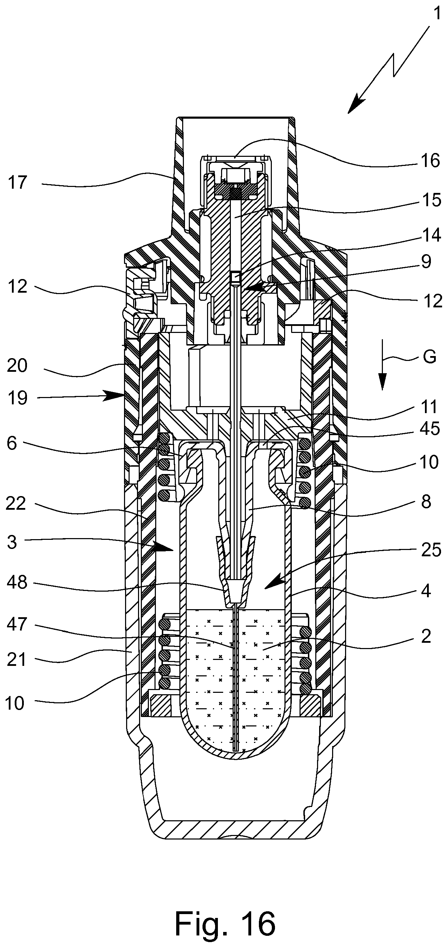

Fig. 16 zeigt den Zerstäuber 1 mit eingesetzter Kartusche 3 gemäß der dritten Ausführungsform.

### Bezugszeichenliste:

- 1: Zerstäuber
- 2: Fluid
- 2A: erste Bestandteil
- 2B: zweite Bestandteil
- 3: Kartusche
- 4: Behälter
- 5: Beutel
- 6: Verschluss
- 7: Siegel
- 8: Anschluss
- 9: Pumpe
- 10: Antriebsfeder
- 11: Halter
- 12: Sperrelement
- 13: Förderelement
- 14: Einwegventil
- 15: Druckkammer
- 16: Düse
- 17: Mundstück
- 18: Belüftungsöffnung
- 19: Gehäuse
- 20: oberes Gehäuseteil
- 21: unteres Gehäuseteil
- 22: innere Gehäuseteil
- 23: Belüftungsmittel
- 24: erste Kammer
- 25: zweite Kammer
- 26: erster Kolben
- 27: zweiter Kolben
- 28: erste Kolbendichtung
- 29: zweite Kolbendichtung
- 30: Fluidverbindung
- 31: Betätigungseinrichtung
- 32: Betätigungselement
- 33: Luftpumpe
- 34: Pumpkolben
- 35: Pumpzylinder
- 36: Pumpkammer
- 37: Pumpventil
- 38: Pumpbelüftung
- 39: Verbindungsteil
- 39A: erstes Verbindungselement
- 39B: zweites Verbindungselement
- 40: Belüftung
- 41: Grundkörper
- 42: Stutzen
- 43: Verschlusselement
- 44: Abdeckung
- 45: Öffnung
- 46: Belüftungsventil
- 47: Förderrohr
- 48: Adapter

- A: Aerosol
- G: Schwerkraft
- L: Längsachse

## Patentansprüche

1. Kartusche (3) für eine Abgabevorrichtung, insbesondere einen Zerstäuber (1), zur Abgabe eines Fluids (2), das ein aus mindestens zwei Bestandteilen (2A, 2B) erzeugtes Arzneimittel ist,
wobei die Kartusche (3) einen Behälter (4) und einen Verschluss (6) zum fluidischen und/oder dichtenden Anschluss des Behälters (4) an die Abgabevorrichtung aufweist,
wobei die Kartusche (3) mindestens zwei fluidisch voneinander getrennte Kammern (24, 25) aufweist, wobei die Kammern (24, 25) jeweils mindestens einen der Bestandteile (2A, 2B) des Fluids (2) enthalten,
wobei die Kartusche (3) eine Betätigungseinrichtung (31) aufweist, wobei die Kammern (24, 25) durch Betätigung der Betätigungseinrichtung (31) fluidisch miteinander verbindbar sind, um durch Zusammenführen der Bestandteile (2A, 2B) das Fluid (2) zu erzeugen,
wobei die Kartusche (3) zwei bewegbare Kolben (26, 27) aufweist,
wobei der Behälter (4) mindestens eine Fluidverbindung (30) aufweist oder bildet, wobei die Fluidverbindung (30) dazu ausgebildet ist, die Kammern (24, 25) fluidisch miteinander zu verbinden und/oder eine Überführung des ersten Bestandteils (2A) von der ersten Kammer (24) in die zweite Kammer (25) zu ermöglichen, und
wobei die Betätigungseinrichtung (31) ein Betätigungselement (32) aufweist, wobei die Betätigungseinrichtung (31) durch Abnehmen des Betätigungselements (32) vom Behälter (4) oder Verschluss (6) oder durch Öffnen bzw. Zertrennen des Betätigungselements (32) betätigbar ist, und
wobei die Betätigungseinrichtung dazu ausgebildet ist, die Kolben in der Kartusche im unbetätigten Zustand der Betätigungseinrichtung gegen ein Verschieben zu sichern,
wobei durch Betätigen der Betätigungseinrichtung (31) die Kolben (26, 27) zum Verschieben in der Kartusche (3) bzw. im Behälter (4) freigebbar sind,
wobei die Fluidverbindung (30) durch Verschieben des ersten Kolbens (26) und des zweiten Kolbens (27) derart freigebbar ist, dass die Kammern (24, 25) fluidisch miteinander verbunden werden,
**dadurch gekennzeichnet,**
**dass** die Kolben (26, 27) derart freigebbar sind, dass sich die Kolben (26, 27) selbsttätig in der Kartusche (3) bzw. im Behälter (4) verschieben.

2. Kartusche nach Anspruch 1, **dadurch gekennzeichnet, dass** sich die Kolben (26, 27) durch eine in Richtung einer der Kammern (24, 25) wirkende Kraft, besonders bevorzugt durch eine Zugkraft, in der Kartusche (3) bzw. im Behälter (4) verschieben, insbesondere wobei die Kraft durch einen Unterdruck in einer der Kammern (24, 25) erzeugt ist.

3. Kartusche (3) für eine Abgabevorrichtung, insbesondere einen Zerstäuber (1), zur Abgabe eines Fluids (2), das ein aus mindestens zwei Bestandteilen (2A, 2B) erzeugtes Arzneimittel ist,
wobei die Kartusche (3) einen Behälter (4) und einen Verschluss (6) zum fluidischen und/oder dichtenden Anschluss des Behälters (4) an die Abgabevorrichtung aufweist,
wobei die Kartusche (3) mindestens zwei fluidisch voneinander getrennte Kammern (24, 25) aufweist, wobei die Kammern (24, 25) jeweils mindestens einen der Bestandteile (2A, 2B) des Fluids (2) enthalten,
wobei die Kartusche (3) eine Betätigungseinrichtung (31) aufweist, wobei die Kammern (24, 25) durch Betätigung der Betätigungseinrichtung (31) fluidisch miteinander verbindbar sind, um durch Zusammenführen der Bestandteile (2A, 2B) das Fluid (2) zu erzeugen,
wobei die Kartusche (3) mindestens einen oder zwei bewegbare Kolben (26, 27) aufweist,
wobei der Behälter (4) mindestens eine Fluidverbindung (30) aufweist oder bildet,
wobei die Fluidverbindung (30) dazu ausgebildet ist, die Kammern (24, 25) fluidisch miteinander zu verbinden und/oder eine Überführung des ersten Bestandteils (2A) von der ersten Kammer (24) in die zweite Kammer (25) zu ermöglichen und
**dadurch gekennzeichnet,**
**dass** durch Betätigen der Betätigungseinrichtung (31) die Fluidverbindung (30) öffenbar ist, wobei durch Drehen der Betätigungseinrichtung (31) oder eines Betätigungselements (32) der Betätigungseinrichtung (31) der/die Kolben (26, 27) in der Kartusche (3) verschoben wird/werden und/oder mindestens eine der Kammern (24, 25), vorzugsweise alle Kammern (24, 25), verkleinert wird /werden.

4. Kartusche nach Anspruch 3, **dadurch gekennzeichnet, dass** die Betätigungseinrichtung (31) bzw. das Betätigungselement (32) mit dem/den Kolben (26, 27) - insbesondere mittels eines Verbindungsteils (39) - mechanisch gekoppelt ist.

5. Kartusche nach einem der voranstehenden Ansprüche, **dadurch gekennzeichnet, dass** die Betätigungseinrichtung (31) - insbesondere nach Betätigung der Betätigungseinrichtung (31) - vom Behälter (4) abnehmbar, insbesondere abdrehbar oder abziehbar, ist, um die Kartusche (3) in die Abgabevorrichtung einzusetzen.

6. Kartusche nach Anspruch 3 oder 4, **dadurch gekennzeichnet, dass** die Fluidverbindung (30) ein Bypass ist.

7. Kartusche (3) für eine Abgabevorrichtung, insbesondere einen Zerstäuber (1), zur Abgabe eines Fluids (2), das ein aus mindestens zwei Bestandteilen (2A, 2B) erzeugtes Arzneimittel ist,
wobei die Kartusche (3) einen Behälter (4) und einen Verschluss (6) zum fluidischen und/oder dichtenden Anschluss des Behälters (4) an die Abgabevorrichtung aufweist,
wobei die Kartusche (3) mindestens zwei fluidisch voneinander getrennte Kammern (24, 25) aufweist, wobei die Kammern (24, 25) jeweils mindestens einen der Bestandteile (2A, 2B) des Fluids (2) enthalten,
wobei die Kartusche (3) eine Betätigungseinrichtung (31) aufweist, wobei die Kammern (24, 25) durch Betätigung der Betätigungseinrichtung (31) fluidisch miteinander verbindbar sind, um durch Zusammenführen der Bestandteile (2A, 2B) das Fluid (2) zu erzeugen,
wobei die Kartusche (3) mindestens einen bewegbaren Kolben (26) aufweist,
wobei der Behälter (4) mindestens eine Fluidverbindung (30) aufweist oder bildet,
wobei die Fluidverbindung (30) dazu ausgebildet ist, die Kammern (24, 25) fluidisch miteinander zu verbinden und/oder eine Überführung des ersten Bestandteils (2A) von der ersten Kammer (24) in die zweite Kammer (25) zu ermöglichen und
**dadurch gekennzeichnet,**
**dass** durch Betätigen der Betätigungseinrichtung (31) die Fluidverbindung (30) öffenbar ist und/oder die Kammern (24, 25) über die Fluidverbindung (30) fluidisch miteinander verbindbar sind, und
**dass** die Betätigungseinrichtung (31) in den Verschluss (6) integriert ist, wobei die Betätigungseinrichtung (31) durch Niederdrücken des Kolbens (26) betätigbar ist.

8. Kartusche nach Anspruch 7, **dadurch gekennzeichnet, dass** eine der Kammern (24, 25) in die Betätigungseinrichtung (31) integriert ist bzw. dass die Betätigungseinrichtung (31) eine der Kammern (24, 25) bzw. einen der Bestandteile (2A, 2B) enthält, und insbesondere wobei die andere Kammer (25, 24) durch den Behälter (4) gebildet ist bzw. der Behälter (4) den anderen Bestandteil (2B, 2A) enthält.

9. Kartusche nach einem der voranstehenden Ansprüche, **dadurch gekennzeichnet, dass** der/die Kolben (26, 27) (jeweils) eine Wand der Kammern (24, 25) bildet/bilden und/oder dass mindestens eine der Kammern (24, 25) oder alle Kammern (24,25) durch Betätigen der Betätigungseinrichtung (31) verkleinerbar ist / sind.

10. Kartusche nach einem der voranstehenden Ansprüche, **dadurch gekennzeichnet, dass** die Betätigungseinrichtung (31) abnehmbar ist.

11. Kartusche nach einem der voranstehenden Ansprüche, **dadurch gekennzeichnet, dass** durch Zusammenführen der Bestandteile (2A, 2B) ein liposomales Arzneimittel als Fluid (2) erzeugbar ist.

12. Kartusche nach einem der voranstehenden Ansprüche, **dadurch gekennzeichnet, dass** eine erste Kammer (24) eine insbesondere ethanolische Lipidlösung als ersten Bestandteil (2A) des Arzneimittels und eine zweite Kammer (25) einen wässrigen Puffer als zweiten Bestandteil (2B) enthält, um durch Zusammenführen der Bestandteile (2A, 2B) ein liposomales Arzneimittel zu erzeugen.

13. Kartusche nach einem der Ansprüche 1 bis 11, **dadurch gekennzeichnet, dass** ein Bestandteil (2A, 2B) eine Flüssigkeit, insbesondere ein Lösungs- oder Suspensionsmittel, und/oder ein (anderer) Bestandteil (2A, 2B) ein - insbesondere fester, lyophilisierter, verkapselter und/oder pulverförmiger -Arzneistoff ist.

14. Kartusche nach einem der voranstehenden Ansprüche, **dadurch gekennzeichnet, dass** die Kartusche (3), insbesondere der Behälter (4) und/oder der zweite Kolben (27), eine Mischeinrichtung, insbesondere ein Rührwerk, aufweist oder bildet, insbesondere um ein Mischen der Bestandteile (2A, 2B) zu unterstützen.

15. System mit einer Kartusche (3) und einer Abgabevorrichtung, insbesondere einem Zerstäuber (1), zur Abgabe eines Fluids (2), das ein aus mindestens zwei Bestandteilen (2A, 2B) erzeugtes Arzneimittel ist,
wobei die Abgabevorrichtung ein Gehäuse (19) zur Aufnahme der Kartusche (3) und eine Pumpe (9) aufweist,
wobei die Pumpe (9) zur Entnahme einer Dosis des Fluids (2) aus der Kartusche (3) und zur Druckbeaufschlagung der entsprechenden Dosis für die Zerstäubung des Fluids (2) ausgebildet ist,
**dadurch gekennzeichnet,**
**dass** die Kartusche (3) nach einem der voranstehenden Ansprüche ausgebildet ist, insbesondere wobei die Kartusche (3) in die Abgabevorrichtung einsetzbar und/oder aus der Abgabevorrichtung herausnehmbar bzw. austauschbar ist.

16. System nach Anspruch 15, **dadurch gekennzeichnet, dass** die Kartusche (3) mehrere Dosen des Fluids (2) aufweist, insbesondere um mindestens 100 Abgabeeinheiten bzw. Dosen zur Verfügung zu stellen.

17. Verfahren zur Vorbereitung eines Systems, das ein System nach Anspruch 15 oder 16 für die Abgabe eines Fluids (2), das ein aus mindestens zwei Bestandteilen (2A, 2B) erzeugtes Arzneimittel ist, aus der Kartusche (3) ist, für die Abgabe mittels der Abgabevorrichtung, insbesondere eines Zerstäubers (1), wobei die Kartusche (3) ein Siegel (7) aufweist,
wobei mehrere zunächst fluidisch voneinander getrennte Kammern (24, 25) der Kartusche (3) fluidisch miteinander verbunden werden,
wobei ein Bestandteil (2A, 2B) des Fluids (2) aus einer der Kammern (24, 25) zumindest teilweise in eine andere der Kammern (24, 25) mit einem weiteren Bestandteil (2A, 2B) des Fluids (2) überführt wird, um die Bestandteile (2A, 2B) zu mischen und/oder das Fluid (2) zu erzeugen,
wobei eine Betätigungseinrichtung insbesondere manuell betätigt wird, um die Kammern fluidisch miteinander zu verbinden, und die Bestandteile (2A, 2B) gemischt werden, und
wobei die Kartusche (3) erst nach dem Mischen der Bestandteile (2A, 2B) in die Abgabevorrichtung eingesetzt und erst anschließend zur Entnahme einer Dosis des Fluids (2) in der Abgabevorrichtung geöffnet wird,
wobei ein Öffnen der Kartusche durch Öffnen, insbesondere Durchstechen, des Siegels (7) erfolgt.

18. Verfahren nach Anspruch 17, **dadurch gekennzeichnet, dass** das Mischen der Bestandteile (2A, 2B) unabhängig von der Abgabevorrichtung erfolgt.

19. Verfahren nach einem der Ansprüche 17 oder 18, **dadurch gekennzeichnet, dass** durch Betätigung einer Betätigungseinrichtung (31) einer der Bestandteile (2A, 2B) selbsttätig von einer in die andere Kammer (24, 25) überführt wird,
insbesondere wobei einer der Bestandteile (2A, 2B) durch eine durch Unterdruck in einer der Kammern (24, 25) erzeugte Kraft von einer in die andere Kammer (24, 25) überführt wird oder die Kartusche (3) nach Verbinden der Kammern (24, 25) bzw. nach Freigeben einer Fluidverbindung (30) zwischen den Kammern (24, 25) derart ausgerichtet wird, dass ein Bestandteil (2A, 2B) - insbesondere ausschließlich - durch Schwerkraft (G) von einer in die andere Kammer (24, 25) überführt wird.

20. Verfahren nach einem der Ansprüche 17 bis 19, **dadurch gekennzeichnet, dass** eine Betätigungseinrichtung (31) zum Verbinden der Kammern (24, 25) nach Betätigung der Betätigungseinrichtung (31) von der Kartusche (3) abgenommen wird, insbesondere um die Kartusche (3) in die Abgabevorrichtung einzusetzen.

21. Verfahren nach einem der Ansprüche 17 bis 20, **dadurch gekennzeichnet, dass** durch Mischen der Bestandteile (2A, 2B) ein liposomales Arzneimittel in der Kartusche (3) erzeugt wird.

## Claims

1. Cartridge (3) for a dispensing device, in particular a nebulizer (1), for dispensing a fluid (2) which is a medicament produced from at least two components (2A, 2B),
wherein the cartridge (3) comprises a container (4) and a closure (6) for the fluidic and/or sealing connection of the container (4) to the dispensing device,
wherein the cartridge (3) comprises at least two fluidically separated chambers (24, 25), with the chambers (24, 25) each containing at least one of the components (2A, 2B) of the fluid (2),
wherein the cartridge (3) comprises an actuating mechanism (31), with the chambers (24, 25) being able to be fluidically connected to one another by actuating the actuating mechanism (31) in order to produce the fluid (2) by bringing the components (2A, 2B) together,
wherein the cartridge (3) comprises two movable plungers (26, 27),
wherein the container (4) comprises or forms at least one fluid connection (30), wherein the fluid connection (30) is designed to fluidically connect the chambers (24, 25) to one another and/or to allow a transfer of the first component (2A) from the first chamber (24) into the second chamber (25), and
wherein the actuating mechanism (31) comprises an actuating element (32), wherein the actuating mechanism (31) can be actuated by removing the actuating element (32) from the container (4) or closure (6) or by opening or separating the actuating element (32), and
wherein the actuating mechanism is designed to secure the plungers against displacement in the cartridge in the non-actuated state of the actuating mechanism, wherein
the plungers (26, 27) in the cartridge (3) or in the container (4) can be released for displacement by actuating the actuating mechanism (31), wherein the fluid connection (30) can be opened by displacing the first plunger (26) and the second plunger (27) in such a way that the chambers (24, 25) are fluidically connected to one another,
**characterized in that**
the plungers (26, 27) can be released in such a way that the plungers (26, 27) are displaced automatically in the cartridge (3) or in the container (4).

2. Cartridge according to claim 1, **characterized in that** the plungers (26, 27) are displaced in the cartridge (3) or in the container (4) by a force, particularly preferably by a tensile force, acting in the direction of one of the chambers (24, 25), in particular wherein the force is generated by a negative pressure in one of the chambers (24, 25).

3. Cartridge (3) for a dispensing device, in particular a nebulizer (1), for dispensing a fluid (2) which is a medicament produced from at least two components (2A, 2B),
wherein the cartridge (3) comprises a container (4) and a closure (6) for the fluidic and/or sealing connection of the container (4) to the dispensing device,
wherein the cartridge (3) comprises at least two fluidically separated chambers (24, 25), with the chambers (24, 25) each containing at least one of the components (2A, 2B) of the fluid (2),
wherein the cartridge (3) comprises an actuating mechanism (31), with the chambers (24, 25) being able to be fluidically connected to one another by actuating the actuating mechanism (31) in order to produce the fluid (2) by bringing the components (2A, 2B) together,
wherein the cartridge (3) comprises at least one or two movable plungers (26, 27),
wherein the container (4) comprises or forms at least one fluid connection (30), wherein the fluid connection (30) is designed to fluidically connect the chambers (24, 25) to one another and/or to allow a transfer of the first component (2A) from the first chamber (24) to the second chamber (25), and
**characterized in that**
the fluid connection (30) can be opened by actuating the actuating mechanism (31),
wherein by turning the actuating mechanism (31) or an actuating element (32) of the actuating mechanism (31), the plunger(s) (26, 27) in the cartridge (3) is/are displaced and/or at least one of the chambers (24, 25), preferably all of the chambers (24, 25), is/are reduced in size.

4. Cartridge according to claim 3, **characterized in that** the actuating mechanism (31) or the actuating element (32) is mechanically coupled to the plunger(s) (26, 27), in particular by means of a connecting part (39).

5. Cartridge according to any of the preceding claims, **characterized in that** the actuating mechanism (31) - in particular after actuation of the actuating mechanism (31) - can be removed from the container (4), in particular twisted off or pulled off, to insert the cartridge (3) into the dispensing device.

6. Cartridge according to claim 3 or 4, **characterized in that** the fluid connection (30) is a bypass.

7. Cartridge (3) for a dispensing device, in particular a nebulizer (1), for dispensing a fluid (2) which is a medicament produced from at least two components (2A, 2B),
wherein the cartridge (3) comprises a container (4) and a closure (6) for the fluidic and/or sealing connection of the container (4) to the dispensing device,
wherein the cartridge (3) comprises at least two fluidically separated chambers (24, 25), with the chambers (24, 25) each containing at least one of the components (2A, 2B) of the fluid (2),
wherein the cartridge (3) comprises an actuating mechanism (31), with the chambers (24, 25) being able to be fluidically connected to one another by actuating the actuating mechanism (31) in order to produce the fluid (2) by bringing the components (2A, 2B) together,
wherein the cartridge (3) has at least one movable plunger (26),
wherein the container (4) comprises or forms at least one fluid connection (30), wherein the fluid connection (30) is designed to fluidically connect the chambers (24, 25) to one another and/or to allow a transfer of the first component (2A) from the first chamber (24) to the second chamber (25), and
**characterized in that**
the fluid connection (30) can be opened by actuating the actuating mechanism (31) and/or the chambers (24, 25) can be fluidically connected to one another via the fluid connection (30), and
**in that** the actuating mechanism (31) is integrated into the closure (6), wherein the actuating mechanism (31) can be actuated by pressing down the plunger (26).

8. Cartridge according to claim 7, **characterized in that** one of the chambers (24, 25) is integrated into the actuating mechanism (31) or **in that** the actuating mechanism (31) contains one of the chambers (24, 25) or one of the components (2A, 2B), and in particular wherein the other chamber (25, 24) is formed by the container (4) or the container (4) contains the other component (2B, 2A).

9. Cartridge according to any of the preceding claims, **characterized in that** the plunger(s) (26, 27) (each) form(s) a wall of the chambers (24, 25) and/or
**in that** at least one of the chambers (24, 25) or all of the chambers (24, 25) can be reduced in size by actuating the actuating mechanism (31).

10. Cartridge according to any of the preceding claims, **characterized in that** the actuating mechanism (31) is removable.

11. Cartridge according to any of the preceding claims, **characterized in that** a liposomal medicament can be produced as a fluid (2) by bringing the components (2A, 2B) together.

12. Cartridge according to any of the preceding claims, **characterized in that** a first chamber (24) contains a solution, in particular an ethanolic lipid solution, as the first component (2A) of the medicament, and a second chamber (25) contains an aqueous buffer as the second component (2B) in order to produce a liposomal medicament by combining the components (2A, 2B).

13. Cartridge according to any of claims 1 to 11, **characterized in that** one component (2A, 2B) is a liquid, in particular a solvent or suspension medium, and/or one (other) component (2A, 2B) is a medicinal agent, in particular a solid, lyophilized, encapsulated and/or powdered medicinal agent.

14. Cartridge according to any of the preceding claims, **characterized in that** the cartridge (3), in particular the container (4) and/or the second plunger (27), comprises or forms a mixing device, in particular a stirrer, in particular to support a mixing of the components (2A, 2B).

15. System having a cartridge (3) and a dispensing device, in particular a nebulizer (1), for dispensing a fluid (2) which is a medicament produced from at least two components (2A, 2B),
wherein the dispensing device has a housing (19) for accommodating the cartridge (3) and a pump (9),
wherein the pump (9) is designed to withdraw a dose of the fluid (2) from the cartridge (3) and to apply pressure to the corresponding dose for nebulizing the fluid (2),
**characterized in that**
the cartridge (3) is designed according to any of the preceding claims, in particular wherein the cartridge (3) is insertable into the dispensing device and/or removable from the dispensing device or replaceable.

16. System according to claim 15, **characterized in that** the cartridge (3) comprises a plurality of doses of the fluid (2), in particular in order to provide at least 100 dispensing units or doses.

17. Method for preparing a system, which is a system according to claim 15 or 16 for dispensing a fluid (2), which is a medicament produced from at least two components (2A, 2B), from a cartridge (3), for dispensing by means of a dispensing device, in particular a nebulizer (1), wherein the cartridge (3) comprises a seal (7),
wherein a plurality of chambers (24, 25) of the cartridge (3) which are initially fluidically separated from one another are fluidically connected to one another,
wherein a component (2A, 2B) of the fluid (2) is at least partially transferred from one of the chambers (24, 25) to another of the chambers (24, 25) with a further component (2A, 2B) of the fluid (2) in order to mix the components (2A, 2B) and/or to produce the fluid (2),
wherein the actuating mechanism is actuated, in particular manually, in order to fluidically connect the chambers to one another, and the components (2A, 2B) are mixed, and
wherein the cartridge (3) is only inserted into the dispensing device after the components (2A, 2B) have been mixed and is only afterwards opened in the dispensing device for the withdrawal of a dose of the fluid (2),
wherein an opening of the cartridge (3) is achieved by opening, in particular piercing, the seal (7).

18. Method according to claim 17, **characterized in that** the mixing of the components (2A, 2B) is independently of the dispensing device.

19. Method according to any of claims 17 or 18, **characterized in that** one of the components (2A, 2B) is automatically transferred from one chamber to the other chamber (24, 25) by actuating an actuating mechanism (31),
in particular wherein one of the components (2A, 2B) is transferred from one chamber to the other chamber (24, 25) by a force generated by negative pressure in one of the chambers (24, 25) or the cartridge (3) is, after the chambers (24, 25) have been connected or after a fluid connection (30) between the chambers (24, 25) has been opened, aligned such that a component (2A, 2B) is transferred from one chamber to the other chamber (24, 25) - in particular exclusively - by gravity (G).

20. Method according to any of claims 17 to 19, **characterized in that** an actuating mechanism (31) for connecting the chambers (24, 25) is removed from the cartridge (3) after the actuating mechanism (31) has been actuated, in particular in order to insert the cartridge (3) into the dispensing device.

21. Method according to any of claims 17 to 20, **characterized in that**, by mixing the components (2A, 2B), a liposomal medicament is produced in the cartridge (3).

## Revendications

1. Cartouche (3) pour un dispositif de distribution, en particulier un pulvérisateur (1), destiné à distribuer un fluide (2), qui est un médicament produit à partir d'au moins deux constituants (2A, 2B), dans laquelle la cartouche (3) présente un contenant (4) et une fermeture (6) pour le raccordement fluidique et/ou étanche du contenant (4) au dispositif de distribution,
dans laquelle la cartouche (3) présente au moins deux chambres (24, 25) séparées l'une de l'autre fluidiquement, dans laquelle les chambres (24, 25) contiennent respectivement au moins un des constituants (2A, 2B) du fluide (2),
dans laquelle la cartouche (3) présente un dispositif d'actionnement (31), dans lequel les chambres (24, 25) peuvent être reliées l'une à l'autre de manière fluidique par l'actionnement du dispositif d'actionnement (31) pour produire le fluide (2) par l'assemblage des constituants (2A, 2B),
dans laquelle la cartouche (3) présente deux pistons (26, 27) pouvant être déplacés,
dans laquelle le contenant (4) présente ou forme au moins une liaison fluidique (30), dans laquelle la liaison fluidique (30) est réalisée pour relier entre elles de manière fluidique les chambres (24, 25) et/ou pour permettre un transfert du premier constituant (2A) de la première chambre (24) dans la deuxième chambre (25), et
dans laquelle le dispositif d'actionnement (31) présente un élément d'actionnement (32), dans laquelle le dispositif d'actionnement (31) peut être actionné en retirant l'élément d'actionnement (32) du contenant (4) ou en ferlant (6) ou en ouvrant ou découpant l'élément d'actionnement (32), et
dans laquelle le dispositif d'actionnement est réalisé pour empêcher tout coulissement des pistons dans la cartouche dans l'état non actionné du dispositif d'actionnement,
dans laquelle les pistons (26, 27) peuvent être débloqués par l'actionnement du dispositif d'actionnement (31) pour coulisser dans la cartouche (3) ou dans le contenant (4),
dans laquelle la liaison fluidique (30) peut être débloquée en faisant coulisser le premier piston (26) et le deuxième piston (27) de telle manière que les chambres (24, 25) sont reliées l'une à l'autre de manière fluidique,
**caractérisée en ce que**
les pistons (26, 27) peuvent être débloqués de telle manière que les pistons (26, 27) coulissent de manière autonome dans la cartouche (3) ou dans le contenant (4).

2. Cartouche selon la revendication 1, **caractérisée en ce que** les pistons (26, 27) coulissent du fait d'une force agissant en direction d'une des chambres (24, 25), de manière particulièrement préférée du fait d'une force de traction, dans la cartouche (3) ou dans le contenant (4), en particulier dans laquelle la force est produite par une dépression dans l'une des chambres (24, 25).

3. Cartouche (3) pour un dispositif de distribution, en particulier un pulvérisateur (1), destiné à distribuer un fluide (2), qui est un médicament produit à partir d'au moins deux constituants (2A, 2B),
dans laquelle la cartouche (3) présente un contenant (4) et une fermeture (6) pour raccorder de manière fluidique et/ou de manière étanche le contenant (4) au dispositif de distribution,
dans laquelle la cartouche (3) présente au moins deux chambres (24, 25) séparées l'une de l'autre de manière fluidique, dans laquelle les chambres (24, 25) contiennent respectivement au moins un des constituants (2A, 2B) du fluide (2),
dans laquelle la cartouche (3) présente un système d'actionnement (31), dans laquelle les chambres (24, 25) peuvent être reliées l'une à l'autre de manière fluidique par l'actionnement du système d'actionnement (31) pour produire le fluide (2) par l'assemblage des constituants (2A, 2B),
dans laquelle la cartouche (3) présente au moins un ou deux pistons (26, 27) pouvant être déplacés,
dans laquelle
le contenant (4) présente ou forme au moins une liaison fluidique (30),
dans laquelle la liaison fluidique (30) est réalisée pour relier l'une à l'autre de manière fluidique les chambres (24, 25), et/ou pour permettre un transfert du premier contenant (2A) de la première chambre (24) dans la deuxième chambre (25),
**caractérisée en ce que**
la liaison fluidique (30) peut être ouverte par l'actionnement du système d'actionnement (31), dans laquelle
le piston/les pistons (26, 27) est coulissé/sont coulissés dans la cartouche (3) par la rotation du système d'actionnement (31) ou d'un élément d'actionnement (32) du système d'actionnement (31) et/ou au moins une des chambres (24, 25), de préférence toutes les chambres (24, 25), est réduite/sont réduites.

4. Cartouche selon la revendication 3, **caractérisée en ce que** le système d'actionnement (31) ou l'élément d'actionnement (32) est couplé mécaniquement au piston/aux pistons (26, 27) en particulier au moyen d'une partie de liaison (39).

5. Cartouche selon l'une quelconque des revendications précédentes, **caractérisée en ce que** le système d'actionnement (31) peut être retiré du contenant (4) - en particulier après l'actionnement du système d'actionnement (31) -, en particulier peut être enlevé par rotation ou peut être tiré pour insérer la cartouche (3) dans le dispositif de distribution.

6. Cartouche selon la revendication 3 ou 4, **caractérisée en ce que** la liaison fluidique (30) est une dérivation bypass.

7. Cartouche (3) pour un dispositif de distribution, en particulier un pulvérisateur (1), destiné à distribuer un fluide (2), qui est un médicament produit à partir d'au moins deux composants (2A, 2B), dans laquelle la cartouche (3) présente un contenant (4) et une fermeture (6) pour le raccordement fluidique et/ou étanche du contenant (4) au dispositif de distribution,
dans laquelle la cartouche (3) présente au moins deux chambres (24, 25) séparées l'une de l'autre de manière fluidique, dans laquelle les chambres (24, 25) contiennent respectivement au moins un des constituants (2A, 2B) du fluide (2),
dans laquelle la cartouche (3) présente un système d'actionnement (31), dans laquelle les chambres (24, 25) peuvent être reliées les unes aux autres de manière fluidique par l'actionnement du système d'actionnement (31) pour produire le fluide (2) en assemblant les constituants (2A, 2B),
dans laquelle la cartouche (3) présente au moins un piston (26) pouvant être déplacé,
dans laquelle
le contenant (4) présente ou forme au moins une liaison fluidique (30),
dans laquelle la liaison fluidique (30) est réalisée pour relier l'une à l'autre de manière fluidique les chambres (24, 25) et/ou pour permettre un transfert du premier constituant (2A) de la première chambre (24) dans la deuxième chambre (25), et
**caractérisée en ce que**
la liaison fluidique (30) peut être ouverte en actionnant le système d'actionnement (31) et/ou les chambres (24, 25) peuvent être reliées de manière fluidique les uns aux autres par l'intermédiaire de la liaison fluidique (30),
et
que le système d'actionnement (31) est intégré dans la fermeture (6), dans laquelle le système d'actionnement (31) peut être actionné en enfonçant le piston (26).

8. Cartouche selon la revendication 7, **caractérisée en ce qu'**une des chambres (24, 25) est intégrée dans le système d'actionnement (31) ou que le système d'actionnement (31) contient une des chambres (24, 25) ou un des constituants (2A, 2B), et en particulier dans laquelle l'autre chambre (25, 24) est formée par le contenant (4) ou le contenant (4) contient l'autre constituant (2B, 2A).

9. Cartouche selon l'une quelconque des revendications précédentes, **caractérisée en ce que** le piston/les pistons (26, 27) forme/forment (respectivement) une paroi des chambres (24, 25), et/ou qu'au moins une des chambres (24, 25) ou toutes les chambres (24, 25) peut être réduite/peuvent être réduites en actionnant le système d'actionnement (31).

10. Cartouche selon l'une quelconque des revendications précédentes, **caractérisée en ce que** le système d'actionnement (31) peut être retiré.

11. Cartouche selon l'une quelconque des revendications précédentes, **caractérisée en ce qu'**un médicament sous forme liposomale peut être produit en tant que fluide (2) en assemblant les constituants (2A, 2B) .

12. Cartouche selon l'une quelconque des revendications précédentes, **caractérisée en ce qu'**une première chambre (24) contient une solution lipidique en particulier à base d'éthanol en tant que premier constituant (2A) du médicament et une deuxième chambre (25) contient un tampon aqueux en tant que deuxième composant (2B) pour produire un médicament sous forme liposomale en assemblant les constituants (2A, 2B).

13. Cartouche selon l'une quelconque des revendications 1 à 11, **caractérisée en ce qu'**un constituant (2A, 2B) est un liquide, en particulier un solvant ou un agent de suspension, et/ou un (autre) constituant (2A, 2B) est un médicament en particulier solide, lyophilisé, encapsulé et/ou pulvérulent.

14. Cartouche selon l'une quelconque des revendications précédentes, **caractérisée en ce que** la cartouche (3), en particulier le contenant (4) et/ou le deuxième piston (27), présente ou forme un système de mélange, en particulier un mécanisme d'agitation, en particulier pour soutenir un mélange des constituants (2A, 2B).

15. Système avec une cartouche (3) et un dispositif de distribution, en particulier un pulvérisateur (1), destiné à distribuer un fluide (2), qui est un médicament produit à partir d'au moins deux constituants (2A, 2B),
dans lequel le dispositif de distribution présente un boîtier (19) pour recevoir la cartouche (3) et une pompe (9),
dans lequel la pompe (9) est réalisée pour prélever une dose du fluide (2) hors de la cartouche (3) et pour soumettre à l'action d'une pression la dose correspondante pour la pulvérisation du fluide (2),
**caractérisé en ce**
**que** la cartouche (3) est réalisée selon l'une quelconque des revendications précédentes, en particulier dans lequel la cartouche (3) peut être insérée dans le dispositif de distribution et/ou peut être enlevée du dispositif de distribution ou peut être remplacée.

16. Système selon la revendication 15, **caractérisé en ce que** la cartouche (3) présente plusieurs doses du fluide (2) pour mettre à disposition en particulier au moins 100 unités de distribution ou doses.

17. Procédé pour préparer un système, qui est un système selon la revendication 15 ou 16 pour la distribution d'un fluide (2), qui est un médicament produit à partir d'au moins deux constituants (2A, 2B), depuis la cartouche (3), pour la distribution au moyen du dispositif de distribution, en particulier d'un pulvérisateur (1),
dans lequel la cartouche (3) présente un sceau (7),
dans lequel plusieurs chambres (24, 25), séparées les unes des autres d'abord de manière fluidique, de la cartouche (3) sont reliées les unes aux autres de manière fluidique,
dans lequel un constituant (2A, 2B) du fluide (2) est transféré depuis une des chambres (24, 25) au moins en partie dans une autre des chambres (24, 25) avec un autre constituant (2A, 2B) du fluide (2) pour mélanger les constituants (2A, 2B) et/ou pour produire le fluide (2),
dans lequel un système d'actionnement est actionné en particulier manuellement pour relier les chambres les unes aux autres de manière fluidique, et les constituants (2A, 2B) sont mélangés, et
dans lequel la cartouche (3) est insérée d'abord après le mélange des constituants (2A, 2B) dans le dispositif de distribution puis est ouverte pour le prélèvement d'une dose du fluide (2) dans le dispositif de distribution,
dans lequel une ouverture de la cartouche est effectuée en ouvrant, en particulier en transperçant, le sceau (7).

18. Procédé selon la revendication 17, **caractérisé en ce que** le mélange des constituants (2A, 2B) est effectué indépendamment du dispositif de distribution.

19. Procédé selon l'une quelconque des revendications 17 ou 18, **caractérisé en ce qu'**un des constituants (2A, 2B) est transféré de manière autonome depuis une dans l'autre chambre (24, 25) en actionnant un système d'actionnement (31),
en particulier dans lequel un des constituants (2A, 2B) est transféré par une force produite par une dépression dans l'une des chambres (24, 25) depuis une dans l'autre chambre (24, 25), ou la cartouche (3) est orientée de telle manière après la liaison des chambres (24, 25) ou après le déblocage d'une liaison fluidique (30) entre les chambres (24, 25) qu'un constituant (2A, 2B) est transféré d'une dans l'autre chambre (24, 25) - en particulier exclusivement - par la force de gravité (G) .

20. Procédé selon l'une quelconque des revendications 17 à 19, **caractérisé en ce qu'**un système d'actionnement (31) est retiré de la cartouche (3) pour relier les chambres (24, 25) après l'actionnement du système d'actionnement (31), en particulier pour insérer la cartouche (3) dans le dispositif de distribution.

21. Procédé selon l'une quelconque des revendications 17 à 20, **caractérisé en ce qu'**un médicament sous forme liposomale est produit dans la cartouche (3) en mélangeant les constituants (2A, 2B).
